# EUROPEAN PATENT APPLICATION

(11) **EP 2 746 252 A1**
(43) Date of publication of application: **25.06.2014**
(21) Application number: 12199224.2
(22) Date of filing: 21.12.2012
(51) Int. Cl.: C07C 401/00

(54) **Crystallization of 1alpha-hydroxy-2-methylene- 18,19-dinor-homopregnacalciferol**

(71) Applicant: Wisconsin Alumni Research Foundation (WARF), Madison, WI 53726 (US)
(72) Inventor: DeLuca, Hector F., Deerfield, WI Wisconsin WI 53531 (US); Barycki, Rafal, Madison, WI Wisconsin WI 53719 (US); Thoden, James B., Madison, WI Wisconsin WI 53711 (US); Holden, Hazel M., Fitchburg, WI Wisconsin WI 53711 (US)
(74) Representative: Boult Wade Tennant

(57) **Abstract**

A method of purifying 1α-hydroxy-2-methylene-18,19-dinor-homopregnacalciferol to obtain 1α-hydroxy-2-methylene-18,19-dinor-homopregnacalciferol in crystalline form using precipitation with hexane from ethyl acetate. A method of preparing 1α-hydroxy-2-methylene-18,19-dinor-homopregnacalciferol crystals acceptable for X-ray experiment using precipitation with hexane from benzene by diffusive exchange of the solvents is also described.

## Description

### STATEMENT REGARDING FEDERALLY SPONSORED RESEARCH OR DEVELOPMENT

This invention was made with government support under DK047814 awarded by the National Institutes of Health. The government has certain rights in the invention.

### BACKGROUND OF THE INVENTION

The present invention relates to purification of organic compounds, and more particularly to the purification of 1α-hydroxy-2-methylene-18,19-dinor-homopregnacalciferol (referred to herein as "051810") by preparing it in crystalline form.

Purification of organic compounds, especially those designated for pharmaceutical use, is of considerable importance for chemists synthesizing such compounds. Preparation of the compound usually requires many synthetic steps and, therefore, the final product can be contaminated not only with side-products derived from the last synthetic step of the procedure but also with compounds that were formed in previous steps. Even chromatographic purification, which is a very efficient but relatively time-consuming process, does not usually provide compounds which are sufficiently pure to be used as drugs.

Depending on the method used to synthesize 1α-hydroxyvitamin D compounds, different minor undesirable compounds can accompany the final product. Thus, for example, if direct C-1 hydroxylation of 5,6-trans geometric isomer of vitamin D is performed, followed by SeO₂/NMO oxidation and photochemical irradiation [see Andrews et al., J. Org. Chem. 51, 1635 (1986); Calverley et al., Tetrahedron 43, 4609 (1987); Choudry et al, J. Org. Chem. 58, 1496 (1993)], the final 1α-hydroxyvitamin D product can be contaminated with 1β-hydroxy- as well as 5,6-trans isomers. If the method consists of C-1 allylic oxidation of the 4-phenyl-1,2,4-triazoline-3,5-dione adduct of the previtamin D compound, followed by cycloreversion of the modified adduct under basic conditions [Nevinckx et al., Tetrahedron 47, 9419 (1991); Vanmaele et al, Tetrahedron 41, 141 (1985) and 40, 1179 (1994); Vanmaele et al., Tetrahedron Lett. 23. 995 (1982)], one can expect that the desired 1α-hydroxyvitamin can be contaminated with the previtamin 5(10), 6,8-triene and 1β-hydroxy isomer. One of the most useful C-1 hydroxylation methods, of very broad scope and numerous applications, is the experimentally simple procedure elaborated by Paaren et al. [see J. Org. Chem. 45, 3253 (1980) and Proc. Natl. Acad. Sci U.S.A. 75, 2080 (1978)]. This method consists of allylic oxidation of 3,5-cyclovitamin D derivatives, readily obtained from the buffered solvolysis of vitamin D tosylates, with SeO₂/t-BuOOH and subsequent acid-catalyzed cycloreversion to the desired 1α-hydroxy compounds. Taking into account this synthetic path it is reasonable to assume that the final product can be contaminated with 1α-hydroxy epimer, 5,6-trans isomer and the previtamin D form. 1α-hydroxyvitamin D₄ is another undesirable contaminant found in 1α-hydroxyvitamin D compounds synthesized from vitamin D₂ or from ergosterol. 1α-hydroxyvitamin D₄ results from C-1 oxidation of vitamin D₄, which in turn is derived from contamination of the commercial ergosterol material. Typically, the final product may contain up to about 1.5% by weight 1α-hydroxyvitamin D₄. Thus, a purification technique that would eliminate or substantially reduce the amount of 1α-hydroxyvitamin D₄ in the final product to less than about 0.1-0.2% would be highly desirable.

The vitamin D conjugated triene system is not only heat- and light-sensitive but it is also prone to oxidation, leading to the complex mixture of very polar compounds. Oxidation usually happens when a vitamin D compound has been stored for a prolonged time. Other types of processes that can lead to a partial decomposition of vitamin D compounds consist of some water-elimination reactions; their driving force is allylic (1α-) and homoallylic (3β) position of the hydroxy groups. The presence of such above-mentioned oxidation and elimination products can be easily detected by thin-layer chromatography.

Usually, all 1α-hydroxylation procedures require at least one chromatographic purification. However, even chromatographically purified 1α-hydroxyvitamin D compounds, although showing consistent spectroscopic data, suggesting homogeneity, do not meet the purity criteria required for therapeutic agents that can be orally, parenterally or transdermally administered. Therefore, it was evident that a suitable method of purification of the 1α-hydroxylated vitamin D compound 051810 is required.

### SUMMARY OF THE INVENTION

The present invention relates to a method of purifying 051810 by means of crystallization to obtain 051810 in crystalline form. The solvent plays a crucial role in the crystallization process, and is typically an individual liquid substance or a suitable mixture of different liquids. For crystallizing 051810, the most appropriate solvent and/or solvent system is characterized by the following factors:
(1) low toxicity;
(2) low boiling point;
(3) significant dependence of solubility properties with regard to temperature (condition necessary for providing satisfactory crystallization yield); and
(4) relatively low cost.

Interestingly, hexane, so frequently used for crystallization purposes, was found less suitable as the sole solvent for crystallization of 051810. However, it was found that a mixture of ethyl acetate and hexane, was most useful for the crystallization of 051810. In particular, it was determined that a mixture of about 1% ethyl acetate with about 99% hexane (by volume) performed well. The ethyl acetate/hexane solvent mixture was also easy to remove by evaporation or other well known methods. In all cases the crystallization process occurred easily and efficiently; and the precipitated crystals were sufficiently large to assure their recovery by filtration or other means.

Accordingly, there is obtained 1α-hydroxy-2-methylene-18,19-dinor-homopregnacalciferol in crystalline form. The crystalline form and three dimensional structure of 1α-hydroxy-2-methylene-18,19-dinor-homopregnacalciferol has a molecular packing arrangement defined by space group P2 and unit cell dimensions a=4.8Å, b=22.9Å, c=36.1Å, α=90°, β=90° and γ=90°.

In one embodiment, there is described a method of purifying 1α-hydroxy-2-methylene-18,19-dinor-homopregnacalciferol, comprising the steps of:
(a) dissolving a product containing 1α-hydroxy-2-methylene-18,19-dinor-homopregnacalciferol to be purified in a solvent comprising ethyl acetate;
(b) adding hexane to said solvent and dissolved product to form a mixture;
(c) cooling said mixture containing said dissolved product below ambient temperature for a sufficient amount of time to form a precipitate of 1α-hydroxy-2-methylene-18,19-dinor-homopregnacalciferol crystals; and
(d) separating the 1α-hydroxy-2-methylene-18,19-dinor-homopregnacalciferol crystals from the mixture.

In another embodiment, there is described a method of preparing 1α-hydroxy-2-methylene-18,19-dinor-homopregnacalciferol crystals by diffusive exchange of solvents, comprising the steps of:
(a) dissolving a product containing 1α-hydroxy-2-methylene-18,19-dinor-homopregnacalciferol in a first solvent comprising benzene;
(b) providing a second solvent comprising hexane;
(c) allowing said first solvent with dissolved product and said second solvent to diffuse together for a sufficient amount of time to form a precipitate of 1α-hydroxy-2-methylene-18,19-dinor-homopregnacalciferol crystals; and
(d) recovering the 1α-hydroxy-2-methyelene-18,19-dinor-homopregnacalciferol crystals.

### BRIEF DESCRIPTION OF THE DRAWINGS

FIG. 1 is an illustration of the three dimensional structure of the first crystallographic asymmetric molecule for 051810 as defined by the atomic positional parameters discovered and set forth herein;

FIG. 2a is an illustration of the three dimensional structure of the second crystallographic asymmetric molecule for 051810 in the absence of the benzene molecule and as defined by the atomic positional parameters discovered and set forth herein;

FIG. 2b is an illustration of the three dimensional structure of the second asymmetric molecule for 051810 in the presence of the benzene molecule and as defined by the atomic positional parameters discovered and set forth herein; and

FIG. 3 is an illustration of a ball-and-stick representation of the entire asymmetric unit for 051810.

### DETAILED DESCRIPTION OF THE INVENTION

The present invention provides 1α-hydroxy-2-methylene-18,19-dinor-homopregnacalciferol (051810) in crystalline form, a pharmacologically important compound, characterized by the formula I shown below:

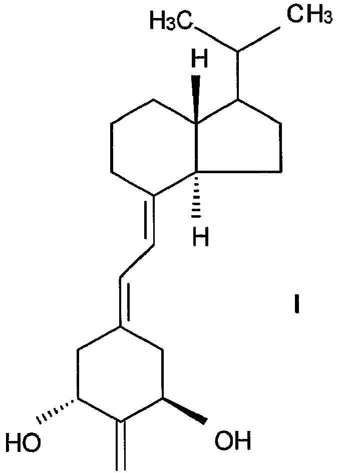

The present invention also provides a valuable method of purification of 051810. The purification technique involves obtaining the 051810 product in crystalline form by utilizing a crystallization procedure wherein the 051810 material to be purified is dissolved using ethyl acetate as the solvent and further precipitation with hexane. Preferably a ratio of ethyl acetate and hexane is about 1:99 (by volume). Thereafter, the solvent can be removed by evaporation, with or without vacuum, or other means as is well known, or the resultant crystals may be filtered from the mother liquor. The technique can be used to purify a wide range of final products containing 051810 obtained from any known synthesis thereof, and in varying concentrations, i.e. from microgram amounts to kilogram amounts. As is well known to those skilled in this art, the amount of solvent utilized should be minimized and/or adjusted according to the amount of 051810 to be purified.

The usefulness and advantages of the present crystallization procedure is shown in the following specific Example 1. After crystallization, the precipitated material was observed under a microscope to confirm its crystalline form. Yields of crystals were relatively high and the obtained crystals showed a relatively sharp melting point of 140-145°C.

The described crystallization process of the synthetic 051810 product represents a valuable purification method, which can remove most side products derived from the synthetic path. Such impurity is the result of the contamination of starting raw materials. The crystallization process occurred easily and efficiently; and the precipitated crystals were sufficiently large to assure their recovery by filtration, or other means.

**Crystallization of 1α-hydroxy-2-methylene-18,19-dinor-homopreg-nacalciferol (051810).**

### EXAMPLE 1

### Crystallization from ethyl acetate /hexane

1.25 g of prepurified (see U.S. Pat. No. 7,238,681) crude 1α-hydroxy-2-methylene-18,19-dinor-homopregnacalciferol was dissolved in ethyl acetate (3 ml) at room temperature and hexane (300 ml) was poured into the vigorously shaken solution. The mixture was kept in a refrigerator (at 4°C) overnight and the resulted crystals were filtered off, washed with one portion (30 ml) of cooled (4°C) hexane and dried under reduced pressure for 3h giving 1.10 g of a pure product.

In order to obtain crystals acceptable for the X-ray experiment, 1α-Hydroxy-2-methylene-18,19-dinor-homopregnacalciferol (12 mg) was placed in an inner tube of a vessel and dissolved in 300 µl of benzene. To an outer tube of the vessel hexane (2 ml) was poured so that a benzene to hexane ratio of about 13:87, by volume, is obtained, and the whole system was carefully purged with argon, and then maintained as a closed system. The vessel was kept tightly closed for 4 days at room temperature. Crystals were grown employing diffusive exchange of the two solvents.

A colorless rod-shaped crystal of dimensions 0.73 x 0.08 x 0.02 mm was selected for structural analysis. Intensity data were collected using a Bruker AXS Platinum 135 CCD detector controlled with the PROTEUM software suite (Bruker AXS Inc., Madison, WI). The x-ray source was CuKα radiation (1.54178 Å) from a Rigaku RU200 x-ray generator equipped with Montel optics, operated at 50 kV and 90 mA. The x-ray data were processed with SAINT version 7.06A (Bruker AXS Inc.) and internally scaled with SADABS version 2005/1 (Bruker AXS Inc.). The sample was mounted on a glass fiber using vacuum grease and cooled to 100 K. The intensity data were measured as a series of phi and omega oscillation frames each of 1° for 60-120 sec/frame. The detector was operated in 1024 x 1024 mode and was positioned 5.0 cm from the sample. Cell parameters were determined from a non-linear least squares fit of 9999 peaks in the range of 3.0 < theta < 50.8°. The data were merged to form a set of 4693 independent data with R(int)=0.0884.

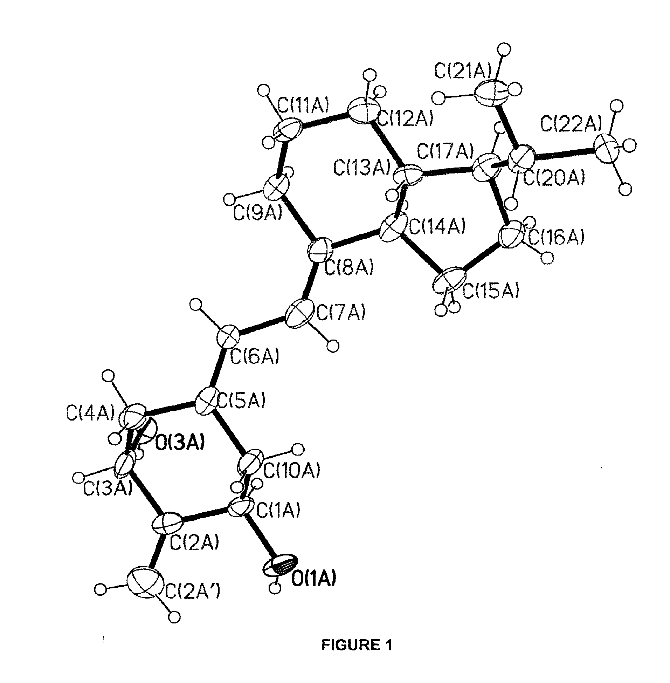

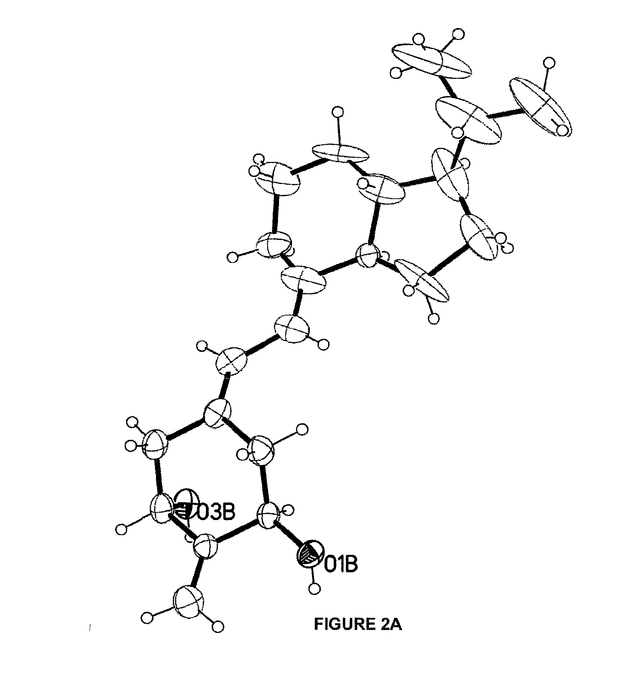

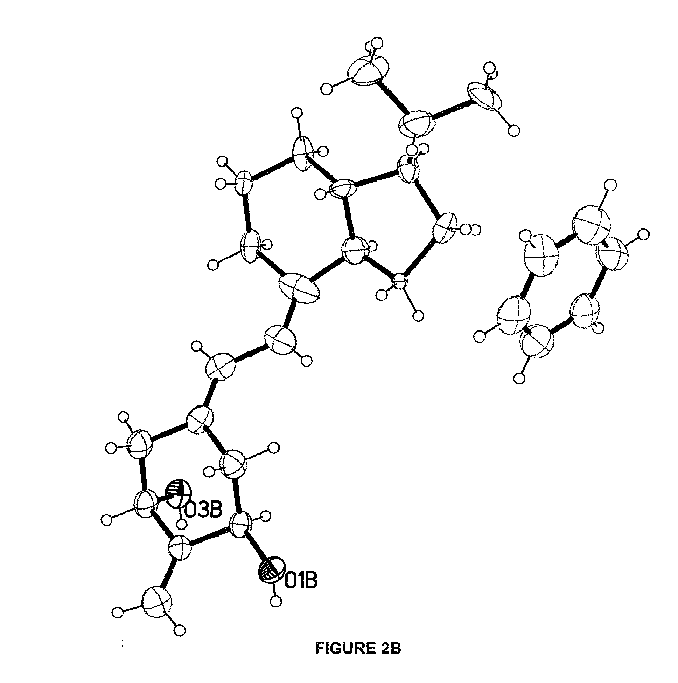

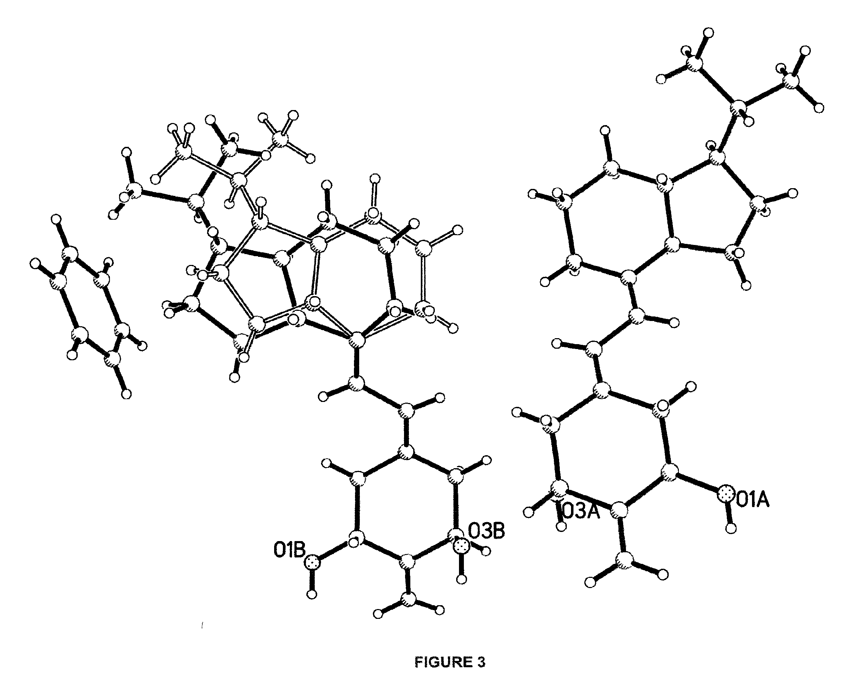

The orthorhombic space group P2(1)2(1)2(1) was determined by systematic absences and statistical tests and verified by subsequent refinement. The structure was solved by direct methods and refined by full-matrix least-squares methods on F², (a) G.M. Sheldrick (1994), SHELXTL Version 5 Reference Manual, Bruker AXS Inc.; (b) International Tables for Crystallography, Vol. C, Kluwer: Boston (1995). The asymmetric unit is comprised of two molecules of 051810 and a benzene molecule. Molecule "A" is shown in Figure 1, with thermal ellipsoids drawn at the 40% probability level. Based on lattice packing, the benzene molecule could not be present at full occupancy, and refinement of its occupancy led to a value of approximately 0.5. The presence of the benzene molecule lead to a disorder in a portion of molecule "B". Refinement of the occupancies of the two disordered configurations independently of the occupancy of the benzene converged also at a value of about 0.5 for each. Figure 2a shows molecule B in its configuration in the absence of the benzene molecule, and Figure 2b shows its configuration in the presence of the benzene molecule; both figures are drawn with thermal ellipsoids at the 40% probability level. Figure 3 shows a ball-and-stick representation of the entire asymmetric unit, with the hollow bonds showing the conformation in the presence of the benzene molecule. Hydrogen atom positions were refined by a riding model with idealized geometry. Non-hydrogen atoms were refined with anisotropic displacement parameters. The benzene molecule was refined with idealized geometry. A total of 559 parameters were refined against 26 restraints and 4693 data to give wR2 = 0.2524 and S = 0.951 for weights of w = 1/[s²(F²) + (0.1719P)²], where P = [Fₒ² + 2F_{c}²]/3. The final R(F) was 0.0910 for the 6781 observed data. The largest shift/s.u. was 0.001 in the final refinement cycle and the final difference map had maxima and minima of 0.366 and -0.305 e/Å³, respectively. The absolute structure was determined by refinement of the Flack parameter, H.D. Flack, Acta Cryst. A, vol. 39, 876-881 (1983).

The three dimensional structure of 051810 as defined by the following physical data and atomic positional parameters described and calculated herein is illustrated in Figures 1, 2a, 2b and 3.

**Table 1. Crystal data and structure refinement for 051810.**

| | |
|---|---|
| Identification Code | 051810 |
| Empirical formula | C45 H67 04 |
| Formula weight | 671.99 |
| Temperature | 100(1) K |
| Wavelength | 1.54178 Å |
| Crystal system | Orthorhombic |
| Space group | P2(1)2(1)2(1) |
| Unit cell dimensions | a = 4.8020(10)Å α = 90° |
| | b = 22.940(5)Å β = 90° |
| | c = 36.124(7)Å γ = 90° |
| Volume | 3979.3(14)Å³ |
| Z | 4 |
| Density (calculated) | 1.122 Mg/m³ |
| Absorption coefficient | 0.533 mm⁻¹ |
| F(000) | 1476 |
| Crystal | 0.73 x 0.08 x 0.02 mm |
| Theta range for data collection | 2.28 to 54.17° |
| Limiting indices | -4<h<4,-24<k<23,-37<1< 37 |
| Reflections collected | 12592 |
| Independent reflections | 4693 [R(int) = 0.0884] |
| Completeness to Theta = 54.17° | 98.2 % |
| Refinement method | Full-matrix least-squares on F² |
| Data / restraints / parameters | 4693 / 26 / 559 |
| Goodness-of-fit on F² | 0.951 |
| Final R indices [I>2σ(I)] | R1 = 0.0910, wR2 = 0.2214 |
| R indices (all data) | R1 = 0.1268, wR2 = 0.2524 |
| Extinction coefficient | 0.0124(12) |
| Largest diff. peak and hole | 0.366 and -0.305 e/A³ |
| Melting Point | 140-145°C |

**Table 2. Atomic coordinates (x 10⁴) and equivalent isotropic displacement parameters (Å² x 10³] for 051810. U(eq) is defined as one third of the trace of the orthogonalized Uᵢⱼ tensor.**

| | X | Y | Z | U(eq) |
|---|---|---|---|---|
| O(1A) | -322(12) | 7615(2) | 6913(2) | 59(2) |
| O(3A) | -2739(11) | 9404(2) | 7179(1) | 51(1) |
| C(10A) | -165(18) | 8424(3) | 6486(2) | 47(2) |
| C(1A) | -994(17) | 8232(3) | 6868(2) | 46(2) |
| C(3A) | 148(16) | 9249(3) | 7108(2) | 49(2) |
| C(14A) | -7331(19) | 8940(3) | 5377(2) | 48(2) |
| C (4A) | 864(18) | 9437(3) | 6719(2) | 49(2) |
| C(13A) | -6378(17) | 9119(3) | 4990(2) | 42(2) |
| C(7A) | -4130(18) | 9025(3) | 5916(2) | 50(2) |
| C(17A) | -7691(17) | 8673(3) | 4726(2) | 47(2) |
| C(2A) | 472(16) | 8595(3) | 7156(2) | 47(2) |
| C(15A) | -7129(18) | 8276(3) | 5360(2) | 49(2) |
| C(8A) | -5795(16) | 9274(3) | 5667(2) | 43(2) |
| C(9A) | -6387(19) | 9924(3) | 5624(2) | 52(2) |
| C(12A) | -7120(18) | 9770(3) | 4931(2) | 46(2) |
| C(5A) | -649(16) | 9072(3) | 6439(2) | 43(2) |
| C(16A) | -8423(18) | 8158(3) | 4986(2) | 50(2) |
| C(21A) | -4792(18) | 9008(3) | 4177(2) | 48(2) |
| C(20A) | -5793 (17) | 8486(3) | 4408(2) | 45(2) |
| C(11A) | -5708(19) | 10137(3) | 5234(2) | 51(2) |
| C(6A) | -2416(17) | 9326(3) | 6193(2) | 47(2) |
| C(22A) | -7262(18) | 8050(3) | 4144(2) | 55(2) |
| C(2A') | 1940(20) | 8381(4) | 7432(2) | 76(3) |
| O(3B) | -3150(11) | 10326(2) | 7677(1) | 48(1) |
| O(1B) | -4693(10) | 12069(2) | 8071(1) | 40(1) |
| C(1B) | -4362(16) | 11592(3) | 7816(2) | 39(2) |
| C(2B) | -6068(16) | 11074(3) | 7930(2) | 40(2) |
| C(10B) | -5147(19) | 11778(3) | 7423(2) | 49(2) |
| C(3B) | -5950(17) | 10575(3) | 7667(2) | 46(2) |
| C(4B) | -6576(18) | 10759(3) | 7270(2) | 48(2) |
| C(6B) | -3204(17) | 11254(3) | 6861(2) | 51(2) |
| C(5B) | -4833(17) | 11275(3) | 7155(2) | 44(2) |
| C(7B) | -1376(17) | 11745(4) | 6734(2) | 58(2) |
| C(2B') | -7707(19) | 11071(3) | 8221(2) | 56(2) |
| C(8B) | 259(18) | 11713(5) | 6435(3) | 75(3) |
| C(12B) | 2040(50) | 12137(10) | 5703(5) | 105(13) |
| C(9B) | 720(60) | 11337(11) | 6171(5) | 65(7) |
| C(14B) | 1730(40) | 12297(7) | 6369(5) | 66(5) |
| C(13B) | 1580(50) | 12609(9) | 5997(5) | 86(8) |
| C(11B) | 420(50) | 11584(11) | 5782(5) | 96(11) |
| C(15B) | 1870(60) | 12851(8) | 6602(7) | 111(14) |
| C(17B) | 3160(40) | 13190(9) | 5995(8) | 109(12) |
| C(16B) | 3350(40) | 13356(9) | 6404(7) | 101(9) |
| C(20B) | 2050(50) | 13662(10) | 5735(7) | 151(15) |
| C(22B) | 3890(50) | 14206(10) | 5741(8) | 185(18) |
| C(21B) | 1610(120) | 13442(14) | 5339(8) | 230(30) |
| C(12') | 1350(40) | 11726(7) | 5609(6) | 45(6) |
| C(9') | 160(70) | 11067(10) | 6145(6) | 46(8) |
| C(14') | 2290(40) | 12120(8) | 6246(4) | 45(8) |
| C(13') | 1240(50) | 12265(7) | 5860(4) | 36(6) |
| C(11') | -560(50) | 11251(8) | 5752(5) | 39(6) |
| C(15') | 2350(40) | 12687(8) | 6459(5) | 33(5) |
| C(17') | 2980(40) | 12809(6) | 5789(4) | 38(5) |
| C(16') | 3750(40) | 13067(7) | 6167(4) | 35(6) |
| C(20') | 1650(50) | 13270(7) | 5543(5) | 72(8) |
| C(22') | 3670(70) | 13750(10) | 5428(8) | 78(9) |
| C(21') | 80(60) | 13041(10) | 5206(6) | 76(9) |
| C(1) | 1350(20) | 14678(5) | 6378(3) | 67(5) |
| C(2) | 1060(20) | 14340(5) | 6695(4) | 64(5) |
| C(3) | 3410(30) | 14137(4) | 6879(2) | 69(5) |
| C(4) | 6050(20) | 14270(4) | 6746(3) | 58(5) |
| C(5) | 6340(20) | 14608(4) | 6429(3) | 62(5) |
| C(6) | 3990(30) | 14811(4) | 6245(3) | 74(6) |

**Table 3. Bond lengths [Å] for 051810.**

| | |
|---|---|
| O(1A)-C(1A) | 1.460(8) |
| O(1A)-H(1AA) | 0.8200 |
| O(3A)-C(3A) | 1.454(9) |
| O(3A)-H(3AA) | 0.8200 |
| C(10A)-C(1A) | 1.502(9) |
| C(10A)-C(5A) | 1.513(10) |
| C(10A)-H(10A) | 0.9700 |
| C(10A)-H(10B) | 0.9700 |
| C(1A)-C(2A) | 1.508(11) |
| C(1A)-H(1AB) | 0.9800 |
| C(3A)-C(4A) | 1.507(10) |
| C(3A)-C(2A) | 1.518(10) |
| C(3A)-H(3AB) | 0.9800 |
| C (14A) -C (8A) | 1.492(10) |
| C(14A)-C(13A) | 1.527(10) |
| C(14A)-C(15A) | 1.529(9) |
| C(14A)-H(14A) | 0.9800 |
| C(4A)-C(5A) | 1.501(10) |
| C(4A)-H(4AA) | 0.9700 |
| C(4A)-H(4AB) | 0.9700 |
| C(13A)-C(17A) | 1.534(10) |
| C(13A)-C(12A) | 1.550(9) |
| C(13A)-H(13A) | 0.9800 |
| C(7A)-C(8A) | 1.332(10) |
| C(7A)-C(6A) | 1.469(10) |
| C(7A)-H(7AA) | 0.9300 |
| C (17A) -C (20A) | 1.529(10) |
| C(17A)-C(16A) | 1.548(10) |
| C(17A)-H(17A) | 0.9800 |
| C(2A)-C(2A') | 1.314(11) |
| C(15A)-C(16A) | 1.511(10) |
| C(15A)-H(15A) | 0.9700 |
| C(15A)-H(15B) | 0.9700 |
| C(8A)-C(9A) | 1.526(10) |
| C(9A)-C(11A) | 1.528(9) |
| C(9A)-H(9AA) | 0.9700 |
| C(9A)-H(9AB) | 0.9700 |
| C(12A)-C(11A) | 1.538(10) |
| C(12A)-H(12A) | 0.9700 |
| C(12A)-H(12B) | 0.9700 |
| C(5A)-C(6A) | 1.359(10) |
| C(16A)-H(16A) | 0.9700 |
| C(16A)-H(16B) | 0.9700 |
| C(21A)-C(20A) | 1.536(10) |
| C(21A)-H(21A) | 0.9600 |
| C(21A)-H(21B) | 0.9600 |
| C(21A)-H(21C) | 0.9600 |
| C(20A)-C(22A) | 1.552(10) |
| C(20A)-H(20A) | 0.9800 |
| C(11A)-H(11A) | 0.9700 |
| C(11A)-H(11B) | 0.9700 |
| C(6A)-H(6AA) | 0.9300 |
| C(22A)-H(22A) | 0.9600 |
| C(22A)-H(22B) | 0.9600 |
| C(22A)-H(22C) | 0.9600 |
| C(2A')-H(1A') | 0.9300 |
| C(2A')-H(2A') | 0.9300 |
| O(3B)-C(3B) | 1.461(10) |
| O(3B)-H(3BA) | 0.8200 |
| O(1B)-C(1B) | 1.439(8) |
| O(1B)-H(1BA) | 0.8200 |
| C(1B)-C(2B) | 1.501(10) |
| C(1B)-C(10B) | 1.532(9) |
| C(1B)-H(1BB) | 0.9800 |
| C(2B)-C(2B') | 1.313(10) |
| C(2B)-C(3B) | 1.488 (10) |
| C(10B)-C(5B) | 1.514(9) |
| C(10B)-H(10C) | 0.9700 |
| C(10B)-H(10D) | 0.9700 |
| C(3B)-C(4B) | 1.526(7) |
| C(3B)-H(3BB) | 0.9800 |
| C(4B)-C(5B) | 1.506(10) |
| C(4B)-H(4BA) | 0.9700 |
| C(4B)-H(4BB) | 0.9700 |
| C(6B)-C(5B) | 1.321(10) |
| C(6B)-C(7B) | 1.499(8) |
| C(6B)-H(6BA) | 0.9300 |
| C(7B)-C(8B) | 1.338(12) |
| C(7B)-H(7BA) | 0.9300 |
| C(2B')-H(1B') | 0.9300 |
| C(2B')-H(2B') | 0.9300 |
| C(8B)-C(9B) | 1.50(2) |
| C(8B)-C(14') | 1.514(10) |
| C(8B)-C(14B) | 1.534(10) |
| C(8B)-C(9') | 1.52(2) |
| C(12B)-C(11B) | 1.516(10) |
| C(12B)-C(13B) | 1.534(10) |
| C(12B)-H(12C) | 0.9700 |
| C(22B)-H(12D) | 0.9700 |
| C(9B)-C(11B) | 1.522(10) |
| C(9B)-H(9BA) | 0.9700 |
| C(9B)-H(9BB) | 0.9700 |
| C(14B)-C(15B) | 1.527(10) |
| C(14B)-C(13B) | 1.524(10) |
| C(14B)-H(14B) | 0.9800 |
| C(13B)-C(17B) | 1.532(10) |
| C(13B)-H(13B) | 0.9800 |
| C(11B)-H(11C) | 0.9700 |
| C(11B)-H(11D) | 0.9700 |
| C(15B)-C(16B) | 1.535(10) |
| C(15B)-H(15C) | 0.9700 |
| C(15B)-H(15D) | 0.9700 |
| C(17B)-C(16B) | 1.528(10) |
| C(17B)-C(20B) | 1.528(10) |
| C(17B)-H(17B) | 0.9800 |
| C(16B)-H(16C) | 0.9700 |
| C(16B)-H(16D) | 0.9700 |
| C(20B)-C(22B) | 1.528(10) |
| C (20B) -C (21B) | 1.532(11) |
| C(20B)-H(20B) | 0.9800 |
| C(22B)-H(22D) | 0.9600 |
| C(22B)-H(22E) | 0.9600 |
| C(22B)-H(22F) | 0.9600 |
| C(21B)-H(21D) | 0.9600 |
| C(21B)-H(21E) | 0.9600 |
| C(21B)-H(21F) | 0.9600 |
| C(12')-C(11') | 1.513(10) |
| C(12')-C(13') | 1.534(10) |
| C(12')-H(12E) | 0.9700 |
| C(12')-H(12F) | 0.9700 |
| C(9')-C(11') | 1.519(10) |
| C(9')-H(9BC) | 0.9700 |
| C(9')-H(9BD) | 0.9700 |
| C(14')-C(15') | 1.512(10) |
| C(14')-C(13') | 1.517(10) |
| C(14')-H(14') | 0.9800 |
| C(13')-C(17') | 1.523(10) |
| C(13')-H(13') | 0.9800 |
| C(11')-H(11E) | 0.9700 |
| C(11')-H(11F) | 0.9700 |
| C(15')-C(16') | 1.524(10) |
| C(15')-H(15E) | 0.9700 |
| C(15')-H(15F) | 0.9700 |
| C(17')-C(20') | 1.523(10) |
| C(17')-C(16') | 1.533(10) |
| C(17')-H(17') | 0.9800 |
| C(16')-H(16E) | 0.9700 |
| C(16')-H(16F) | 0.9700 |
| C(20')-C(21') | 1.525(10) |
| C(20')-C(22') | 1.528(10) |
| C(20')-H(20C) | 0.9800 |
| C(22')-H(22G) | 0.9600 |
| C(22')-H(22H) | 0.9600 |
| C(22')-H(22I) | 0.9600 |
| C(21')-H(21G) | 0.9600 |
| C(21') -H (21H) | 0.9600 |
| C(21')-H(21I) | 0.9600 |
| C(1)-C(2) | 1.3900 |
| C(1)-C(6) | 1.3900 |
| C(1)-H(1A) | 0.9300 |
| C(2) -C(3) | 1.3900 |
| C(2)-H(2A) | 0.9300 |
| C(3)-C(4) | 1.3900 |
| C(3)-H(3A) | 0.9300 |
| C(4)-C(5) | 1.3900 |
| C(4)-H(4A) | 0.9300 |
| C(5)-C(6) | 1.3900 |
| C(5)-H(5A) | 0.9300 |
| C(6)-H(6A) | 0.9300 |

**Table 4. Bond angles [°] for 051810.**

| | |
|---|---|
| C(1A)-O(1A)-H(1AA) | 109.5 |
| C(3A)-O(3A)-H(3AA) | 109.5 |
| C(1A)-C(10A)-C(5A) | 110.6(6) |
| C(1A)-C(10A)-H(10A) | 109.7 |
| C(5A)-C(10A)-H(10A) | 109.6 |
| C(1A)-C(10A)-H(10B) | 109.4 |
| C(5A)-C(10A)-H(10B) | 109.4 |
| H(10A)-C(10A)-H(10B) | 108.1 |
| O(1A)-C(1A)-C(10A) | 109.1(6) |
| O(1A)-C(1A)-C(2A) | 110.9(6) |
| C(10A)-C(1A)-C(2A) | 110.4(6) |
| O(1A)-C(1A)-H(1AB) | 108.8 |
| C(10A)-C(1A)-H(1AB) | 108.8 |
| C(2A)-C(1A)-H(1AB) | 108.8 |
| O(3A)-C(3A)-C(4A) | 108.2(6) |
| O(3A)-C(3A)-C(2A) | 108.6(6) |
| C(4A)-C(3A)-C(2A) | 111.5(6) |
| O(3A)-C(3A)-H(3AB) | 109.5 |
| C(4A)-C(3A)-H(3AB) | 109.5 |
| C(2A)-C(3A)-H(3AB) | 109.5 |
| C(8A)-C(14A)-C(13A) | 110.9(7) |
| C(8A)-C(14A)-C(15A) | 120.6(7) |
| C(13A)-C(14A)-C(15A) | 102.2(6) |
| C(8A)-C(14A)-H(14A) | 107.5 |
| C(13A)-C(14A)-H(14A) | 107.5 |
| C(15A)-C(14A)-H(14A) | 107.6 |
| C(5A)-C(4A)-C(3A) | 111.0(6) |
| C(5A)-C(4A)-H(4AA) | 109.4 |
| C(3A)-C(4A)-H(4AA) | 109.4 |
| C(5A)-C(4A)-H(4AB) | 109.4 |
| C(3A)-C(4A)-H(4AB) | 109.5 |
| H(4AA)-C(4A)-H (4AB) | 108.0 |
| C(14A)-C(13A)-C(17A) | 105.5(6) |
| C(14A)-C(13A)-C(12A) | 108.4(6) |
| C(17A)-C(13A)-C(12A) | 117.6(6) |
| C(14A)-C(13A)-H(13A) | 108.4 |
| C(17A)-C(13A)-H(13A) | 108.3 |
| C(12A)-C(13A)-H(13A) | 108.4 |
| C(8A)-C(7A)-C(6A) | 126.6(7) |
| C(8A)-C(7A)-H(7AA) | 116.8 |
| C(6A)-C(7A)-H(7AA) | 116.6 |
| C(20A)-C(17A)-C(13A) | 114.2(7) |
| C(20A)-C(17A)-C(16A) | 112.1(6) |
| C(13A)-C(17A)-C(16A) | 103.1(5) |
| C(20A)-C(17A)-H(17A) | 109.1 |
| C(13A)-C(17A)-H(17A) | 109.1 |
| C(16A)-C(17A)-H(17A) | 109.1 |
| C(2A')-C(2A)-C(1A) | 124.4(7) |
| C(2A')-C(2A)-C(3A) | 120.9(8) |
| C(1A)-C(2A)-C(3A) | 114.7(6) |
| C(16A)-C(15A)-C(14A) | 100.9(6) |
| C(16A)-C(15A)-H(15A) | 111.6 |
| C(14A)-C(15A)-H(15A) | 111.6 |
| C(16A)-C(15A)-H(15B) | 111.6 |
| C(14A)-C(15A)-H(15B) | 111.5 |
| H(15A)-C(15A)-H(15B) | 109.4 |
| C(7A)-C(8A)-C(14A) | 123.4(6) |
| C(7A)-C(8A)-C(9A) | 126.8(7) |
| C(14A)-C(8A)-C(9A) | 109.8(6) |
| C(11A)-C(9A)-C(8A) | 111.5(6) |
| C(11A)-C(9A)-H(9AA) | 109.3 |
| C(8A)-C(9A)-H(9AA) | 109.4 |
| C(11A)-C(9A)-H(9AB) | 109.3 |
| C(8A)-C(9A)-H(9AB) | 109.4 |
| H(9AA)-C(9A)-H(9AB) | 108.0 |
| C(11A)-C(12A)-C(13A) | 109.2(6) |
| C(11A)-C(12A)-H(12A) | 109.8 |
| C(13A)-C(12A)-H(12A) | 109.8 |
| C(11A)-C(12A)-H(12B) | 109.9 |
| C(13A)-C(12A)-H(12B) | 109.8 |
| H(12A)-C(12A)-H(12B) | 108.3 |
| C(6A)-C(5A)-C(4A) | 120.2(6) |
| C(6A)-C(5A)-C(10A) | 126.2(7) |
| C(4A)-C(5A)-C(10A) | 113.4(6) |
| C(15A)-C(16A)-C(17A) | 108.1(6) |
| C(15A)-C(16A)-H(16A) | 110.1 |
| C(17A)-C(16A)-H(16A) | 110.1 |
| C(15A)-C(16A)-H(16B) | 110.0 |
| C(17A)-C(16A)-H(16B) | 110.1 |
| H(16A)-C(16A)-H(168) | 108.4 |
| C(20A)-C(21A)-H(21A) | 109.6 |
| C(20A)-C(21A)-H(21B) | 109.4 |
| H(21A)-C(21A)-H(21B) | 109.5 |
| C(20A)-C(21A)-H(21C) | 109.4 |
| H(21A)-C(21A)-H(21C) | 109.5 |
| H(21B)-C(21A)-H(21C) | 109.5 |
| C(17A)-C(20A)-C(21A) | 112.2(6) |
| C(17A)-C(20A)-C(22A) | 111.9(7) |
| C(21A)-C(20A)-C(22A) | 108,1(6) |
| C(17A)-C(20A)-H(20A) | 108.2 |
| C(21A)-C(20A)-H(20A) | 108.2 |
| C(22A)-C(20A)-H(20A) | 108.2 |
| C(9A)-C(11A)-C(12A) | 112.8(7) |
| C(9A)-C(11A)-H(11A) | 109.0 |
| C(12A)-C(11A)-H(11A) | 109.0 |
| C(9A)-C(11A)-H(11B) | 109.1 |
| C(12A)-C(11A)-H(11B) | 109.0 |
| H(11A)-C(11A)-H(11B) | 107.8 |
| C(5A)-C(6A)-C(7A) | 126.5(7) |
| C(5A)-C(6A)-H(6AA) | 116.8 |
| C(7A)-C(6A)-H(6AA) | 116.7 |
| C(20A)-C(22A)-H(22A) | 109.4 |
| C(20A)-C(22A)-H(22B) | 109.5 |
| H(22A)-C(22A)-H(22B) | 109.5 |
| C(20A)-C(22A)-H(22C) | 109.5 |
| H(22A)-C(22A)-H(22C) | 109.5 |
| H(22B)-C(22A)-H(22C) | 109.5 |
| C(2A)-C(2A')-H(1A') | 120.0 |
| C(2A)-C(2A')-H(2A') | 120.0 |
| H(1A')-C(2A')-H(2A') | 120.0 |
| C(3B)-O(3B)-H(3BA) | 109.4 |
| C(1B)-O(1B)-H(1BA) | 109.4 |
| O(1B)-C(1B)-C(2B) | 111.5(5) |
| O(1B)-C(1B)-C(10B) | 110.7(5) |
| C(2B)-C(1B)-C(10B) | 110.0(6) |
| O(1B)-C(1B)-H(1BB) | 108.2 |
| C(2B)-C(1B)-H(1BB) | 108.2 |
| C(10B)-C(1B)-H(1BB) | 108.2 |
| C(2B')-C(2B)-C(3B) | 122.1(7) |
| C(2B')-C(2B)-C(1B) | 123.4(7) |
| C(3B)-C(2B)-C(1B) | 114.4(6) |
| C(5B)-C(10B)-C(1B) | 110.8(6) |
| C(5B)-C(10B)-H(10C) | 109.6 |
| C(1B)-C(10B)-H(10C) | 109.5 |
| C(5B)-C(10B)-H(10D) | 109.4 |
| C(1B)-C(10B)-H(10D) | 109.5 |
| H(10C)-C(10B)-H(10D) | 108.1 |
| O(3B)-C(3B)-C(2B) | 108.7(6) |
| O(3B)-C(3B)-C(4B) | 108.2(6) |
| C(2B)-C(3B)-C(4B) | 112.4(6) |
| 0(3B)-C(3B)-H(3BB) | 109.1 |
| C(2B)-C(3B)-H(3BB) | 109.2 |
| C(4B)-C(3B)-H(3BB) | 109.2 |
| C(5B)-C(4B)-C(3B) | 111.5(6) |
| C(5B)-C(4B)-H(4BA) | 109.3 |
| C(3B)-C(4B)-H(4BA) | 109.2 |
| C(5B)-C(4B)-H(4BB) | 109.4 |
| C(3B)-C(4B)-H(4BB) | 109.4 |
| H(4BA)-C(4B)-H(4BB) | 108.0 |
| C(5B)-C(6B)-C(7B) | 124.5(7) |
| C(5B)-C(6B)-H(6BA) | 117.8 |
| C(7B)-C(6B)-H(6BA) | 117.7 |
| C(6B)-C(5B)-C(4B) | 121.5(6) |
| C(6B)-C(5B)-C(10B) | 126.9(7) |
| C(4B)-C(5B)-C(10B) | 111.6(6) |
| C(8B)-C(7B)-C(6B) | 123.3(8) |
| C(8B)-C(7B)-H(7BA) | 118.3 |
| C(6B)-C(7B)-H(7BA) | 118.4 |
| C(2B)-C(2B')-H(1B') | 120.1 |
| C(2B)-C(2B')-H(2B') | 119.9 |
| H(1B')-C(2B')-H(2B') | 120.0 |
| C(9B)-C(8B)-C(7B) | 136.7(13) |
| C(9B)-C(8B)-C(14') | 88.1(14) |
| C(7B)-C(8B)-C(14') | 135.1(12) |
| C(9B)-C(8B)-C(14B) | 112.6(15) |
| C(7B)-C(8B)-C(14B) | 110.4(11) |
| C(14')-C(8B)-C(14B) | 24.9(8) |
| C(9B)-C(8B)-C(9') | 16.9(16) |
| C(7B)-C(8B)-C(9') | 119.8(12) |
| C(14')-C(8B)-C(9') | 105.0(13) |
| C(14B)-C(8B)-C(9') | 129.4(13) |
| C(11B)-C(12B)-C(13B) | 112.7(18) |
| C(11B)-C(12B)-H(12C) | 108.9 |
| C(13B)-C(12B)-H(12C) | 108.6 |
| C(11B)-C(12B)-H(12D) | 109.3 |
| C(13B)-C(12B)-H(12D) | 109.5 |
| H(12C)-C(12B)-H(12D) | 107.8 |
| C(8B)-C(9B)-C(11B) | 114.4(19) |
| C(8B)-C(9B)-H(9BA) | 109.1 |
| C(11B)-C(9B)-H(9BA) | 108.4 |
| C(8B)-C(9B)-H(9BB) | 108.6 |
| C(11B)-C(9B)-H(9BB) | 108.6 |
| H(9BA)-C(9B)-H(9BB) | 107.6 |
| C(15B)-C(14B)-C(13B) | 95.5(15) |
| C(15B)-C(14B)-C(8B) | 131.5(13) |
| C(13B)-C(14B)-C(8B) | 121.8(14) |
| C(15B)-C(14B)-H(14B) | 100.6 |
| C(13B)-C(14B)-H(14B) | 101.3 |
| C(8B)-C(14B)-H(14B) | 101.0 |
| C(14B)-C(13B)-C(17B) | 113.0(14) |
| C(14B)-C(13B)-C(12B) | 105.8(15) |
| C(17B)-C(13B)-C(12B) | 122.6(18) |
| C(14B)-C(13B)-H(13B) | 104.3 |
| C(17B)-C(13B)-H(13B) | 104.4 |
| C(12B)-C(13B)-H(13B) | 105.0 |
| C(12B)-C(11B)-C(9B) | 115.9(19) |
| C(12B)-C(11B)-H(11C) | 108.0 |
| C(9B)-C(11B)-H(11C) | 108.5 |
| C(12B)-C(11B)-H(11D) | 108.5 |
| C(9B)-C(11B)-H(11D) | 108.3 |
| H(11C)-C(11B)-H(11D) | 107.4 |
| C(14B)-C(15B)-C(16B) | 113.1(17) |
| C(14B)-C(15B)-H(15C) | 109.4 |
| C(16B)-C(15B)-H(15C) | 109.8 |
| C(14B)-C(15B)-H(15D) | 107.9 |
| C(16B)-C(15B)-H(15D) | 108.7 |
| H(15C)-C(15B)-H(15D) | 107.6 |
| C(16B)-C(17B)-C(20B) | 115.9(19) |
| C(16B)-C(17B)-C(13B) | 104.0(16) |
| C(20B)-C(17B)-C(13B) | 116.6(16) |
| C(16B)-C(17B)-H(17B) | 106.2 |
| C(20B)-C(17B)-H(17B) | 106.6 |
| C(13B)-C(17B)-H(17B) | 106.7 |
| C(17B)-C(16B)-C(15B) | 103.5(18) |
| C(17B)-C(16B)-H(16C) | 111.1 |
| C(15B)-C(16B)-H(16C) | 110.8 |
| C(17B)-C(16B)-H(16D) | 111.7 |
| C(15B)-C(16B)-H(16D) | 110.5 |
| H(16C)-C(16B)-H(16D) | 109.2 |
| C(22B)-C(20B)-C(17B) | 111.7(17) |
| C(22B)-C(20B)-C(21B) | 111(2) |
| C(17B)-C(20B)-C(21B) | 113(2) |
| C(22B)-C(20B)-H(20B) | 107.2 |
| C(17B)-C(20B)-H(20B) | 107.2 |
| C(21B)-C(20B)-H(20B) | 106.3 |
| C(20B)-C(22B)-H(22D) | 109.5 |
| C(20B)-C(22B)-H(22E) | 109.5 |
| H(22D)-C(22B)-H(22E) | 109.5 |
| C(20B)-C(22B)-H(22F) | 109.3 |
| H(22D)-C(22B)-H(22F) | 109.5 |
| H(22E)-C(22B)-H(22F) | 109.5 |
| C(20B)-C(21B)-H(21D) | 110.2 |
| C(20B)-C(21B)-H(21E) | 109.4 |
| H(21D)-C(21B)-H(21E) | 109.5 |
| C(20B)-C(21B)-H(21F) | 108.8 |
| H(21D)-C(21B)-H(21F) | 109.5 |
| H(21E)-C(21B)-H(21F) | 109.5 |
| C(11')-C(12')-C(13') | 111.0(13) |
| C(11')-C(12')-H(12E) | 109.4 |
| C(13')-C(12')-H(12E) | 109.7 |
| C(11')-C(12')-H(12F) | 109.8 |
| C(13')-C(12')-H(12F) | 108.9 |
| H(12E)-C(12')-H(12F) | 108.0 |
| C(11')-C(9')-C(8B) | 108.5(15) |
| C(11')-C(9')-H(9BC) | 110.2 |
| C(8B)-C(9')-H(9BC) | 109.7 |
| C(11')-C(9')-H(9BD) | 109.9 |
| C(8B)-C(9')-H(9BD) | 110.3 |
| H(9BC)-C(9')-H(9BD) | 108.3 |
| C(8B)-C(14')-C(15') | 108.3(13) |
| C(8B)-C(14')-C(13') | 109.7(13) |
| C(15')-C(14')-C(13') | 106.5(15) |
| C(8B)-C(14')-H(14') | 111.1 |
| C(15')-C(14')-H(14') | 110.6 |
| C(13')-C(14')-H(14') | 110.5 |
| C(14')-C(13')-C(17') | 98.8(11) |
| C(14')-C(13')-C(12') | 110.8(17) |
| C(17')-C(13')-C(12') | 122.8(15) |
| C(14')-C(13')-H(13') | 107.9 |
| C(17')-C(13')-H(13') | 107.7 |
| C(12')-C(13')-H(13') | 107.8 |
| C(12')-C(11')-C(9') | 112.5(18) |
| C(12')-C(11')-H(11E) | 108.6 |
| C(9')-C(11')-H(11E) | 108.7 |
| C(12')-C(11')-H(11F) | 109.6 |
| C(9')-C(11')-H(11F) | 109.5 |
| H(11E)-C(11')-H(11F) | 107.9 |
| C(14')-C(15')-C(16') | 98.5(12) |
| C(14')-C(15')-H(15E) | 112.0 |
| C(16')-C(15')-H(15E) | 112.7 |
| C(14')-C(15')-H(15F) | 111.7 |
| C(16')-C(15')-H(15F) | 111.9 |
| H(15E)-C(15')-H(15F) | 109.7 |
| C(20')-C(17')-C(13') | 115.8(13) |
| C(20')-C(17')-C(16') | 110.6(13) |
| C(13')-C(17')-C(16') | 107.3(13) |
| C(20')-C(17')-H(17') | 107.9 |
| C(13')-C(17')-H(17') | 107.4 |
| C(16')-C(17')-H(17') | 107.5 |
| C(15')-C(16')-C(17') | 106.8(14) |
| C(15')-C(16')-H(16E) | 109.9 |
| C(17')-C(16')-H(16E) | 110.7 |
| C(15')-C(16')-H(16F) | 110.1 |
| C(17')-C(16')-H(16F) | 110.8 |
| H(16E)-C(16')-H(16F) | 108.6 |
| C(17')-C(20')-C(21') | 115.7(14) |
| C(17')-C(20')-C(22') | 112.9(18) |
| C(21')-C(20')-C(22') | 110(2) |
| C(17')-C(20')-H(20C) | 105.6 |
| C(21')-C(20')-H(20C) | 105.5 |
| C(22')-C(20')-H(20C) | 105.8 |
| C(20')-C(22')-H(22G) | 109.6 |
| C(20')-C(22')-H(22H) | 109.7 |
| H(22G)-C(22')-H(22H) | 109.5 |
| C(20')-C(22')-H(22I) | 109.1 |
| H(22G)-C(22')-H(22I) | 109.5 |
| H(22H)-C(22')-H(22I) | 109.5 |
| C(20')-C(21')-H(21G) | 109.3 |
| C(20')-C(21')-H(21H) | 109.7 |
| H(21G)-C(21')-H(21H) | 109.5 |
| C(20')-C(21')-H(21I) | 109.5 |
| H(21G)-C(21')-H(21I) | 109.5 |
| H(21H)-C(21')-H(21I) | 109.5 |
| C(2)-C(1)-C(6) | 120.0 |
| C(2)-C(1)-H(1A) | 120.0 |
| C(6)-C(1)-H(1A) | 120.0 |
| C(1)-C(2)-C(3) | 120.0 |
| C(1)-C(2)-H(2A) | 120.0 |
| C(3)-C(2)-H(2A) | 120.0 |
| C(2)-C(3)-C(4) | 120.0 |
| C(2)-C(3)-H(3A) | 120.0 |
| C(4)-C(3)-H(3A) | 120.0 |
| C(5)-C(4)-C(3) | 120.0 |
| C(5)-C(4)-H(4A) | 120.0 |
| C(3)-C(4)-H(4A) | 120.0 |
| C(4)-C(5)-C(6) | 120.00(5) |
| C(4)-C(5)-H(5A) | 120.0 |
| C(6)-C(5)-H(5A) | 120.0 |
| C(5)-C(6)-C(1) | 120.0 |
| C(5)-C(6)-H(6A) | 120.0 |
| C(1)-C(6)-H(6A) | 120.0 |

**Table 5. Anisotropic displacement parameters [Å² x 10³] for 051810. The anisotropic displacement factor exponent takes the form: -2π² [h²a*²U₁₁ + ... + 2hka*b*U₁₂]**

| | U₁₁ | U₂₂ | U₃₃ | U₂₃ | U₁₃ | U₁₂ |
|---|---|---|---|---|---|---|
| O(1A) | 50(4) | 34(3) | 93(4) | 21(3) | 2(3) | 9(3) |
| O(3A) | 41(4) | 48(3) | 64(3) | -2(3) | 7(3) | 6(3) |
| C(10A) | 53(6) | 33(4) | 54(5) | -5(3) | -2(4) | -4(4) |
| C(1A) | 35(5) | 27(4) | 76(5) | 12(4) | 3(4) | 5(3) |
| C(3A) | 37(5) | 36(4) | 75(5) | -2(4) | -6(4) | 4(4) |
| C(14A) | 53(6) | 36(4) | 56(5) | 3(4) | 0(4) | 6(4) |
| C(4A) | 50(6) | 36(4) | 61(5) | 7(4) | 0(4) | -6(4) |
| C(13A) | 42(5) | 35(4) | 50(4) | 8(3) | 4(4) | 9(4) |
| C(7A) | 60(6) | 35(4) | 55(5) | -8(4) | 13(4) | -9(4) |
| C(17A) | 42(5) | 45(4) | 52(5) | 7(4) | -3(4) | 3(4) |
| C(2A) | 41(5) | 50(5) | 50(4) | 12(4) | 8(4) | 4(4) |
| C(15A) | 52(6) | 39(4) | 58(5) | 8(4) | 6(4) | 0(4) |
| C(8A) | 44(5) | 35(4) | 50(5) | 8(4) | 4(4) | 6(4) |
| C(9A) | 53(6) | 47(5) | 56(5) | 2(4) | 10(4) | 1(4) |
| C(12A) | 54(6) | 36(4) | 46(4) | 6(3) | 2(4) | 9(4) |
| C(5A) | 36(5) | 39(4) | 55(4) | 0(4) | 4(4) | 1(4) |
| C(16A) | 43(5) | 44(5) | 62(5) | 4(4) | -3(4) | 2(4) |
| C(21A) | 48(6) | 43(4) | 53 (4) | 1(4) | 2(4) | 10(4) |
| C(20A) | 39(5) | 38(4) | 57(5) | 1(4) | -1(4) | 4(4) |
| C(11A) | 59(6) | 40(4) | 53 (5) | 4(4) | 3(4) | 5(4) |
| C(6A) | 50(5) | 33(4) | 60(5) | 2(4) | 8 (4) | -3(4) |
| C(22A) | 59(6) | 47(5) | 59(5) | 0(4) | -12(4) | -1(4) |
| C(2A') | 77(8) | 78(6) | 74(6) | -4(5) | -5(5) | 33(6) |
| O(3B) | 49(4) | 34(3) | 60(3) | -1(2) | -3(3) | 4(2) |
| O(1B) | 48(3) | 31(3) | 41(3) | -5(2) | -3(2) | 4(2) |
| C(1B) | 44(5) | 28(4) | 45(4) | -2(3) | -1(3) | -4(3) |
| C(2B) | 37(5) | 42(4) | 41(4) | -3(3) | 0(3) | -4(3) |
| C(10B) | 54(6) | 38(4) | 56(5) | -3(4) | 3(4) | -3(4) |
| C(3B) | 49(5) | 32(4) | 57(5) | -6(4) | 0(4) | -3(4) |
| C(4B) | 49(5) | 39(4) | 55(5) | -10(4) | 3(4) | -2(4) |
| C(6B) | 49(6) | 55(5) | 48(5) | -6(4) | -4(4) | 10(4) |
| C(5B) | 45(5) | 36(4) | 51(4) | -10(3) | -3(4) | 10(4) |
| C(7B) | 38(5) | 79(6) | 57(5) | 15(4) | 1(4) | 12(5) |
| C(2B') | 59(6) | 42(5) | 66(5) | -1(4) | 1(5) | 4(4) |
| C(8B) | 33(6) | 124(9) | 68(6) | 37(7) | 6(5) | 28(6) |
| C(12B) | 83(18) | 190(30) | 43(14) | 64(16) | 31(12) | 80(20) |
| C(9B) | 43(13) | 100(20) | 48(13) | 5(13) | -7(9) | 8(16) |
| C(14B) | 16(11) | 118(13) | 63(11) | 38(10) | 26(8) | 27(10) |
| C(13B) | 74(16) | 109(19) | 74(15) | 40(15) | 38(13) | 44(15) |
| C(11B) | 32(16) | 170(40) | 89(19) | 40(20) | 22(12) | 20(17) |
| C(15B) | 44(14) | 140(30) | 150(30) | 110(20) | 30(20) | 20(20) |
| C(17B) | 9(12) | 87(19) | 230(30) | 70(20) | -3(15) | 13(11) |
| C(16B) | 50(13) | 84(15) | 170(20) | 66(17) | 0(13) | -1(11) |
| C(20B) | 110(20) | 140(30) | 200(30) | 120(30) | 70(20) | 90(20) |
| C(22B) | 120(20) | 140(20) | 300(40) | 140(30) | 130(30) | 51(19) |
| C(21B) | 410(90) | 170(50) | 120(30) | 100(40) | 100(50) | 100(50) |
| C(12') | 44(15) | 10(9) | 82(17) | -11(11) | 3(11) | 0(9) |
| C(9') | 38(17) | 23(13) | 77(17) | -8(11) | -5(11) | -1(10) |
| C(14') | 18(16) | 64(14) | 53(16) | 17(12) | 35(12) | 35(12) |
| C(13') | 50(16) | 34(14) | 26(13) | -8(9) | 2(11) | 0(12) |
| C(11') | 34(14) | 42(12) | 41(11) | -8(9) | 0(9) | -22(9) |
| C(15') | 15(13) | 44(9) | 39(10) | 12(7) | 5(7) | -3(8) |
| C(17') | 44(13) | 13(11) | 56(12) | 2(9) | -7(10) | -4(9) |
| C(16') | 37(14) | 14(10) | 56(13) | -22(10) | -7(11) | 12(9) |
| C(20') | 130(20) | 48(17) | 42(14) | -7(13) | -1(15) | -9(16) |
| C(22') | 160(30) | 31(13) | 46(14) | 5(11) | 35(15) | -24(14) |
| C(21') | 120(20) | 49(15) | 56(14) | 18(11) | -35(14) | 9(14) |
| C(1) | 55(14) | 51(11) | 95(14) | -8(10) | 2(10) | 11(9) |
| C(2) | 62(14) | 50(10) | 80(13) | -31(10) | 15(10) | 9(10) |
| C(3) | 83(16) | 53(10) | 71(12) | -10(9) | 12(12) | -1(11) |
| C(4) | 63(13) | 36(9) | 74(12) | -12(8) | 3(9) | 4(9) |
| C(5) | 91(15) | 30(9) | 67(12) | 7(8) | 7(11) | 3(9) |
| C(6) | 87(17) | 49(11) | 85(13) | -1(10) | 7(13) | 11(11) |

**Table 6. Hydrogen coordinates (x 10⁴) and isotropic displacement parameters (Å² x 10³) for 051810.**

| | x | y | z | U(eq) |
|---|---|---|---|---|
| H(1AA) | -772 | 7508 | 7121 | 88 |
| H(3AA) | -3165 | 9304 | 7389 | 76 |
| H(10A) | -1247 | 8212 | 6304 | 56 |
| H(10B) | 1787 | 8337 | 6445 | 56 |
| H(1AB) | -3008 | 8283 | 6896 | 55 |
| H(3AB) | 1358 | 9452 | 7284 | 59 |
| H(14A) | -9307 | 9042 | 5399 | 58 |
| H(4AA) | 363 | 9843 | 6686 | 59 |
| H(4AB) | 2855 | 9400 | 6681 | 59 |
| H(13A) | -4349 | 9078 | 4977 | 51 |
| H(7AA) | -4044 | 8620 | 5914 | 60 |
| H(17A) | -9414 | 8836 | 4624 | 56 |
| H(15A) | -5210 | 8145 | 5369 | 59 |
| H(15B) | -8177 | 8093 | 5557 | 59 |
| H(9AA) | -8336 | 9998 | 5677 | 62 |
| H(9AB) | -5281 | 10140 | 5802 | 62 |
| H(12A) | -6487 | 9896 | 4689 | 55 |
| H(12B) | -9123 | 9821 | 4943 | 55 |
| H(16A) | -10428 | 8123 | 5010 | 60 |
| H(16B) | -7704 | 7797 | 4884 | 60 |
| H(21A) | -3850 | 9281 | 4334 | 72 |
| H(21B) | -6364 | 9194 | 4063 | 72 |
| H(21C) | -3535 | 8874 | 3988 | 72 |
| H(20A) | -4155 | 8293 | 4514 | 53 |
| H(11A) | -3707 | 10126 | 5198 | 61 |
| H(11B) | -6304 | 10539 | 5209 | 61 |
| H(6AA) | -2553 | 9730 | 6201 | 57 |
| H(22A) | -5981 | 7928 | 3955 | 82 |
| H(22B) | -8841 | 8235 | 4031 | 82 |
| H(22C) | -7870 | 7716 | 4282 | 82 |
| H(1A') | 2123 | 7979 | 7458 | 114 |
| H(2A') | 2789 | 8630 | 7600 | 114 |
| H(3BA) | -2777 | 10226 | 7889 | 72 |
| H(1BA) | -4248 | 11964 | 8280 | 60 |
| H(1BB) | -2397 | 11477 | 7816 | 47 |
| H(10C) | -7059 | 11915 | 7420 | 59 |
| H(10D) | -3958 | 12098 | 7346 | 59 |
| H(3BB) | -7298 | 10278 | 7744 | 55 |
| H(4BA) | -6208 | 10435 | 7105 | 57 |
| H(4BB) | -8532 | 10859 | 7249 | 57 |
| H(6BA) | -3190 | 10912 | 6723 | 61 |
| H(7BA) | -1379 | 12089 | 6870 | 69 |
| H(1B') | -8841 | 10751 | 8268 | 84 |
| H(2B') | -7733 | 11389 | 8381 | 84 |
| H(12C) | 1489 | 12288 | 5463 | 126 |
| H(12D) | 4012 | 12045 | 5691 | 126 |
| H(9BA) | -563 | 11014 | 6199 | 78 |
| H(9BB) | 2594 | 11184 | 6199 | 78 |
| H(14B) | 3687 | 12175 | 6372 | 79 |
| H(13B) | -381 | 12719 | 5970 | 103 |
| H(11C) | 1004 | 11288 | 5606 | 115 |
| H(11D) | -1538 | 11661 | 5737 | 115 |
| H(15C) | 2846 | 12767 | 6831 | 134 |
| H(15D) | -5 | 12972 | 6664 | 134 |
| H(17B) | 5058 | 13103 | 5913 | 131 |
| H(16C) | 2418 | 13724 | 6451 | 121 |
| H(16D) | 5278 | 13387 | 6482 | 121 |
| H(20B) | 220 | 13778 | 5830 | 181 |
| H(22D) | 3864 | 14386 | 5502 | 277 |
| H(22E) | 5760 | 14097 | 5804 | 277 |
| H(22F) | 3189 | 14475 | 5922 | 277 |
| H(21D) | -117 | 13593 | 5245 | 349 |
| H(21E) | 1547 | 13024 | 5339 | 349 |
| H(21F) | 3116 | 13572 | 5186 | 349 |
| H(12E) | 785 | 11834 | 5360 | 54 |
| H(12F) | 3241 | 11581 | 5598 | 54 |
| H(9BC) | 1963 | 10874 | 6148 | 55 |
| H(9BD) | -1223 | 10796 | 6237 | 55 |
| H(14') | 4150 | 11946 | 6235 | 54 |
| H(13') | -708 | 12386 | 5882 | 44 |
| H(11E) | -427 | 10915 | 5590 | 47 |
| H(11F) | -2464 | 11390 | 5746 | 47 |
| H(15E) | 3449 | 12658 | 6683 | 39 |
| H(15F) | 490 | 12824 | 6519 | 39 |
| H(17') | 4716 | 12684 | 5669 | 45 |
| H(16E) | 5753 | 13064 | 6201 | 42 |
| H(16F) | 3094 | 13466 | 6185 | 42 |
| H(20C) | 240 | 13461 | 5697 | 87 |
| H(22G) | 2711 | 14036 | 5282 | 118 |
| H(22H) | 5163 | 13584 | 5286 | 118 |
| H(22I) | 4425 | 13933 | 5646 | 118 |
| H(21G) | -707 | 13362 | 5071 | 114 |
| H(21H) | -1382 | 12784 | 5285 | 114 |
| H(21I) | 1345 | 12832 | 5048 | 114 |
| H(1A) | -219 | 14814 | 6255 | 81 |
| H(2A) | -706 | 14251 | 6784 | 77 |
| H(3A) | 3213 | 13911 | 7091 | 83 |
| H(4A) | 7619 | 14134 | 6869 | 69 |
| H(5A) | 8105 | 14697 | 6340 | 75 |
| H(6A) | 4186 | 15037 | 6032 | 89 |

**Table 7. Observed and calculated structure factors for 051810.**

| h | k | l | 10Fo | 10Fc | 10s | h | k | l | 10Fo | 10Fc | 10s | h | k | l | 10Fo | 10Fc | 10s | h | k | l | 10Fo | 10Fc | 10s | h | k | l | 10Fo | 10Fc | 10s |
|---|---|---|---|---|---|---|---|---|---|---|---|---|---|---|---|---|---|---|---|---|---|---|---|---|---|---|---|---|---|
| 2 | 0 | 0 | 42 | 37 | 16 | 3 | 0 | 1 | 336 | 325 | 28 | 2 | 11 | 1 | 322 | 300 | 23 | -3 | 1 | 2 | 263 | 203 | 23 | 2 | 211 | 2 | 356 | 337 | 16 |
| 4 | 0 | 0 | 138 | 110 | 15 | 4 | 0 | 1 | 0 | 25 | 1 | 3 | 11 | 1 | 63 | 25 | 49 | -2 | 1 | 2 | 315 | 296 | 10 | 3 | 311 | 2 | 206 | 176 | 15 |
| 1 | 1 | 0 | 437 | 450 | 11 | -3 | 1 | 1 | 201 | 154 | 25 | -4 | 12 | 1 | 194 | 205 | 21 | -1 | 1 | 2 | 1086 | 934 | 28 | -4 | -412 | 2 | 92 | 98 | 42 |
| 2 | 1 | 0 | 362 | 322 | 16 | -2 | 1 | 1 | 116 | 114 | 8 | -3 | 12 | 1 | 232 | 275 | 22 | 0 | 1 | 2 | 752 | 671 | 17 | -3 | -312 | 2 | 200 | 196 | 18 |
| 3 | 1 | 0 | 252 | 218 | 24 | -1 | 1 | 1 | 143 | 962 | 30 | -2 | 12 | 1 | 284 | 258 | 14 | 1 | 1 | 2 | 1040 | 934 | 33 | -2 | -112 | 2 | 178 | 157 | 9 |
| 0 | 2 | 0 | 271 | 282 | 8 | 0 | 1 | 1 | 134 | 157 | 3 | -1 | 12 | 1 | 522 | 526 | 18 | 2 | 1 | 2 | 338 | 296 | 14 | -1 | 12 | 2 | 294 | 255 | 15 |
| 1 | 2 | 0 | 426 | 437 | 14 | 1 | 1 | 1 | 1115 | 961 | 54 | 0 | 12 | 1 | 680 | 694 | 30 | 3 | 1 | 2 | 241 | 203 | 26 | 0 | 012 | 2 | 286 | 242 | 28 |
| 2 | 2 | 0 | 277 | 256 | 13 | 2 | 1 | 1 | 125 | 114 | 9 | 1 | 12 | 1 | 509 | 525 | 23 | 4 | 1 | 2 | 147 | 115 | 13 | 1 | 112 | 2 | 275 | 255 | 15 |
| 3 | 2 | 0 | 352 | 250 | 29 | 3 | 1 | 1 | 212 | 154 | 23 | 2 | 12 | 1 | 260 | 258 | 9 | -4 | 2 | 2 | 0 | 44 | 1 | 2 | 212 | 2 | 167 | 157 | 9 |
| 4 | 2 | 0 | 158 | 155 | 27 | 4 | 1 | 1 | 159 | 141 | 16 | 3 | 12 | 1 | 285 | 275 | 14 | -3 | 2 | 2 | 297 | 289 | 26 | 3 | 312 | 2 | 216 | 196 | 31 |
| 1 | 3 | 0 | 177 | 158 | 10 | -3 | 2 | 1 | 441 | 430 | 24 | 4 | 12 | 1 | 198 | 205 | 20 | -2 | 2 | 2 | 679 | 628 | 20 | 4 | -412 | 2 | 110 | 98 | 16 |
| 2 | 3 | 0 | 400 | 366 | 25 | -2 | 2 | 1 | 436 | 399 | 19 | -4 | 13 | 1 | 80 | 79 | 45 | 0 | 2 | 2 | 84 | 89 | 5 | -4 | 13 | 2 | 161 | 165 | 22 |
| 3 | 3 | 0 | 502 | 441 | 19 | -1 | 2 | 1 | 1871 | 1484 | 56 | -2 | 13 | 1 | 91 | 91 | 13 | 2 | 2 | 2 | 663 | 627 | 43 | -3 | -313 | 2 | 209 | 217 | 18 |
| 4 | 3 | 0 | 108 | 115 | 41 | 0 | 2 | 1 | 142 | 155 | 3 | -1 | 13 | 1 | 164 | 146 | 11 | 3 | 2 | 2 | 299 | 289 | 25 | -2 | -213 | 2 | 67 | 40 | 28 |
| 0 | 4 | 0 | 2471 | 1827 | 86 | 1 | 2 | 1 | 819 | 1485 | 117 | 0 | 13 | 1 | 365 | 339 | 38 | 4 | 2 | 2 | 23 | 44 | 23 | -1 | -113 | 2 | 136 | 167 | 32 |
| 1 | 4 | 0 | 364 | 399 | 10 | 2 | 2 | 1 | 432 | 400 | 28 | 1 | 13 | 1 | 152 | 147 | 10 | -3 | 3 | 2 | 176 | 158 | 17 | 0 | 013 | 2 | 457 | 437 | 21 |
| 2 | 4 | 0 | 337 | 336 | 22 | 3 | 2 | 1 | 453 | 431 | 34 | 2 | 13 | 1 | 69 | 92 | 17 | -2 | 3 | 2 | 554 | 530 | 24 | 1 | 113 | 2 | 173 | 167 | 12 |
| 3 | 4 | 0 | 262 | 232 | 10 | 4 | 2 | 1 | 117 | 84 | 36 | 3 | 13 | 1 | 292 | 333 | 16 | -1 3 | | 2 | 403 | 447 | 12 | 2 | 213 | 2 | 60 | 40 | 20 |
| 4 | 4 | 0 | 121 | 91 | 37 | -3 | 3 | 1 | 95 | 101 | 19 | 4 | 13 | 1 | 80 | 79 | 19 | 0 | 3 | 2 | 207 | 208 | 5 | 3 | 313 | 2 | 221 | 217 | 20 |
| 1 | 5 | 0 | 76 | 86 | 7 | -2 | 3 | 1 | 356 | 355 | 23 | -4 | 14 | 1 | 132 | 123 | 26 | 1 | 3 | 2 | 400 | 446 | 11 | 4 | 413 | 2 | 166 | 164 | 11 |
| 2 | 5 | 0 | 79 | 85 | 29 | -1 | 3 | 1 | 861 | 840 | 26 | -3 | 14 | 1 | 56 | 95 | 55 | 2 | 3 | 2 | 481 | 530 | 37 | -4 | -414 | 2 | 106 | 114 | 34 |
| 3 | 5 | 0 | 117 | 101 | 12 | 0 | 3 | 1 | 1901 | 1442 | 47 | -2 | 14 | 1 | 247 | 228 | 11 | 3 | 3 | 2 | 153 | 158 | 7 | -3 | -214 | 2 | 170 | 192 | 14 |
| 4 | 5 | 0 | 175 | 222 | 27 | 1 | 3 | 1 | 857 | 839 | 30 | -1 | 14 | 1 | 83 | 79 | 16 | 4 | 3 | 2 | 62 | 50 | 62 | -2 | 14 | 2 | 118 | 88 | 17 |
| 0 | 6 | 0 | 296 | 309 | 14 | 2 | 3 | 1 | 358 | 354 | 23 | 0 | 14 | 1 | 324 | 311 | 11 | -3 | 4 | 2 | 300 | 241 | 11 | -1 | -114 | 2 | 197 | 158 | 23 |
| 1 | 6 | 0 | 589 | 676 | 20 | 3 | 3 | 1 | 139 | 100 | 15 | 1 | 14 | 1 | 65 | 79 | 30 | -2 | 4 | 2 | 218 | 219 | 11 | 0 | 014 | 2 | 66 | 57 | 26 |
| 2 | 6 | 0 | 86 | 46 | 29 | 4 | 3 | 1 | 89 | 72 | 57 | 2 | 14 | 1 | 261 | 227 | 9 | -1 | 4 | 2 | 639 | 687 | 17 | 1 | 114 | 2 | 171 | 158 | 11 |
| 3 | 6 | 0 | 199 | 199 | 14 | -4 | 4 | 1 | 146 | 157 | 30 | 3 | 14 | 1 | 77 | 94 | 31 | 0 | 4 | 2 | 122 | 154 | 5 | 2 | 214 | 2 | 81 | 89 | 16 |
| 1 | 7 | 0 | 111 | 130 | 6 | -3 | 4 | 1 | 294 | 284 | 11 | 4 | 14 | 1 | 116 | 123 | 14 | 1 | 4 | 2 | 651 | 686 | 15 | 3 | 314 | 2 | 165 | 192 | 18 |
| 2 | 7 | 0 | 99 | 12 | 27 | -2 | 4 | 1 | 334 | 364 | 20 | -3 | 15 | 1 | 11 | 72 | 11 | 2 | 4 | 2 | 200 | 219 | 18 | 4 | 414 | 2 | 118 | 114 | 14 |
| 3 | 7 | 0 | 164 | 132 | 10 | -1 | 4 | 1 | 415 | 464 | 11 | -2 | 15 | 1 | 164 | 167 | 10 | 3 | 4 | 2 | 310 | 241 | 12 | -3 | -215 | 2 | 92 | 86 | 21 |
| 0 | 8 | 0 | 833 | 923 | 65 | 0 | 4 | 1 | 961 | 903 | 26 | -1 | 15 | 1 | 119 | 1.02 | 13 | 4 | 4 | 2 | 113 | 91 | 40 | -2 | -215 | 2 | 201 | 183 | 9 |
| 1 | 8 | 0 | 272 | 335 | 26 | 1 | 4 | 1 | 415 | 464 | 10 | 0 | 15 | 1 | 62 | 54 | 23 | -3 | 5 | 2 | 346 | 301 | 12 | -1 | -115 | 2 | 122 | 96 | 13 |
| 2 | 8 | 0 | 75 | 86 | 41 | 2 | 4 | 1 | 308 | 364 | 22 | 1 | 15 | 1 | 88 | 103 | 19 | -2 | 5 | 2 | 56 | 61 | 24 | 0 | 015 | 2 | 16 | 5 | 15 |
| 3 | 8 | 0 | 158 | 156 | 23 | 3 | 4 | 1 | 292 | 283 | 12 | 2 | 15 | 1 | 166 | 167 | 10 | -1 | 5 | 2 | 761 | 787 | 25 | 1 | 115 | 2 | 125 | 97 | 13 |
| 1 | 9 | 0 | 300 | 305 | 18 | 4 | 4 | 1 | 154 | 156 | 28 | 3 | 15 | 1 | 0 | 72 | 1 | 0 | 5 | 2 | 393 | 436 | 12 | 2 | 215 | 2 | 197 | 182 | 13 |
| 2 | 9 | 0 | 293 | 277 | 14 | -3 | 5 | 1 | 218 | 162 | 21 | -3 | 16 | 1 | 106 | 124 | 20 | 1 | 5 | 2 | 744 | 787 | 20 | 3 | 315 | 2 | 71 | 87 | 41 |
| 3 | 9 | 0 | 306 | 302 | 16 | -2 | 5 | 1 | 251 | 211 | 16 | -2 | 16 | 1 | 98 | 95 | 14 | 2 | 5 | 2 | 0 | 61 | 1 | -3 | -316 | 2 | 161 | 175 | 29 |
| 0 | 10 | 0 | 259 | 184 | 39 | -1 | 5 | 1 | 621 | 673 | 26 | -1 | 16 | 1 | 79 | 59 | 29 | 3 | 5 | 2 | 344 | 301 | 14 | -2 | -216 | 2 | 117 | 99 | 12 |
| 1 | 10 | 0 | 259 | 210 | 11 | 0 | 5 | 1 | 143 | 120 | 6 | 0 | 16 | 1 | 40 | 1 | 40 | 4 | 5 | 2 | 110 | 82 | 42 | -1 | -116 | 2 | 157 | 148 | 14 |
| 2 | 10 | 0 | 77 | 5 | 43 | 1 | 5 | 1 | 624 | 673 | 16 | 1 | 16 | 1 | 87 | 59 | 25 | -3 | 6 | 2 | 175 | 123 | 10 | 0 | 016 | 2 | 64 | 71 | 21 |
| 3 | 10 | 0 | 97 | 83 | 21 | 2 | 5 | 1 | 214 | 212 | 19 | 2 | 16 | 1 | 88 | 94 | 17 | -2 | 6 | 2 | 245 | 245 | 12 | 1 | 116 | 2 | 152 | 148 | 15 |
| 1 | 11 | 0 | 348 | 359 | 13 | 3 | 5 | 1 | 193 | 163 | 11 | 3 | 16 | 1 | 111 | 124 | 18 | -1 | 6 | 2 | 194 | 197 | 18 | 2 | 216 | 2 | 108 | 100 | 14 |
| 2 | 11 | 0 | 86 | 11 | 31 | 4 | 5 | 1 | 97 | 32 | 53 | -3 | 17 | 1 | 85 | 71 | 25 | 0 | 6 | 2 | 717 | 764 | 21 | 3 | 316 | 2 | 165 | 175 | 14 |
| 3 | 11 | 0 | 236 | 225 | 17 | -3 | 6 | 1 | 286 | 246 | 11 | -2 | 17 | 1 | 272 | 247 | 10 | 1 | 6 | 2 | 184 | 199 | 7 | -3 | -317 | 2 | 85 | 87 | 25 |
| 4 | 11 | 0 | 288 | 303 | 23 | -2 | 6 | 1 | 179 | 174 | 25 | -1 | 17 | 1 | 162 | 152 | 15 | 2 | 6 | 2 | 252 | 246 | 18 | -2 | -217 | 2 | 52 | 37 | 36 |
| 0 | 12 | 0 | 585 | 527 | 37 | -1 | 6 | 1 | 187 | 202 | 12 | 0 | 17 | 1 | 76 | 18 | 14 | 3 | 6 | 2 | 154 | 123 | 12 | -1 | -117 | 2 | 91 | 86 | 25 |
| 1 | 12 | 0 | 283 | 234 | 12 | 0 | 6 | 1 | 478 | 526 | 14 | 1 | 17 | 1 | 159 | 153 | 15 | 4 | 6 | 2 | 71 | 16 | 71 | 0 | 017 | 2 | 365 | 396 | 10 |
| 2 | 12 | 0 | 344 | 321 | 15 | 1 | 6 | 1 | 170 | 202 | 6 | 2 | 17 | 1 | 280 | 248 | 10 | -3 | 7 | 2 | 204 | 172 | 11 | 1 | 117 | 2 | 87 | 86 | 26 |
| 3 | 12 | 0 | 88 | 37 | 25 | 2 | 6 | 1 | 172 | 175 | 18 | 3 | 17 | 1 | 85 | 71 | 18 | -2 | 7 | 2 | 233 | 236 | 19 | 2 | 217 | 2 | 53 | 37 | 35 |
| 4 | 12 | 0 | 169 | 156 | 21 | 3 | 6 | 1 | 284 | 246 | 15 | -3 | 18 | 1 | 96 | 80 | 16 | -1 | 7 | 2 | 144 | 136 | 16 | 3 | 17 | 2 | 80 | 87 | 20 |
| 1 | 13 | 0 | 38 | 9 | 37 | -3 | 7 | 1 | 122 | 113 | 11 | -2 | 18 | 1 | 103 | 83 | 13 | 0 | 7 | 2 | 133 | 137 | 11 | -3 | 18 | 2 | 46 | 47 | 45 |
| 2 | 13 | 0 | 97 | 124 | 22 | -2 | 7 | 1 | 399 | 369 | 17 | -1 | 18 | 1 | 147 | 118 | 15 | 1 | 7 | 2 | 123 | 135 | 6 | -2 | 18 | 2 | 128 | 146 | 12 |
| 3 | 13 | 0 | 168 | 188 | 18 | -1 | 7 | 1 | 317 | 373 | 10 | 0 | 18 | 1 | 236 | 218 | 10 | 2 | 7 | 2 | 224 | 236 | 20 | -1 | 18 | 2 | 161 | 73 | 48 |
| 4 | 13 | 0 | 88 | 4 | 40 | 0 | 7 | 1 | 185 | 170 | 7 | 1 | 18 | 1 | 119 | 118 | 24 | 3 | 7 | 2 | 201 | 172 | 10 | 0 | 18 | 2 | 153 | 126 | 16 |
| 0 | 14 | 0 | 105 | 104 | 19 | 1 | 7 | 1 | 313 | 372 | 9 | 2 | 18 | 1 | 66 | 83 | 22 | 4 | 7 | 2 | 141 | 21 | 25 | 1 | 18 | 2 | 74 | 73 | 35 |
| 1 | 14 | 0 | 164 | 161 | 11 | 2 | 7 | 1 | 368 | 369 | 28 | 3 | 18 | 1 | 46 | 80 | 46 | -4 | 8 | 2 | 97 | 21 | 38 | 2 | 18 | 2 | 156 | 145 | 13 |
| 2 | 14 | 0 | 123 | 93 | 11 | 3 | 7 | 1 | 128 | 112 | 17 | -3 | 19 | 1 | 75 | 77 | 21 | -3 | 8 | 2 | 215 | 196 | 10 | 3 | 18 | 2 | 51 | 46 | 50 |
| 3 | 14 | 0 | 89 | 139 | 27 | 4 | 7 | 1 | 146 | 100 | 24 | -2 | 19 | 1 | 115 | 124 | 11 | -2 | 8 | 2 | 211 | 197 | 12 | -3 | 19 | 2 | 20 | 9 | 20 |
| 4 | 14 | 0 | 0 | 50 | 1 | -3 | 8 | 1 | 116 | 91 | 14 | -1 | 19 | 1 | 103 | 120 | 19 | -1 | 8 | 2 | 194 | 171 | 15 | -2 | 19 | 2 | 157 | 149 | 18 |
| 1 | 15 | 0 | 83 | 60 | 30 | -2 | 8 | 1 | 104 | 93 | 13 | 0 | 19 | 1 | 175 | 179 | 11 | 0 | 8 | 2 | 561 | 743 | 24 | -1 | 19 | 2 | 143 | 167 | 14 |
| 2 | 15 | 0 | 248 | 227 | 10 | -1 | 8 | 1 | 196 | 223 | 11 | 1 | 19 | 1 | 75 | 120 | 28 | 1 | 8 | 2 | 163 | 171 | 12 | 0 | 19 | 2 | 109 | 68 | 20 |
| 3 | 15 | 0 | 283 | 273 | 15 | 0 | 8 | 1 | 115 | 77 | 14 | 2 | 19 | 1 | 118 | 124 | 11 | 2 | 8 | 2 | 217 | 196 | 19 | 1 | 19 | 2 | 119 | 167 | 16 |
| 0 | 16 | 0 | 77 | 26 | 25 | 1 | 8 | 1 | 194 | 222 | 13 | 3 | 19 | 1 | 49 | 77 | 48 | 3 | 8 | 2 | 203 | 197 | 10 | 2 | 19 | 2 | 160 | 149 | 17 |
| 1 | 16 | 0 | 40 | 24 | 40 | 2 | 8 | 1 | 107 | 92 | 12 | -2 | 20 | 1 | 215 | 209 | 11 | 4 | 8 | 2 | 110 | 22 | 32 | 3 | 19 | 2 | 55 | 9 | 41 |
| 2 | 16 | 0 | 63 | 39 | 23 | 3 | 8 | 1 | 135 | 91 | 12 | -1 | 20 | 1 | 64 | 54 | 32 | -4 | 9 | 2 | 160 | 150 | 24 | -2 | 20 | 2 | 98 | 96 | 13 |
| 3 | 16 | 0 | 62 | 93 | 61 | 4 | 8 | 1 | 101 | 62 | 36 | 0 | 20 | 1 | 69 | 84 | 27 | -3 | 9 | 2 | 183 | 181 | 12 | -1 | 20 | 2 | 0 | 92 | 1 |
| 1 | 17 | 0 | 50 | 8 | 50 | -3 | 9 | 1 | 114 | 91 | 17 | 1 | 20 | 1 | 0 | 54 | 1 | -2 | 9 | 2 | 248 | 217 | 12 | 0 | 20 | 2 | 71 | 19 | 42 |
| 2 | 17 | 0 | 58 | 46 | 57 | -2 | 9 | 1 | 122 | 115 | 16 | 2 | 20 | 1 | 207 | 209 | 14 | -1 | 9 | 2 | 146 | 110 | 18 | 1 | 20 | 2 | 45 | 93 | 45 |
| 3 | 17 | 0 | 114 | 143 | 14 | -1 | 9 | 1 | 522 | 569 | 22 | -2 | 21 | 1 | 0 | 24 | 1 | 0 | 9 | 2 | 352 | 351 | 13 | 2 | 20 | 2 | 104 | 96 | 28 |
| 0 | 18 | 0 | 100 | 100 | 25 | 0 | 9 | 1 | 556 | 610 | 16 | -1 | 21 | 1 | 77 | 97 | 21 | 1 | 9 | 2 | 136 | 111 | 15 | -2 | 21 | 2 | 112 | 116 | 19 |
| 1 | 18 | 0 | 122 | 122 | 27 | 1 | 9 | 1 | 513 | 570 | 22 | 0 | 21 | 1 | 155 | 180 | 11 | 2 | 9 | 2 | 243 | 217 | 13 | -1 | 21 | 2 | 0 | 31 | 1 |
| 2 | 18 | 0 | 65 | 44 | 29 | 2 | 9 | 1 | 126 | 116 | 11 | 1 | 21 | 1 | 83 | 97 | 19 | 3 | 9 | 2 | 185 | 180 | 13 | 0 | 21 | 2 | 64 | 29 | 31 |
| 3 | 18 | 0 | 67 | 21 | 37 | 3 | 9 | 1 | 79 | 92 | 30 | 2 | 21 | 1 | 42 | 25 | 42 | 4 | 9 | 2 | 172 | 150 | 21 | 1 | 21 | 2 | 0 | 31 | 1 |
| 1 | 19 | 0 | 67 | 82 | 34 | -4 | 10 | 1 | 132 | 78 | 27 | -1 | 22 | 1 | 72 | 95 | 22 | -4 | 10 | 2 | 93 | 53 | 39 | 2 | 21 | 2 | 127 | 116 | 17 |
| 2 | 19 | 0 | 86 | 77 | 15 | -3 | 10 | 1 | 195 | 194 | 19 | 0 | 22 | 1 | 130 | 155 | 15 | -3 | 10 | 2 | 137 | 102 | 14 | -2 | 22 | 2 | 93 | 47 | 38 |
| 3 | 19 | 0 | 44 | 9 | 43 | -2 | 10 | 1 | 262 | 248 | 10 | 1 | 22 | 1 | 54 | 95 | 54 | -2 | 10 | 2 | 97 | 72 | 16 | -1 | 22 | 2 | 50 | 67 | 43 |
| 0 | 20 | 0 | 188 | 199 | 19 | -1 | 10 | 1 | 375 | 400 | 14 | 2 | 22 | 1 | 0 | 55 | 1 | -1 | 10 | 2 | 77 | 76 | 17 | 0 | 22 | 2 | 171 | 153 | 11 |
| 1 | 20 | 0 | 0 | 42 | 1 | 0 | 10 | 1 | 690 | 690 | 21 | -1 | 23 | 1 | 120 | 133 | 12 | 0 | 10 | 2 | 950 | 934 | 28 | 1 | 22 | 2 | 40 | 67 | 40 |
| 2 | 20 | 0 | 154 | 143 | 15 | 1 | 10 | 1 | 375 | 401 | 14 | 0 | 23 | 1 | 100 | 89 | 17 | 1 | 10 | 2 | 87 | 77 | 14 | -1 | 23 | 2 | 73 | 67 | 20 |
| 1 | 21 | 0 | 54 | 48 | 41 | 2 | 10 | 1 | 264 | 247 | 10 | 1 | 23 | 1 | 93 | 133 | 21 | 2 | 10 | 2 | 70 | 71 | 35 | 0 | 23 | 2 | 56 | 89 | 56 |
| 2 | 21 | 0 | 138 | 110 | 30 | 3 | 10 | 1 | 197 | 193 | 13 | 0 | 24 | 1 | 73 | 28 | 65 | 3 | 10 | 2 | 112 | 103 | 19 | 1 | 23 | 2 | 77 | 68 | 23 |
| 0 | 22 | 0 | 127 | 96 | 19 | -4 | 11 | 1 | 35 | 51 | 35 | 0 | 0 | 2 | 730 | 648 | 22 | -4 | 11 | 2 | 118 | 83 | 30 | 1 | 0 | 3 | 1331 | 1063 | 37 |
| 1 | 22 | 0 | 46 | 3 | 46 | -3 | 11 | 1 | 50 | 25 | 50 | 1 | 0 | 2 | 696 | 652 | 18 | -3 | 11 | 2 | 200 | 176 | 15 | 2 | 0 | 3 | 191 | 167 | 10 |
| 2 | 22 | 0 | 0 | 4 | 1 | -2 | 11 | 1 | 328 | 299 | 17 | 2 | 0 | 2 | 545 | 482 | 23 | -2 | 11 | 2 | 351 | 337 | 11 | 3 | 0 | 3 | 0 | 15 | 1 |
| 1 | 23 | 0 | 56 | 47 | 55 | -1 | 11 | 1 | 242 | 239 | 11 | 3 | 0 | 2 | 143 | 121 | 29 | -1 | 11 | 2 | 276 | 254 | 11 | 4 | 0 | 3 | 61 | 39 | 38 |
| 1 | 0 | 1 | 273 | 277 | 9 | 0 | 11 | 1 | 128 | 104 | 10 | 4 | 0 | 2 | 158 | 104 | 14 | 0 | 11 | 2 | 223 | 180 | 12 | -4 | 1 | 3 | 113 | 112 | 17 |
| 2 | 0 | 1 | 277 | 257 | 12 | 1 | 11 | 1 | 247 | 240 | 10 | -4 | 1 | 2 | 120 | 115 | 17 | 1 | 11 | 2 | 258 | 253 | 11 | -3 | 1 | 3 | 63 | 76 | 63 |
| -2 | 1 | 3 | 883 | 766 | 31 | -3 | 11 | 3 | 156 | 130 | 16 | 0 | 0 | 4 | 609 | 605 | 21 | -2 | 10 | 4 | 196 | 193 | 10 | 2 | 21 | 4 | 55 | 99 | 54 |
| 0 | 1 | 3 | 237 | 298 | 5 | -2 | 11 | 3 | 131 | 124 | 8 | 1 | 0 | 4 | 1364 | 1149 | 59 | -1 | 10 | 4 | 241 | 225 | 11 | -2 | 22 | 4 | 79 | 106 | 78 |
| 1 | 1 | 3 | 2688 | 1939 | 125 | -1 | 11 | 3 | 353 | 357 | 14 | 2 | 0 | 4 | 258 | 251 | 18 | 0 | 10 | 4 | 181 | 202 | 9 | -1 | 22 | 4 | 0 | 34 | 1 |
| 2 | 1 | 3 | 881 | 767 | 54 | 0 | 11 | 3 | 308 | 330 | 12 | 3 | 0 | 4 | 124 | 63 | 36 | 1 | 10 | 4 | 247 | 224 | 10 | 0 | 22 | 4 | 60 | 3 | 43 |
| 3 | 1 | 3 | 55 | 76 | 55 | 1 | 11 | 3 | 368 | 355 | 13 | 4 | 0 | 4 | 85 | 25 | 72 | 2 | 10 | 4 | 192 | 194 | 8 | 1 | 22 | 4 | 54 | 34 | 50 |
| 4 | 1 | 3 | 86 | 112 | 73 | 2 | 11 | 3 | 130 | 124 | 18 | -4 | 1 | 4 | 155 | 142 | 14 | 3 | 10 | 4 | 83 | 75 | 27 | -1 | 23 | 4 | 52 | 28 | 52 |
| -4 | 2 | 3 | 193 | 185 | 13 | 3 | 11 | 3 | 172 | 130 | 19 | -3 | 1 | 4 | 183 | 177 | 26 | 4 | 10 | 4 | 78 | 68 | 19 | 0 | 23 | 4 | 0 | 63 | 1 |
| -3 | 2 | 3 | 158 | 182 | 30 | 4 | 11 | 3 | 123 | 118 | 15 | -2 | 1 | 4 | 330 | 312 | 11 | -4 | 11 | 4 | 107 | 84 | 32 | 1 | 23 | 4 | 92 | 29 | 31 |
| -2 | 2 | 3 | 709 | 661 | 24 | -4 | 12 | 3 | 108 | 23 | 28 | -1 | 1 | 4 | 1251 | 1117 | 36 | -3 | 11 | 4 | 145 | 166 | 16 | 1 | 0 | 5 | 1538 | 1300 | 53 |
| -1 | 2 | 3 | 718 | 720 | 18 | -3 | 12 | 3 | 85 | 72 | 27 | 0 | 1 | 4 | 148 | 164 | 5 | -2 | 11 | 4 | 129 | 128 | 9 | 2 | 0 | 5 | 212 | 190 | 16 |
| 0 | 2 | 3 | 861 | 785 | 21 | -2 | 12 | 3 | 232 | 213 | 10 | 1 | 1 | 4 | 1211 | 1116 | 37 | -1 | 11 | 4 | 639 | 621 | 22 | 3 | 0 | 5 | 110 | 101 | 16 |
| 1 | 2 | 3 | 688 | 719 | 21 | -1 | 12 | 3 | 247 | 211 | 11 | 2 | 1 | 4 | 309 | 311 | 23 | 0 | 11 | 4 | 366 | 378 | 14 | 4 | 0 | 5 | 50 | 70 | 50 |
| 2 | 2 | 3 | 682 | 660 | 45 | 0 | 12 | 3 | 416 | 436 | 19 | 3 | 1 | 4 | 186 | 177 | 7 | 1 | 11 | 4 | 633 | 621 | 28 | -4 | 1 | 5 | 94 | 60 | 51 |
| 3 | 2 | 3 | 189 | 182 | 23 | 1 | 12 | 3 | 232 | 212 | 17 | 4 | 1 | 4 | 147 | 142 | 30 | 2 | 11 | 4 | 127 | 129 | 10 | -3 | 1 | 5 | 166 | 162 | 22 |
| 4 | 2 | 3 | 163 | 185 | 30 | 2 | 12 | 3 | 261 | 213 | 10 | -4 | 2 | 4 | 91 | 65 | 21 | 3 | 11 | 4 | 169 | 167 | 14 | -2 | 1 | 5 | 184 | 163 | 9 |
| -4 | 3 | 3 | 30 | 73 | 30 | 3 | 12 | 3 | 105 | 72 | 21 | -3 | 2 | 4 | 24 | 55 | 24 | 4 | 11 | 4 | 76 | 84 | 26 | -1 | 1 | 5 | 353 | 405 | 11 |
| -3 | 3 | 3 | 270 | 222 | 17 | 4 | 12 | 3 | 50 | 24 | 50 | -2 | 2 | 4 | 502 | 497 | 17 | -4 | 12 | 4 | 126 | 129 | 25 | 1 | 1 | 5 | 353 | 405 | 9 |
| -2 | 3 | 3 | 431 | 447 | 21 | -4 | 13 | 3 | 78 | 40 | 53 | -1 | 2 | 4 | 1553 | 1343 | 44 | -3 | 12 | 4 | 157 | 174 | 13 | 2 | 1 | 5 | 160 | 164 | 18 |
| -1 | 3 | 3 | 824 | 853 | 29 | -3 | 13 | 3 | 69 | 103 | 28 | 0 | 2 | 4 | 912 | 866 | 24 | -2 | 12 | 4 | 174 | 162 | 9 | 3 | 1 | 5 | 177 | 162 | 7 |
| 0 | 3 | 3 | 12 | 22 | 12 | -2 | 13 | 3 | 114 | 129 | 11 | 1 | 2 | 4 | 1484 | 1343 | 47 | -1 | 12 | 4 | 247 | 267 | 11 | 4 | 1 | 5 | 57 | 60 | 56 |
| 1 | 3 | 3 | 845 | 854 | 22 | -1 | 13 | 3 | 362 | 354 | 14 | 2 | 2 | 4 | 440 | 497 | 34 | 0 | 12 | 4 | 0 | 15 | 1 | -4 | 2 | 5 | 239 | 234 | 21 |
| 2 | 3 | 3 | 369 | 448 | 30 | 0 | 13 | 3 | 229 | 201 | 14 | 3 | 2 | 4 | 33 | 55 | 33 | 1 | 12 | 4 | 259 | 267 | 20 | -3 | 2 | 5 | 226 | 219 | 25 |
| 3 | 3 | 3 | 261 | 223 | 16 | 1 | 13 | 3 | 357 | 352 | 11 | 4 | 2 | 4 | 0 | 65 | 1 | 2 | 12 | 4 | 177 | 162 | 9 | -2 | 2 | 5 | 545 | 510 | 18 |
| 4 | 3 | 3 | 0 | 72 | 1 | 2 | 13 | 3 | 145 | 128 | 9 | -4 | 3 | 4 | 175 | 159 | 14 | 3 | 12 | 4 | 150 | 175 | 17 | -1 | 2 | 5 | 827 | 843 | 50 |
| -4 | 4 | 3 | 167 | 219 | 28 | 3 | 13 | 3 | 113 | 104 | 18 | -3 | 3 | 4 | 197 | 181 | 27 | 4 | 12 | 4 | 137 | 129 | 15 | 0 | 2 | 5 | 539 | 558 | 14 |
| -3 | 4 | 3 | 427 | 405 | 17 | 4 | 13 | 3 | 67 | 40 | 32 | -2 | 3 | 4 | 147 | 125 | 7 | -4 | 13 | 4 | 126 | 148 | 29 | 1 | 2 | 5 | 821 | 843 | 25 |
| -2 | 4 | 3 | 285 | 265 | 11 | -4 | 14 | 3 | 28 | 46 | 27 | -1 | 3 | 4 | 580 | 609 | 24 | -3 | 13 | 4 | 66 | 64 | 32 | 2 | 2 | 5 | 501 | 510 | 36 |
| -1 | 4 | 3 | 655 | 695 | 22 | -3 | 14 | 3 | 165 | 200 | 15 | 0 | 3 | 4 | 1479 | 1230 | 40 | -2 | 13 | 4 | 231 | 228 | 9 | 3 | 2 | 5 | 217 | 219 | 10 |
| 0 | 4 | 3 | 745 | 728 | 20 | -2 | 14 | 3 | 335 | 300 | 21 | 1 | 3 | 4 | 569 | 608 | 15 | -1 | 13 | 4 | 204 | 186 | 11 | 4 | 2 | 5 | 199 | 234 | 29 |
| 1 | 4 | 3 | 643 | 694 | 17 | -1 | 14 | 3 | 224 | 190 | 9 | 2 | 3 | 4 | 117 | 125 | 20 | 0 | 13 | 4 | 183 | 142 | 13 | -4 | 3 | 5 | 194 | 196 | 15 |
| 2 | 4 | 3 | 255 | 266 | 21 | 0 | 14 | 3 | 0 | 20 | 1 | 3 | 3 | 4 | 201 | 181 | 10 | 1 | 13 | 4 | 202 | 185 | 8 | -3 | 3 | 5 | 0 | 25 | 1 |
| 3 | 4 | 3 | 430 | 406 | 16 | 1 | 14 | 3 | 214 | 190 | 10 | 4 | 3 | 4 | 140 | 160 | 34 | 2 | 13 | 4 | 257 | 228 | 7 | -2 | 3 | 5 | 377 | 386 | 22 |
| 4 | 4 | 3 | 197 | 219 | 24 | 2 | 14 | 3 | 326 | 301 | 15 | -4 | 4 | 4 | 25 | 49 | 25 | 3 | 13 | 4 | 64 | 65 | 43 | -1 | 3 | 5 | 297 | 351 | 14 |
| -4 | 5 | 3 | 93 | 140 | 53 | 3 | 14 | 3 | 160 | 199 | 28 | -3 | 4 | 4 | 247 | 224 | 14 | 4 | 13 | 4 | 171 | 148 | 14 | 0 | 3 | 5 | 232 | 241 | 7 |
| -3 | 5 | 3 | 296 | 252 | 19 | 4 | 14 | 3 | 69 | 46 | 26 | -2 | 4 | 4 | 282 | 307 | 13 | -4 | 14 | 4 | 80 | 97 | 54 | 1 | 3 | 5 | 300 | 352 | 13 |
| -2 | 5 | 3 | 65 | 54 | 16 | -3 | 15 | 3 | 92 | 115 | 21 | -1 | 4 | 4 | 566 | 591 | 19 | -3 | 14 | 4 | 100 | 85 | 17 | 2 | 3 | 5 | 342 | 384 | 25 |
| -1 | 5 | 3 | 623 | 686 | 16 | -2 | 15 | 3 | 193 | 171 | 9 | 0 | 4 | 4 | 249 | 269 | 7 | -2 | 14 | 4 | 190 | 201 | 10 | 3 | 3 | 5 | 42 | 25 | 41 |
| 0 | 5 | 3 | 360 | 383 | 11 | -1 | 15 | 3 | 307 | 285 | 13 | 1 | 4 | 4 | 551 | 591 | 15 | -1 | 14 | 4 | 277 | 261 | 10 | 4 | 3 | 5 | 209 | 195 | 25 |
| 1 | 5 | 3 | 620 | 685 | 16 | 0 | 15 | 3 | 60 | 54 | 25 | 2 | 4 | 4 | 264 | 307 | 21 | 0 | 14 | 4 | 0 | 25 | 1 | -4 | 4 | 5 | 88 | 99 | 27 |
| 2 | 5 | 3 | 0 | 55 | 1 | 1 | 15 | 3 | 311 | 285 | 13 | 3 | 4 | 4 | 261 | 224 | 11 | 1 | 14 | 4 | 286 | 262 | 17 | -3 | 4 | 5 | 231 | 244 | 30 |
| 3 | 5 | 3 | 288 | 252 | 13 | 2 | 15 | 3 | 198 | 171 | 10 | 4 | 4 | 4 | 59 | 48 | 59 | 2 | 14 | 4 | 187 | 200 | 9 | -2 | 4 | 5 | 246 | 225 | 12 |
| 4 | 5 | 3 | 88 | 140 | 64 | 3 | 15 | 3 | 90 | 116 | 29 | -4 | 5 | 4 | 131 | 93 | 13 | 3 | 14 | 4 | 69 | 84 | 34 | -1 | 4 | 5 | 204 | 241 | 7 |
| -4 | 6 | 3 | 1.05 | 78 | 32 | -3 | 16 | 3 | 0 | 30 | 1 | -3 | 5 | 4 | 192 | 167 | 12 | 4 | 14 | 4 | 76 | 97 | 22 | 0 | 4 | 5 | 518 | 540 | 16 |
| -3 | 6 | 3 | 328 | 334 | 12 | -2 | 16 | 3 | 115 | 84 | 12 | -2 | 5 | 4 | 285 | 263 | 13 | -3 | 15 | 4 | 66 | 55 | 44 | 1 | 4 | 5 | 207 | 242 | 6 |
| -2 | 6 | 3 | 443 | 439 | 20 | -1 | 16 | 3 | 194 | 185 | 13 | -1 | 5 | 4 | 129 | 136 | 8 | -2 | 15 | 4 | 365 | 355 | 12 | 2 | 4 | 5 | 242 | 225 | 18 |
| -1 | 6 | 3 | 306 | 362 | 15 | 0 | 16 | 3 | 61 | 49 | 38 | 0 | 5 | 4 | 165 | 169 | 7 | -1 | 15 | 4 | 170 | 143 | 19 | 3 | 4 | 5 | 269 | 244 | 11 |
| 0 | 6 | 3 | 350 | 407 | 11 | 1 | 16 | 3 | 180 | 185 | 16 | 1 | 5 | 4 | 140 | 137 | 5 | 0 | 15 | 4 | 163 | 141 | 8 | 4 | 4 | 5 | 75 | 100 | 75 |
| 1 | 6 | 3 | 308 | 362 | 8 | 2 | 16 | 3 | 89 | 84 | 19 | 2 | 5 | 4 | 255 | 262 | 22 | 1 | 15 | 4 | 145 | 142 | 12 | -4 | 5 | 5 | 150 | 147 | 14 |
| 2 | 6 | 3 | 449 | 440 | 28 | 3 | 16 | 3 | 0 | 30 | 1 | 3 | 5 | 4 | 200 | 168 | 11 | 2 | 15 | 4 | 374 | 356 | 13 | -3 | 5 | 5 | 68 | 52 | 19 |
| 3 | 6 | 3 | 331 | 334 | 13 | -3 | 17 | 3 | 108 | 104 | 45 | 4 | 5 | 4 | 77 | 93 | 77 | 3 | 15 | 4 | 36 | 55 | 36 | -2 | 5 | 5 | 141 | 109 | 10 |
| 4 | 6 | 3 | 0 | 78 | 1 | -2 | 17 | 3 | 95 | 79 | 15 | -4 | 6 | 4 | 128 | 89 | 33 | -3 | 16 | 4 | 104 | 97 | 17 | -1 | 5 | 5 | 58 | 69 | 11 |
| -4 | 7 | 3 | 144 | 112 | 24 | -1 | 17 | 3 | 121 | 85 | 20 | -3 | 6 | 4 | 211 | 179 | 11 | -2 | 16 | 4 | 222 | 202 | 9 | 0 | 5 | 5 | 356 | 366 | 11 |
| -3 | 7 | 3 | 143 | 125 | 15 | 0 | 17 | 3 | 223 | 184 | 9 | -2 | 6 | 4 | 568 | 553 | 37 | -1 | 16 | 4 | 192 | 188 | 15 | 1 | 5 | 5 | 63 | 68 | 8 |
| -2 | 7 | 3 | 283 | 250 | 17 | 1 | 17 | 3 | 103 | 85 | 21 | -1 | 6 | 4 | 195 | 211 | 19 | 0 | 16 | 4 | 134 | 150 | 9 | 2 | 5 | 5 | 120 | 109 | 23 |
| -1 | 7 | 3 | 322 | 398 | 11 | 2 | 17 | 3 | 87 | 79 | 16 | 0 | 6 | 4 | 616 | 607 | 18 | 1 | 16 | 4 | 186 | 188 | 14 | 3 | 5 | 5 | 70 | 52 | 38 |
| 0 | 7 | 3 | 0 | 13 | 1 | 3 | 17 | 3 | 88 | 104 | 19 | 1 | 6 | 4 | 196 | 210 | 6 | 2 | 16 | 4 | 231 | 203 | 10 | 4 | 5 | 5 | 130 | 148 | 35 |
| 1 | 7 | 3 | 315 | 397 | 9 | -3 | 18 | 3 | 158 | 169 | 19 | 2 | 6 | 4 | 578 | 553 | 35 | 3 | 16 | 4 | 56 | 97 | 48 | -4 | 6 | 5 | 159 | 142 | 15 |
| 2 | 7 | 3 | 269 | 251 | 21 | -2 | 18 | 3 | 79 | 73 | 18 | 3 | 6 | 4 | 177 | 179 | 12 | -3 | 17 | 4 | 44 | 71 | 44 | -3 | 6 | 5 | 294 | 243 | 11 |
| 3 | 7 | 3 | 136 | 125 | 12 | -1 | 18 | 3 | 196 | 135 | 19 | 4 | 6 | 4 | 69 | 89 | 68 | -2 | 17 | 4 | 127 | 109 | 11 | -2 | 6 | 5 | 340 | 329 | 18 |
| -4 | 8 | 3 | 111 | 95 | 32 | 0 | 18 | 3 | 0 | 17 | 1 | -4 | 7 | 4 | 81 | 88 | 28 | -1 | 17 | 4 | 233 | 230 | 15 | -1 | 6 | 5 | 562 | 622 | 22 |
| -3 | 8 | 3 | 219 | 191 | 11 | 1 | 18 | 3 | 145 | 135 | 21 | -3 | 7 | 4 | 184 | 153 | 9 | 0 | 17 | 4 | 231 | 204 | 14 | 0 | 6 | 5 | 941 | 879 | 28 |
| -2 | 8 | 3 | 284 | 259 | 14 | 2 | 18 | 3 | 54 | 73 | 54 | -2 | 7 | 4 | 205 | 180 | 11 | 1 | 17 | 4 | 243 | 230 | 14 | 1 | 6 | 5 | 569 | 622 | 15 |
| -1 | 8 | 3 | 169 | 174 | 16 | 3 | 18 | 3 | 178 | 170 | 11 | -1 | 7 | 4 | 311 | 377 | 19 | 2 | 17 | 4 | 114 | 109 | 12 | 2 | 6 | 5 | 351 | 329 | 23 |
| 0 | 8 | 3 | 56 | 67 | 16 | -3 | 19 | 3 | 27 | 63 | 26 | 0 | 7 | 4 | 179 | 176 | 7 | 3 | 17 | 4 | 51 | 72 | 51 | 3 | 6 | 5 | 263 | 243 | 13 |
| 1 | 8 | 3 | 156 | 174 | 9 | -2 | 19 | 3 | 91 | 52 | 25 | 1 | 7 | 4 | 318 | 376 | 8 | -3 | 18 | 4 | 126 | 120 | 36 | 4 | 6 | 5 | 127 | 142 | 38 |
| 2 | 8 | 3 | 293 | 259 | 21 | -1 | 19 | 3 | 180 | 152 | 20 | 2 | 7 | 4 | 177 | 181 | 22 | -2 | 18 | 4 | 88 | 68 | 16 | -4 | 7 | 5 | 122 | 137 | 16 |
| 3 | 8 | 3 | 207 | 191 | 10 | 0 | 19 | 3 | 86 | 24 | 20 | 3 | 7 | 4 | 173 | 152 | 44 | -1 | 18 | 4 | 350 | 341 | 17 | -3 | 7 | 5 | 297 | 305 | 13 |
| 4 | 8 | 3 | 138 | 95 | 25 | 1 | 19 | 3 | 154 | 153 | 14 | -4 | 8 | 4 | 71 | 32 | 71 | 0 | 18 | 4 | 88 | 117 | 19 | -2 | 7 | 5 | 71 | 64 | 20 |
| -4 | 9 | 3 | 157 | 129 | 24 | 2 | 19 | 3 | 80 | 52 | 27 | -3 | 8 | 4 | 116 | 106 | 17 | 1 | 18 | 4 | 337 | 341 | 18 | -1 | 7 | 5 | 376 | 436 | 24 |
| -3 | 9 | 3 | 83 | 76 | 32 | 3 | 19 | 3 | 78 | 63 | 21 | -2 | 8 | 4 | 83 | 58 | 18 | 2 | 18 | 4 | 38 | 68 | 37 | 0 | 7 | 5 | 389 | 456 | 14 |
| -2 | 9 | 3 | 117 | 95 | 13 | -2 | 20 | 3 | 74 | 55 | 17 | -1 | 8 | 4 | 258 | 239 | 21 | 3 | 18 | 4 | 120 | 120 | 14 | 1 | 7 | 5 | 394 | 436 | 11 |
| -1 | 9 | 3 | 442 | 456 | 15 | -1 | 20 | 3 | 102 | 102 | 17 | 0 | 8 | 4 | 145 | 138 | 10 | -3 | 19 | 4 | 64 | 37 | 64 | 2 | 7 | 5 | 64 | 65 | 64 |
| 0 | 9 | 3 | 61 | 69 | 16 | 0 | 20 | 3 | 108 | 69 | 31 | 1 | 8 | 4 | 259 | 238 | 11 | -2 | 19 | 4 | 85 | 89 | 16 | 3 | 7 | 5 | 312 | 305 | 13 |
| 1 | 9 | 3 | 440 | 457 | 17 | 1 | 20 | 3 | 83 | 102 | 19 | 2 | 8 | 4 | 64 | 58 | 63 | -1 | 19 | 4 | 151 | 158 | 13 | 4 | 7 | 5 | 132 | 137 | 34 |
| 2 | 9 | 3 | 110 | 94 | 31 | 2 | 20 | 3 | 74 | 54 | 74 | 3 | 8 | 4 | 82 | 105 | 23 | 0 | 19 | 4 | 342 | 328 | 12 | -4 | 8 | 5 | 71 | 47 | 27 |
| 3 | 9 | 3 | 109 | 77 | 18 | -2 | 21 | 3 | 77 | 50 | 27 | 4 | 8 | 4 | 0 | 32 | 1 | 1 | 19 | 4 | 146 | 158 | 16 | -3 | 8 | 5 | 111 | 96 | 17 |
| 4 | 9 | 3 | 161 | 129 | 23 | -1 | 21 | 3 | 101 | 115 | 21 | -4 | 9 | 4 | 61 | 70 | 60 | 2 | 19 | 4 | 112 | 88 | 16 | -2 | 8 | 5 | 167 | 148 | 14 |
| -4 | 10 | 3 | 77 | 59 | 64 | 0 | 21 | 3 | 334 | 357 | 12 | -3 | 9 | 4 | 118 | 111 | 17 | 3 | 19 | 4 | 68 | 36 | 68 | -1 | 8 | 5 | 335 | 365 | 21 |
| -3 | 10 | 3 | 195 | 187 | 14 | 1 | 21 | 3 | 86 | 116 | 19 | -2 | 9 | 4 | 141 | 100 | 12 | -2 | 20 | 4 | 130 | 122 | 10 | 0 | 8 | 5 | 532 | 614 | 16 |
| -2 | 10 | 3 | 244 | 211 | 8 | 2 | 21 | 3 | 94 | 51 | 21 | -1 | 9 | 4 | 259 | 237 | 16 | -1 | 20 | 4 | 110 | 103 | 16 | 1 | 8 | 5 | 346 | 365 | 22 |
| -1 | 10 | 3 | 201 | 177 | 10 | -1 | 22 | 3 | 91 | 97 | 20 | 0 | 9 | 4 | 692 | 686 | 21 | 0 | 20 | 4 | 185 | 173 | 10 | 2 | 8 | 5 | 120 | 148 | 30 |
| 0 | 10 | 3 | 74 | 69 | 14 | 0 | 22 | 3 | 154 | 148 | 12 | 1 | 9 | 4 | 253 | 238 | 10 | 1 | 20 | 4 | 84 | 103 | 19 | 3 | 8 | 5 | 95 | 96 | 19 |
| 1 | 10 | 3 | 197 | 178 | 10 | 1 | 22 | 3 | 72 | 97 | 25 | 2 | 9 | 4 | 136 | 100 | 25 | 2 | 20 | 4 | 105 | 121 | 20 | 4 | 8 | 5 | 44 | 48 | 44 |
| 2 | 10 | 3 | 245 | 211 | 9 | 2 | 22 | 3 | 133 | 39 | 26 | 3 | 9 | 4 | 127 | 110 | 16 | -2 | 21 | 4 | 98 | 100 | 11 | -4 | 9 | 5 | 80 | 68 | 69 |
| 3 | 10 | 3 | 183 | 188 | 14 | -1 | 23 | 3 | 107 | 109 | 19 | 4 | 9 | 4 | 103 | 69 | 36 | -1 | 21 | 4 | 222 | 213 | 15 | -3 | 9 | 5 | 90 | 87 | 23 |
| 4 | 10 | 3 | 95 | 59 | 41 | 0 | 23 | 3 | 85 | 21 | 27 | -4 | 10 | 4 | 83 | 68 | 50 | 0 | 21 | 4 | 157 | 165 | 11 | -2 | 9 | 5 | 200 | 164 | 9 |
| -4 | 11 | 3 | 123 | 119 | 28 | 1 | 23 | 3 | 111 | 109 | 19 | -3 | 10 | 4 | 84 | 75 | 25 | 1 | 21 | 4 | 212 | 213 | 29 | -1 | 9 | 5 | 101 | 96 | 12 |
| 0 | 9 | 5 | 428 | 468 | 13 | 1 | 20 | 5 | 53 | 77 | 52 | 3 | 8 | 6 | 61 | 34 | 39 | -2 | 20 | 6 | 72 | 78 | 19 | 0 | 8 | 7 | 153 | 138 | 12 |
| 1 | 9 | 5 | 67 | 98 | 19 | 2 | 20 | 5 | 185 | 163 | 21 | 4 | 8 | 6 | 168 | 155 | 21 | -1 | 20 | 6 | 65 | 69 | 23 | 1 | 8 | 7 | 606 | 588 | 24 |
| 2 | 9 | 5 | 170 | 165 | 17 | -2 | 21 | 5 | 64 | 67 | 18 | -4 | 9 | 6 | 93 | 83 | 40 | 0 | 20 | 6 | 39 | 20 | 38 | 2 | 8 | 7 | 193 | 157 | 20 |
| 3 | 9 | 5 | 97 | 87 | 20 | -1 | 21 | 5 | 117 | 88 | 20 | -3 | 9 | 6 | 148 | 142 | 23 | 1 | 20 | 6 | 64 | 69 | 28 | 3 | 8 | 7 | 203 | 187 | 12 |
| 4 | 9 | 5 | 50 | 68 | 50 | 0 | 21 | 5 | 86 | 21 | 22 | -2 | 9 | 6 | 334 | 327 | 10 | 2 | 20 | 6 | 77 | 78 | 26 | 4 | 8 | 7 | 81 | 59 | 18 |
| -4 | 10 | 5 | 80 | 79 | 56 | 1 | 21 | 5 | 96 | 89 | 26 | -1 | 9 | 6 | 429 | 454 | 21 | -2 | 21 | 6 | 38 | 48 | 37 | -4 | 9 | 7 | 206 | 236 | 18 |
| -3 | 10 | 5 | 87 | 107 | 87 | 2 | 21 | 5 | 50 | 67 | 49 | 0 | 9 | 6 | 210 | 205 | 8 | -1 | 21 | 6 | 165 | 177 | 20 | -3 | 9 | 7 | 77 | 96 | 35 |
| -2 | 10 | 5 | 136 | 116 | 23 | -1 | 22 | 5 | 50 | 2 | 50 | 1 | 9 | 6 | 442 | 455 | 15 | 0 | 21 | 6 | 28 | 28 | 27 | -2 | 9 | 7 | 170 | 158 | 7 |
| -1 | 10 | 5 | 213 | 215 | 10 | 0 | 22 | 5 | 0 | 26 | 1 | 2 | 9 | 6 | 348 | 326 | 38 | 1 | 21 | 6 | 135 | 177 | 13 | -1 | 9 | 7 | 554 | 575 | 20 |
| 0 | 10 | 5 | 143 | 156 | 9 | 1 | 22 | 5 | 12 | 2 | 11 | 3 | 9 | 6 | 167 | 143 | 13 | 2 | 21 | 6 | 36 | 47 | 36 | 0 | 9 | 7 | 192 | 205 | 8 |
| 1 | 10 | 5 | 214 | 214 | 10 | -1 | 23 | 5 | 122 | 106 | 12 | 4 | 9 | 6 | 98 | 83 | 15 | -1 | 22 | 6 | 149 | 175 | 11 | 1 | 9 | 7 | 582 | 575 | 24 |
| 2 | 10 | 5 | 84 | 116 | 23 | 0 | 23 | 5 | 0 | 23 | 1 | -4 | 10 | 6 | 89 | 78 | 50 | 0 | 22 | 6 | 0 | 5 | 1 | 2 | 9 | 7 | 171 | 158 | 12 |
| 3 | 10 | 5 | 103 | 106 | 21 | 1 | 23 | 5 | 121 | 105 | 14 | -3 | 10 | 6 | 34 | 37 | 34 | 1 | 22 | 6 | 172 | 175 | 12 | 3 | 9 | 7 | 106 | 96 | 18 |
| 4 | 10 | 5 | 95 | 79 | 16 | 0 | 0 | 6 | 129 | 145 | 6 | -2 | 10 | 6 | 139 | 145 | 8 | -1 | 23 | 6 | 73 | 67 | 37 | 4 | 9 | 7 | 248 | 236 | 12 |
| -4 | 11 | 5 | 0 | 47 | 1 | 1 | 0 | 6 | 172 | 176 | 11 | -1 | 10 | 6 | 260 | 235 | 20 | 0 | 23 | 6 | 92 | 91 | 24 | -4 | 10 | 7 | 113 | 124 | 24 |
| -3 | 11 | 5 | 0 | 26 | 1 | 2 | 0 | 6 | 0 | 14 | 1 | 0 | 10 | 6 | 171 | 165 | 10 | 1 | 23 | 6 | 65 | 67 | 29 | -3 | 10 | 7 | 149 | 142 | 14 |
| -2 | 11 | 5 | 264 | 252 | 9 | 3 | 0 | 6 | 99 | 89 | 11 | 1 | 10 | 6 | 242 | 236 | 13 | 1 | 0 | 7 | 452 | 500 | 15 | -2 | 10 | 7 | 145 | 147 | 12 |
| -1 | 11 | 5 | 162 | 141 | 12 | 4 | 0 | 6 | 30 | 38 | 29 | 2 | 10 | 6 | 163 | 146 | 12 | 2 | 0 | 7 | 124 | 86 | 17 | -1 | 10 | 7 | 382 | 391 | 14 |
| 0 | 11 | 5 | 0 | 7 | 1 | -4 | 1 | 6 | 79 | 50 | 78 | 3 | 10 | 6 | 44 | 38 | 43 | 3 | 0 | 7 | 0 | 17 | 1 | 0 | 10 | 7 | 440 | 419 | 16 |
| 1 | 11 | 5 | 156 | 141 | 13 | -3 | 1 | 6 | 251 | 245 | 28 | 4 | 10 | 6 | 85 | 78 | 20 | 4 | 0 | 7 | 0 | 55 | 1 | 1 | 10 | 7 | 396 | 390 | 17 |
| 2 | 11 | 5 | 272 | 253 | 13 | -2 | 1 | 6 | 215 | 195 | 10 | -4 | 11 | 6 | 103 | 83 | 23 | -4 | 1 | 7 | 0 | 86 | 1 | 2 | 10 | 7 | 141 | 147 | 14 |
| 3 | 11 | 5 | 0 | 26 | 1 | -1 | 1 | 6 | 367 | 431 | 11 | -3 | 11 | 6 | 78 | 87 | 59 | -3 | 1 | 7 | 63 | 60 | 63 | 3 | 10 | 7 | 175 | 141 | 14 |
| 4 | 11 | 5 | 0 | 47 | 1 | 0 | 1 | 6 | 226 | 263 | 6 | -2 | 11 | 6 | 238 | 221 | 8 | -2 | 1 | 7 | 383 | 375 | 13 | 4 | 10 | 7 | 151 | 124 | 12 |
| -4 | 12 | 5 | 0 | 40 | 1 | 1 | 1 | 6 | 378 | 430 | 10 | -1 | 11 | 6 | 214 | 203 | 10 | -1 | 1 | 7 | 312 | 379 | 10 | -4 | 11 | 7 | 107 | 92 | 21 |
| -3 | 12 | 5 | 121 | 118 | 38 | 2 | 1 | 6 | 195 | 194 | 17 | 0 | 11 | 6 | 153 | 142 | 11 | 0 | 1 | 7 | 753 | 753 | 23 | -3 | 11 | 7 | 43 | 72 | 43 |
| -2 | 12 | 5 | 235 | 223 | 8 | 3 | 1 | 6 | 242 | 245 | 11 | 1 | 11 | 6 | 200 | 203 | 13 | 1 | 1 | 7 | 309 | 379 | 10 | -2 | 11 | 7 | 238 | 235 | 7 |
| -1 | 12 | 5 | 301 | 282 | 12 | 4 | 1 | 6 | 24 | 51 | 23 | 2 | 11 | 6 | 223 | 221 | 10 | 2 | 1 | 7 | 358 | 375 | 26 | -1 | 11 | 7 | 313 | 312 | 12 |
| 0 | 12 | 5 | 113 | 115 | 17 | -4 | 2 | 6 | 115 | 110 | 39 | 3 | 11 | 6 | 81 | 87 | 31 | 3 | 1 | 7 | 0 | 61 | 1 | 0 | 11 | 7 | 101 | 102 | 14 |
| 1 | 12 | 5 | 291 | 282 | 15 | -3 | 2 | 6 | 169 | 163 | 21 | 4 | 11 | 6 | 71 | 83 | 25 | 4 | 1 | 7 | 50 | 86 | 50 | 1 | 11 | 7 | 304 | 312 | 16 |
| 2 | 12 | 5 | 238 | 222 | 8 | -2 | 2 | 6 | 243 | 247 | 9 | -4 | 12 | 6 | 74 | 8 | 50 | -4 | 2 | 7 | 0 | 41 | 1 | 2 | 11 | 7 | 230 | 234 | 10 |
| 3 | 12 | 5 | 129 | 118 | 23 | -1 | 2 | 6 | 869 | 904 | 38 | -3 | 12 | 6 | 34 | 16 | 33 | -3 | 2 | 7 | 227 | 243 | 17 | 3 | 11 | 7 | 14 | 72 | 13 |
| 4 | 12 | 5 | 0 | 40 | 1 | 0 | 2 | 6 | 141 | 143 | 5 | -2 | 12 | 6 | 106 | 108 | 10 | -2 | 2 | 7 | 412 | 384 | 18 | 4 | 11 | 7 | 99 | 92 | 17 |
| -4 | 13 | 5 | 125 | 126 | 26 | 1 | 2 | 6 | 877 | 903 | 30 | -1 | 12 | 6 | 295 | 291 | 11 | -1 | 2 | 7 | 221 | 229 | 17 | -4 | 12 | 7 | 162 | 173 | 18 |
| -3 | 13 | 5 | 170 | 200 | 12 | 2 | 2 | 6 | 226 | 247 | 19 | 0 | 12 | 6 | 58 | 46 | 46 | 0 | 2 | 7 | 409 | 440 | 12 | -3 | 12 | 7 | 130 | 151 | 14 |
| -2 | 13 | 5 | 161 | 154 | 8 | 3 | 2 | 6 | 174 | 164 | 9 | 1 | 12 | 6 | 302 | 291 | 12 | 1 | 2 | 7 | 223 | 228 | 11 | -2 | 12 | 7 | 264 | 228 | 8 |
| -1 | 13 | 5 | 389 | 384 | 14 | 4 | 2 | 6 | 120 | 109 | 36 | 2 | 12 | 6 | 100 | 109 | 19 | 2 | 2 | 7 | 429 | 384 | 26 | -1 | 12 | 7 | 328 | 309 | 12 |
| 0 | 13 | 5 | 110 | 91 | 19 | -4 | 3 | 6 | 36 | 37 | 36 | 3 | 12 | 6 | 23 | 17 | 22 | 3 | 2 | 7 | 233 | 243 | 10 | 0 | 12 | 7 | 70 | 116 | 40 |
| 1 | 13 | 5 | 377 | 384 | 11 | -3 | 3 | 6 | 183 | 185 | 14 | 4 | 12 | 6 | 0 | 8 | 1 | 4 | 2 | 7 | 0 | 42 | 1 | 1 | 12 | 7 | 310 | 309 | 11 |
| 2 | 13 | 5 | 167 | 154 | 8 | -2 | 3 | 6 | 556 | 546 | 33 | -4 | 13 | 6 | 37 | 62 | 36 | -4 | 3 | 7 | 48 | 89 | 48 | 2 | 12 | 7 | 246 | 227 | 11 |
| 3 | 13 | 5 | 204 | 200 | 18 | -1 | 3 | 6 | 639 | 665 | 26 | -3 | 13 | 6 | 122 | 91 | 44 | -3 | 3 | 7 | 118 | 128 | 18 | 3 | 12 | 7 | 148 | 151 | 19 |
| 4 | 13 | 5 | 92 | 126 | 23 | 0 | 3 | 6 | 622 | 615 | 16 | -2 | 13 | 6 | 79 | 64 | 19 | -2 | 3 | 7 | 289 | 283 | 19 | 4 | 12 | 7 | 203 | 172 | 16 |
| -4 | 14 | 5 | 88 | 65 | 42 | 1 | 3 | 6 | 621 | 664 | 19 | -1 | 13 | 6 | 272 | 248 | 10 | -1 | 3 | 7 | 216 | 235 | 6 | -4 | 13 | 7 | 49 | 64 | 49 |
| -3 | 14 | 5 | 142 | 145 | 37 | 2 | 3 | 6 | 536 | 547 | 35 | 0 | 13 | 6 | 106 | 103 | 13 | 0 | 3 | 7 | 107 | 109 | 7 | -3 | 13 | 7 | 150 | 175 | 18 |
| -2 | 14 | 5 | 206 | 207 | 10 | 3 | 3 | 6 | 205 | 185 | 9 | 1 | 13 | 6 | 271 | 247 | 9 | 1 | 3 | 7 | 210 | 234 | 6 | -2 | 13 | 7 | 13 | 67 | 12 |
| -1 | 14 | 5 | 95 | 104 | 15 | 4 | 3 | 6 | 88 | 37 | 62 | 2 | 13 | 6 | 62 | 64 | 28 | 2 | 3 | 7 | 291 | 281 | 20 | -1 | 13 | 7 | 117 | 120 | 12 |
| 0 | 14 | 5 | 190 | 197 | 8 | -4 | 4 | 6 | 149 | 155 | 12 | 3 | 13 | 6 | 89 | 90 | 24 | 3 | 3 | 7 | 141 | 129 | 15 | 0 | 13 | 7 | 74 | 67 | 20 |
| 1 | 14 | 5 | 105 | 104 | 13 | -3 | 4 | 6 | 297 | 245 | 25 | 4 | 13 | 6 | 74 | 62 | 28 | 4 | 3 | 7 | 115 | 89 | 38 | 1 | 13 | 7 | 125 | 120 | 9 |
| 2 | 14 | 5 | 212 | 208 | 9 | -2 | 4 | 6 | 547 | 550 | 23 | -3 | 14 | 6 | 234 | 298 | 26 | -4 | 4 | 7 | 165 | 165 | 19 | 2 | 13 | 7 | 43 | 67 | 42 |
| 3 | 14 | 5 | 143 | 145 | 15 | -1 | 4 | 6 | 122 | 130 | 6 | -2 | 14 | 6 | 183 | 161 | 13 | -3 | 4 | 7 | 336 | 294 | 31 | 3 | 13 | 7 | 173 | 175 | 13 |
| 4 | 14 | 5 | 73 | 65 | 28 | 0 | 4 | 6 | 270 | 306 | 9 | -1 | 14 | 6 | 0 | 40 | 1 | -2 | 4 | 7 | 407 | 419 | 17 | 4 | 13 | 7 | 88 | 65 | 22 |
| -3 | 15 | 5 | 18 | 60 | 17 | 1 | 4 | 6 | 118 | 129 | 5 | 0 | 14 | 6 | 184 | 178 | 8 | -1 | 4 | 7 | 213 | 237 | 14 | -3 | 14 | 7 | 74 | 42 | 25 |
| -2 | 15 | 5 | 288 | 249 | 11 | 2 | 4 | 6 | 543 | 549 | 34 | 1 | 14 | 6 | 64 | 39 | 23 | 0 | 4 | 7 | 160 | 175 | 7 | -2 | 14 | 7 | 48 | 68 | 47 |
| -1 | 15 | 5 | 170 | 151 | 12 | 3 | 4 | 6 | 268 | 244 | 12 | 2 | 14 | 6 | 165 | 161 | 11 | 1 | 4 | 7 | 210 | 237 | 6 | -1 | 14 | 7 | 287 | 284 | 10 |
| 0 | 15 | 5 | 143 | 134 | 9 | 4 | 4 | 6 | 136 | 155 | 33 | 3 | 14 | 6 | 255 | 298 | 14 | 2 | 4 | 7 | 392 | 419 | 27 | 0 | 14 | 7 | 102 | 96 | 11 |
| 1 | 15 | 5 | 175 | 151 | 10 | -4 | 5 | 6 | 194 | 189 | 22 | -3 | 15 | 6 | 103 | 110 | 18 | 3 | 4 | 7 | 320 | 295 | 13 | 1 | 14 | 7 | 293 | 285 | 10 |
| 2 | 15 | 5 | 300 | 249 | 14 | -3 | 5 | 6 | 74 | 88 | 16 | -2 | 15 | 6 | 168 | 159 | 9 | 4 | 4 | 7 | 141 | 165 | 33 | 2 | 14 | 7 | 0 | 68 | 1 |
| 3 | 15 | 5 | 54 | 60 | 38 | -2 | 5 | 6 | 201 | 189 | 11 | -1 | 15 | 6 | 156 | 148 | 12 | -4 | 5 | 7 | 195 | 199 | 38 | 3 | 14 | 7 | 53 | 42 | 53 |
| -3 | 16 | 5 | 111 | 66 | 22 | -1 | 5 | 6 | 462 | 479 | 14 | 0 | 15 | 6 | 137 | 133 | 9 | -3 | 5 | 7 | 128 | 107 | 9 | -3 | 15 | 7 | 216 | 234 | 59 |
| -2 | 16 | 5 | 394 | 383 | 12 | 0 | 5 | 6 | 458 | 503 | 14 | 1 | 15 | 6 | 151 | 149 | 11 | -2 | 5 | 7 | 426 | 404 | 19 | -2 | 15 | 7 | 265 | 246 | 9 |
| -1 | 16 | 5 | 142 | 132 | 16 | 1 | 5 | 6 | 462 | 480 | 12 | 2 | 15 | 6 | 180 | 159 | 15 | -1 | 5 | 7 | 304 | 411 | 18 | -1 | 15 | 7 | 100 | 102 | 17 |
| 0 | 16 | 5 | 51 | 32 | 34 | 2 | 5 | 6 | 208 | 189 | 18 | 3 | 15 | 6 | 113 | 110 | 14 | 0 | 5 | 7 | 649 | 688 | 19 | 0 | 15 | 7 | 35 | 43 | 35 |
| 1 | 16 | 5 | 142 | 132 | 15 | 3 | 5 | 6 | 76 | 87 | 23 | -3 | 16 | 6 | 68 | 55 | 30 | 1 | 5 | 7 | 339 | 409 | 9 | 1 | 15 | 7 | 96 | 101 | 15 |
| 2 | 16 | 5 | 394 | 383 | 13 | 4 | 5 | 6 | 165 | 189 | 30 | -2 | 16 | 6 | 233 | 208 | 10 | 2 | 5 | 7 | 424 | 405 | 28 | 2 | 15 | 7 | 259 | 246 | 21 |
| 3 | 16 | 5 | 85 | 65 | 22 | -4 | 6 | 6 | 141 | 141 | 13 | -1 | 16 | 6 | 174 | 158 | 14 | 3 | 5 | 7 | 151 | 107 | 16 | 3 | 15 | 7 | 221 | 234 | 10 |
| -3 | 17 | 5 | 154 | 169 | 35 | -3 | 6 | 6 | 83 | 50 | 17 | 0 | 16 | 6 | 48 | 4 | 43 | 4 | 5 | 7 | 160 | 199 | 30 | -3 | 16 | 7 | 120 | 90 | 15 |
| -2 | 17 | 5 | 207 | 189 | 9 | -2 | 6 | 6 | 225 | 209 | 12 | 1 | 16 | 6 | 182 | 157 | 13 | -4 | 6 | 7 | 109 | 93 | 24 | -2 | 16 | 7 | 181 | 157 | 9 |
| -1 | 17 | 5 | 151 | 161 | 16 | -1 | 6 | 6 | 139 | 147 | 10 | 2 | 16 | 6 | 254 | 207 | 20 | -3 | 6 | 7 | 102 | 112 | 13 | -1 | 16 | 7 | 141 | 122 | 16 |
| 0 | 17 | 5 | 212 | 195 | 12 | 1 | 6 | 6 | 138 | 148 | 11 | 3 | 16 | 6 | 62 | 55 | 38 | -2 | 6 | 7 | 299 | 290 | 14 | 0 | 16 | 7 | 308 | 304 | 9 |
| 1 | 17 | 5 | 149 | 161 | 15 | 2 | 6 | 6 | 222 | 208 | 20 | -3 | 17 | 6 | 108 | 88 | 25 | -1 | 6 | 7 | 464 | 491 | 41 | 1 | 16 | 7 | 129 | 122 | 16 |
| 2 | 17 | 5 | 195 | 189 | 10 | 3 | 6 | 6 | 56 | 51 | 44 | -2 | 17 | 6 | 21 | 16 | 20 | 0 | 6 | 7 | 476 | 496 | 17 | 2 | 16 | 7 | 172 | 156 | 21 |
| 3 | 17 | 5 | 169 | 169 | 12 | 4 | 6 | 6 | 152 | 141 | 29 | -1 | 17 | 6 | 241 | 232 | 18 | 1 | 6 | 7 | 477 | 493 | 12 | 3 | 16 | 7 | 72 | 91 | 25 |
| -3 | 18 | 5 | 135 | 110 | 39 | -4 | 7 | 6 | 0 | 40 | 1 | 0 | 17 | 6 | 435 | 454 | 15 | 2 | 6 | 7 | 311 | 290 | 21 | -3 | 17 | 7 | 62 | 69 | 62 |
| -2 | 18 | 5 | 79 | 74 | 19 | -3 | 7 | 6 | 116 | 114 | 14 | 1 | 17 | 6 | 228 | 232 | 24 | 3 | 6 | 7 | 137 | 112 | 19 | -2 | 17 | 7 | 0 | 86 | 1 |
| -1 | 18 | 5 | 172 | 140 | 20 | -2 | 7 | 6 | 349 | 334 | 16 | 2 | 17 | 6 | 56 | 15 | 55 | 4 | 6 | 7 | 75 | 92 | 75 | -1 | 17 | 7 | 113 | 86 | 18 |
| 0 | 18 | 5 | 45 | 19 | 45 | -1 | 7 | 6 | 503 | 537 | 21 | 3 | 17 | 6 | 52 | 88 | 51 | -4 | 7 | 7 | 90 | 73 | 31 | 0 | 17 | 7 | 179 | 169 | 10 |
| 1 | 18 | 5 | 126 | 140 | 16 | 0 | 7 | 6 | 144 | 139 | 7 | -3 | 18 | 6 | 150 | 150 | 52 | -3 | 7 | 7 | 106 | 97 | 40 | 1 | 17 | 7 | 83 | 86 | 26 |
| 2 | 18 | 5 | 49 | 73 | 49 | 1 | 7 | 6 | 501 | 537 | 20 | -2 | 18 | 6 | 124 | 107 | 12 | -2 | 7 | 7 | 130 | 149 | 13 | 2 | 17 | 7 | 77 | 86 | 34 |
| 3 | 18 | 5 | 105 | 109 | 13 | 2 | 7 | 6 | 346 | 333 | 24 | -1 | 18 | 6 | 92 | 59 | 24 | -1 | 7 | 7 | 303 | 299 | 16 | 3 | 17 | 7 | 62 | 69 | 62 |
| -2 | 19 | 5 | 71 | 50 | 35 | 3 | 7 | 6 | 127 | 114 | 14 | 0 | 18 | 6 | 55 | 52 | 51 | 0 | 7 | 7 | 528 | 546 | 16 | -3 | 18 | 7 | 91 | 56 | 27 |
| -1 | 19 | 5 | 87 | 99 | 41 | 4 | 7 | 6 | 0 | 40 | 1 | 1 | 18 | 6 | 54 | 60 | 54 | 1 | 7 | 7 | 296 | 300 | 11 | -2 | 18 | 7 | 198 | 200 | 25 |
| 0 | 19 | 5 | 79 | 48 | 22 | -4 | 8 | 6 | 152 | 154 | 19 | 2 | 18 | 6 | 94 | 107 | 22 | 2 | 7 | 7 | 123 | 149 | 27 | -1 | 18 | 7 | 66 | 49 | 37 |
| 1 | 19 | 5 | 81 | 100 | 21 | -3 | 8 | 6 | 0 | 33 | 1 | 3 | 18 | 6 | 159 | 149 | 15 | 3 | 7 | 7 | 106 | 97 | 17 | 0 | 18 | 7 | 98 | 111 | 18 |
| 2 | 19 | 5 | 59 | 50 | 59 | -2 | 8 | 6 | 455 | 416 | 20 | -2 | 19 | 6 | 319 | 338 | 11 | 4 | 7 | 7 | 30 | 73 | 29 | 1 | 18 | 7 | 48 | 49 | 47 |
| 3 | 19 | 5 | 55 | 64 | 55 | -1 | 8 | 6 | 544 | 525 | 23 | -1 | 19 | 6 | 86 | 69 | 18 | -4 | 8 | 7 | 78 | 59 | 40 | 2 | 18 | 7 | 182 | 201 | 14 |
| -2 | 20 | 5 | 172 | 163 | 12 | 0 | 8 | 6 | 0 | 35 | 1 | 0 | 19 | 6 | 351 | 348 | 13 | -3 | 8 | 7 | 197 | 188 | 12 | 3 | 18 | 7 | 68 | 57 | 27 |
| -1 | 20 | 5 | 44 | 77 | 43 | 1 | 8 | 6 | 542 | 524 | 25 | 1 | 19 | 6 | 72 | 69 | 25 | -2 | 8 | 7 | 162 | 156 | 12 | -2 | 19 | 7 | 93 | 70 | 16 |
| 0 | 20 | 5 | 115 | 116 | 14 | 2 | 8 | 6 | 451 | 416 | 30 | 2 | 19 | 6 | 317 | 338 | 23 | -1 | 8 | 7 | 579 | 589 | 21 | -1 | 19 | 7 | 107 | 124 | 14 |
| 0 | 19 | 7 | 85 | 56 | 32 | -4 | 8 | 8 | 104 | 64 | 24 | 2 | 18 | 8 | 117 | 142 | 18 | 4 | 7 | 9 | 82 | 108 | 82 | 1 | 18 | 9 | 35 | 3 | 35 |
| 1 | 19 | 7 | 93 | 124 | 20 | -3 | 8 | 8 | 189 | 196 | 12 | 3 | 18 | 8 | 49 | 35 | 48 | -4 | 8 | 9 | 112 | 119 | 23 | 2 | 18 | 9 | 211 | 260 | 16 |
| 2 | 19 | 7 | 52 | 70 | 51 | -2 | 8 | 8 | 110 | 97 | 8 | -2 | 19 | 8 | 252 | 257 | 14 | -3 | 8 | 9 | 226 | 233 | 13 | 3 | 18 | 9 | 39 | 47 | 38 |
| -2 | 20 | 7 | 68 | 82 | 20 | -1 | 8 | 8 | 417 | 439 | 15 | -1 | 19 | 8 | 123 | 137 | 14 | -2 | 8 | 9 | 230 | 201 | 7 | -2 | 19 | 9 | 101 | 91 | 14 |
| -1 | 20 | 7 | 49 | 66 | 49 | 0 | 8 | 8 | 391 | 381 | 12 | 0 | 19 | 8 | 323 | 306 | 12 | -1 | 8 | 9 | 171 | 137 | 10 | -1 | 19 | 9 | 88 | 117 | 17 |
| 0 | 20 | 7 | 96 | 94 | 17 | 1 | 8 | 8 | 438 | 440 | 16 | 1 | 19 | 8 | 113 | 137 | 38 | 0 | 8 | 9 | 132 | 135 | 9 | 0 | 19 | 9 | 66 | 24 | 30 |
| 1 | 20 | 7 | 61 | 66 | 39 | 2 | 8 | 8 | 104 | 98 | 24 | 2 | 19 | 8 | 256 | 256 | 20 | 1 | 8 | 9 | 161 | 138 | 12 | 1 | 19 | 9 | 79 | 117 | 21 |
| 2 | 20 | 7 | 50 | 82 | 50 | 3 | 8 | 8 | 210 | 196 | 12 | -2 | 20 | 8 | 127 | 108 | 14 | 2 | 8 | 9 | 243 | 201 | 15 | 2 | 19 | 9 | 65 | 92 | 36 |
| -2 | 21 | 7 | 0 | 22 | 1 | 4 | 8 | 8 | 54 | 65 | 42 | -1 | 20 | 8 | 147 | 123 | 32 | 3 | 8 | 9 | 259 | 232 | 11 | -2 | 20 | 9 | 92 | 85 | 14 |
| -1 | 21 | 7 | 88 | 97 | 16 | -4 | 9 | 8 | 36 | 57 | 35 | 0 | 20 | 8 | 59 | 76 | 46 | 4 | 8 | 9 | 115 | 120 | 14 | -1 | 20 | 9 | 221 | 209 | 21 |
| 0 | 21 | 7 | 375 | 331 | 26 | -3 | 9 | 8 | 96 | 119 | 19 | 1 | 20 | 8 | 101 | 123 | 17 | -4 | 9 | 9 | 89 | 63 | 30 | 0 | 20 | 9 | 154 | 167 | 12 |
| 1 | 21 | 7 | 95 | 96 | 17 | -2 | 9 | 8 | 174 | 165 | 7 | 2 | 20 | 8 | 94 | 108 | 21 | -3 | 9 | 9 | 136 | 161 | 38 | 1 | 20 | 9 | 213 | 210 | 13 |
| 2 | 21 | 7 | 59 | 22 | 59 | -1 | 9 | 8 | 292 | 269 | 12 | -2 | 21 | 8 | 69 | 75 | 18 | -2 | 9 | 9 | 189 | 181 | 7 | 2 | 20 | 9 | 62 | 86 | 41 |
| -1 | 22 | 7 | 53 | 89 | 42 | 0 | 9 | 8 | 95 | 83 | 12 | -1 | 21 | 8 | 197 | 226 | 18 | -1 | 9 | 9 | 319 | 285 | 12 | -2 | 21 | 9 | 28 | 43 | 28 |
| 0 | 22 | 7 | 0 | 31 | 1 | 1 | 9 | 8 | 292 | 269 | 14 | 0 | 21 | 8 | 89 | 45 | 21 | 0 | 9 | 9 | 53 | 9 | 34 | -1 | 21 | 9 | 25 | 20 | 24 |
| 1 | 22 | 7 | 78 | 89 | 23 | 2 | 9 | 8 | 167 | 165 | 11 | 1 | 21 | 8 | 204 | 226 | 17 | 1 | 9 | 9 | 307 | 285 | 16 | 0 | 21 | 9 | 113 | 127 | 29 |
| -1 | 23 | 7 | 26 | 38 | 26 | 3 | 9 | 8 | 127 | 119 | 16 | 2 | 21 | 8 | 97 | 76 | 27 | 2 | 9 | 9 | 194 | 181 | 11 | 1 | 21 | 9 | 46 | 19 | 45 |
| 0 | 23 | 7 | 0 | 2 | 1 | 4 | 9 | 8 | 0 | 57 | 1 | -1 | 22 | 8 | 69 | 57 | 23 | 3 | 9 | 9 | 162 | 161 | 13 | 2 | 21 | 9 | 80 | 43 | 36 |
| 1 | 23 | 7 | 40 | 38 | 40 | -4 | 10 | 8 | 77 | 77 | 36 | 0 | 22 | 8 | 0 | 27 | 1 | 4 | 9 | 9 | 63 | 63 | 30 | -1 | 22 | 9 | 99 | 96 | 15 |
| 0 | 0 | 8 | 682 | 694 | 29 | -3 | 10 | 8 | 82 | 58 | 82 | 1 | 22 | 8 | 67 | 57 | 29 | -4 | 10 | 9 | 99 | 62 | 22 | 0 | 22 | 9 | 0 | 5 | 1 |
| 1 | 0 | 8 | 126 | 138 | 6 | -2 | 10 | 8 | 209 | 230 | 9 | 0 | 23 | 8 | 88 | 71 | 20 | -3 | 10 | 9 | 0 | 23 | 1 | 1 | 22 | 9 | 113 | 96 | 15 |
| 2 | 0 | 8 | 457 | 482 | 29 | -1 | 10 | 8 | 377 | 364 | 14 | 1 | 0 | 9 | 73 | 75 | 10 | -2 | 10 | 9 | 262 | 256 | 8 | 0 | 23 | 9 | 69 | 43 | 26 |
| 3 | 0 | 8 | 159 | 153 | 24 | 0 | 10 | 8 | 126 | 150 | 12 | 2 | 0 | 9 | 184 | 183 | 17 | -1 | 10 | 9 | 328 | 335 | 13 | 0 | 0 | 10 | 268 | 335 | 12 |
| 4 | 0 | 8 | 0 | 44 | 1 | 1 | 10 | 8 | 391 | 364 | 17 | 3 | 0 | 9 | 30 | 10 | 30 | 0 | 10 | 9 | 127 | 113 | 12 | 1 | 0 | 10 | 362 | 410 | 13 |
| -4 | 1 | 8 | 53 | 90 | 53 | 2 | 10 | 8 | 245 | 230 | 19 | 4 | 0 | 9 | 135 | 165 | 33 | 1 | 10 | 9 | 347 | 334 | 16 | 2 | 0 | 10 | 241 | 247 | 12 |
| -3 | 1 | 8 | 0 | 25 | 1 | 3 | 10 | 8 | 80 | 59 | 32 | -4 | 1 | 9 | 39 | 86 | 38 | 2 | 10 | 9 | 272 | 257 | 10 | 3 | 0 | 10 | 84 | 89 | 16 |
| -2 | 1 | 8 | 362 | 346 | 16 | 4 | 10 | 8 | 112 | 77 | 14 | -3 | 1 | 9 | 0 | 117 | 1 | 3 | 10 | 9 | 0 | 23 | 1 | 4 | 0 | 10 | 0 | 2 | 1 |
| -1 | 1 | 8 | 98 | 106 | 7 | -4 | 11 | 8 | 109 | 110 | 22 | -2 | 1 | 9 | 338 | 335 | 12 | 4 | 10 | 9 | 83 | 62 | 21 | -4 | 1 | 10 | 273 | 264 | 43 |
| 0 | 1 | 8 | 184 | 165 | 7 | -3 | 11 | 8 | 70 | 87 | 69 | -1 | 1 | 9 | 251 | 304 | 12 | -4 | 11 | 9 | 111 | 109 | 21 | -3 | 1 | 10 | 81 | 73 | 36 |
| 1 | 1 | 8 | 91 | 106 | 6 | -2 | 11 | 8 | 205 | 202 | 8 | 0 | 1 | 9 | 461 | 508 | 14 | -3 | 11 | 9 | 92 | 105 | 20 | -2 | 1 | 10 | 102 | 99 | 11 |
| 2 | 1 | 8 | 368 | 346 | 24 | -1 | 11 | 8 | 0 | 23 | 1 | 1 | 1 | 9 | 258 | 304 | 8 | -2 | 11 | 9 | 59 | 66 | 28 | -1 | 1 | 10 | 203 | 213 | 11 |
| 3 | 1 | 8 | 58 | 24 | 57 | 0 | 11 | 8 | 592 | 594 | 21 | 2 | 1 | 9 | 344 | 335 | 22 | -1 | 11 | 9 | 123 | 99 | 13 | 0 | 1 | 10 | 246 | 265 | 8 |
| 4 | 1 | 8 | 64 | 89 | 64 | 1 | 11 | 8 | 48 | 22 | 47 | 3 | 1 | 9 | 118 | 117 | 11 | 0 | 11 | 9 | 193 | 205 | 10 | 1 | 1 | 10 | 195 | 214 | 11 |
| -4 | 2 | 8 | 86 | 53 | 80 | 2 | 11 | 8 | 209 | 201 | 10 | 4 | 1 | 9 | 76 | 86 | 75 | 1 | 11 | 9 | 122 | 99 | 18 | 2 | 1 | 10 | 114 | 99 | 22 |
| -3 | 2 | 8 | 165 | 151 | 8 | 3 | 11 | 8 | 131 | 86 | 42 | -4 | 2 | 9 | 136 | 76 | 19 | 2 | 11 | 9 | 96 | 66 | 15 | 3 | 1 | 10 | 82 | 73 | 18 |
| -2 | 2 | 8 | 142 | 123 | 10 | 4 | 11 | 8 | 135 | 110 | 30 | -3 | 2 | 9 | 175 | 157 | 16 | 3 | 11 | 9 | 110 | 105 | 21 | 4 | 1 | 10 | 242 | 263 | 26 |
| -1 | 2 | 8 | 249 | 274 | 7 | -4 | 12 | 8 | 124 | 110 | 19 | -2 | 2 | 9 | 239 | 238 | 12 | 4 | 11 | 9 | 114 | 109 | 15 | -4 | 2 | 10 | 163 | 116 | 19 |
| 0 | 2 | 8 | 640 | 649 | 19 | -3 | 12 | 8 | 128 | 173 | 14 | -1 | 2 | 9 | 713 | 816 | 31 | -4 | 12 | 9 | 83 | 75 | 33 | -3 | 2 | 10 | 112 | 89 | 9 |
| 1 | 2 | 8 | 239 | 275 | 8 | -2 | 12 | 8 | 30 | 10 | 29 | 0 | 2 | 9 | 741 | 783 | 26 | -3 | 12 | 9 | 87 | 94 | 19 | -2 | 2 | 10 | 233 | 253 | 11 |
| 2 | 2 | 8 | 103 | 122 | 27 | -1 | 12 | 8 | 36 | 58 | 35 | 1 | 2 | 9 | 731 | 816 | 29 | -2 | 12 | 9 | 0 | 25 | 1 | -1 | 2 | 10 | 437 | 476 | 29 |
| 3 | 2 | 8 | 166 | 152 | 10 | 0 | 12 | 8 | 0 | 42 | 1 | 2 | 2 | 9 | 232 | 237 | 19 | -1 | 12 | 9 | 313 | 323 | 11 | 0 | 2 | 10 | 139 | 133 | 7 |
| 4 | 2 | 8 | 0 | 53 | 1 | 1 | 12 | 8 | 64 | 59 | 47 | 3 | 2 | 9 | 156 | 157 | 11 | 0 | 12 | 9 | 318 | 324 | 16 | 1 | 2 | 10 | 426 | 477 | 13 |
| -4 | 3 | 8 | 92 | 119 | 30 | 2 | 12 | 8 | 49 | 9 | 48 | 4 | 2 | 9 | 87 | 76 | 67 | 1 | 12 | 9 | 315 | 325 | 11 | 2 | 2 | 10 | 214 | 254 | 20 |
| -3 | 3 | 8 | 216 | 228 | 7 | 3 | 12 | 8 | 185 | 173 | 13 | -4 | 3 | 9 | 78 | 76 | 39 | 2 | 12 | 9 | 37 | 25 | 37 | 3 | 2 | 10 | 67 | 89 | 24 |
| -2 | 3 | 8 | 288 | 261 | 13 | 4 | 12 | 8 | 110 | 110 | 19 | -3 | 3 | 9 | 205 | 188 | 7 | 3 | 12 | 9 | 97 | 95 | 17 | 4 | 2 | 10 | 58 | 116 | 58 |
| -1 | 3 | 8 | 710 | 725 | 27 | -4 | 13 | 8 | 89 | 10 | 88 | -2 | 3 | 9 | 181 | 181 | 10 | 4 | 12 | 9 | 44 | 75 | 43 | -4 | 3 | 10 | 259 | 235 | 29 |
| 0 | 3 | 8 | 0 | 2 | 1 | -3 | 13 | 8 | 88 | 106 | 19 | -1 | 3 | 9 | 147 | 154 | 7 | -4 | 13 | 9 | 117 | 124 | 35 | -3 | 3 | 10 | 154 | 148 | 7 |
| 1 | 3 | 8 | 650 | 725 | 20 | -2 | 13 | 8 | 185 | 188 | 10 | 0 | 3 | 9 | 519 | 573 | 18 | -3 | 13 | 9 | 171 | 191 | 12 | -2 | 3 | 10 | 262 | 254 | 13 |
| 2 | 3 | 8 | 272 | 260 | 21 | -1 | 13 | 8 | 131 | 116 | 14 | 1 | 3 | 9 | 139 | 154 | 6 | -2 | 13 | 9 | 69 | 79 | 24 | -1 | 3 | 10 | 271 | 277 | 12 |
| 3 | 3 | 8 | 242 | 228 | 20 | 0 | 13 | 8 | 300 | 282 | 10 | 2 | 3 | 9 | 142 | 181 | 24 | -1 | 13 | 9 | 324 | 308 | 10 | 0 | 3 | 10 | 356 | 421 | 11 |
| 4 | 3 | 8 | 136 | 119 | 31 | 1 | 13 | 8 | 136 | 116 | 9 | 3 | 3 | 9 | 199 | 188 | 17 | 0 | 13 | 9 | 109 | 61 | 29 | 1 | 3 | 10 | 278 | 277 | 12 |
| -4 | 4 | 8 | 208 | 185 | 15 | 2 | 13 | 8 | 194 | 188 | 10 | 4 | 3 | 9 | 96 | 77 | 48 | 1 | 13 | 9 | 317 | 308 | 10 | 2 | 3 | 10 | 280 | 254 | 19 |
| -3 | 4 | 8 | 104 | 93 | 10 | 3 | 13 | 8 | 108 | 106 | 14 | -4 | 4 | 9 | 98 | 87 | 28 | 2 | 13 | 9 | 108 | 79 | 15 | 3 | 3 | 10 | 165 | 147 | 15 |
| -2 | 4 | 8 | 321 | 348 | 16 | 4 | 13 | 8 | 0 | 10 | 1 | -3 | 4 | 9 | 127 | 128 | 12 | 3 | 13 | 9 | 152 | 191 | 11 | 4 | 3 | 10 | 156 | 234 | 33 |
| -1 | 4 | 8 | 117 | 132 | 7 | -3 | 14 | 8 | 51 | 64 | 51 | -2 | 4 | 9 | 243 | 254 | 12 | 4 | 13 | 9 | 135 | 123 | 15 | -4 | 4 | 10 | 259 | 233 | 17 |
| 0 | 4 | 8 | 536 | 592 | 16 | -2 | 14 | 8 | 185 | 178 | 11 | -1 | 4 | 9 | 626 | 693 | 46 | -3 | 14 | 9 | 133 | 128 | 27 | -3 | 4 | 10 | 41 | 37 | 41 |
| 1 | 4 | 8 | 131 | 131 | 5 | -1 | 14 | 8 | 148 | 136 | 15 | 0 | 4 | 9 | 822 | 828 | 29 | -2 | 14 | 9 | 142 | 120 | 12 | -2 | 4 | 10 | 221 | 211 | 16 |
| 2 | 4 | 8 | 364 | 347 | 21 | 0 | 14 | 8 | 259 | 236 | 8 | 1 | 4 | 9 | 626 | 694 | 19 | -1 | 14 | 9 | 206 | 187 | 9 | -1 | 4 | 10 | 586 | 665 | 32 |
| 3 | 4 | 8 | 82 | 93 | 20 | 1 | 14 | 8 | 159 | 137 | 10 | 2 | 4 | 9 | 253 | 254 | 19 | 0 | 14 | 9 | 95 | 103 | 12 | 0 | 4 | 10 | 144 | 158 | 7 |
| 4 | 4 | 8 | 122 | 185 | 42 | 2 | 14 | 8 | 204 | 178 | 11 | 3 | 4 | 9 | 138 | 128 | 12 | 1 | 14 | 9 | 199 | 187 | 9 | 1 | 4 | 10 | 616 | 663 | 20 |
| -4 | 5 | 8 | 207 | 215 | 15 | 3 | 14 | 8 | 61 | 64 | 37 | 4 | 4 | 9 | 54 | 87 | 53 | 2 | 14 | 9 | 120 | 121 | 16 | 2 | 4 | 10 | 201 | 212 | 21 |
| -3 | 5 | 8 | 138 | 139 | 13 | -3 | 15 | 8 | 67 | 64 | 30 | -4 | 5 | 9 | 203 | 177 | 29 | 3 | 14 | 9 | 117 | 128 | 20 | 3 | 4 | 10 | 9 | 37 | 8 |
| -2 | 5 | 8 | 261 | 278 | 13 | -2 | 15 | 8 | 75 | 69 | 18 | -3 | 5 | 9 | 211 | 223 | 9 | -3 | 15 | 9 | 45 | 47 | 45 | -4 | 5 | 10 | 95 | 80 | 30 |
| -1 | 5 | 8 | 220 | 239 | 13 | -1 | 15 | 8 | 118 | 118 | 14 | -2 | 5 | 9 | 288 | 283 | 14 | -2 | 15 | 9 | 65 | 72 | 24 | -3 | 5 | 10 | 246 | 254 | 19 |
| 0 | 5 | 8 | 773 | 792 | 27 | 0 | 15 | 8 | 464 | 472 | 12 | -1 | 5 | 9 | 89 | 100 | 10 | -1 | 15 | 9 | 187 | 178 | 10 | -2 | 5 | 10 | 39 | 23 | 38 |
| 1 | 5 | 8 | 225 | 240 | 7 | 1 | 15 | 8 | 133 | 117 | 11 | 0 | 5 | 9 | 273 | 345 | 9 | 0 | 15 | 9 | 84 | 65 | 15 | -1 | 5 | 10 | 171 | 162 | 17 |
| 2 | 5 | 8 | 291 | 277 | 19 | 2 | 15 | 8 | 44 | 70 | 43 | 1 | 5 | 9 | 102 | 99 | 9 | 1 | 15 | 9 | 190 | 178 | 11 | 0 | 5 | 10 | 846 | 854 | 25 |
| 3 | 5 | 8 | 150 | 139 | 15 | 3 | 15 | 8 | 69 | 64 | 25 | 2 | 5 | 9 | 301 | 284 | 21 | 2 | 15 | 9 | 27 | 72 | 26 | 1 | 5 | 10 | 164 | 162 | 7 |
| 4 | 5 | 8 | 144 | 215 | 31 | -3 | 16 | 8 | 76 | 52 | 49 | 3 | 5 | 9 | 224 | 223 | 11 | 3 | 15 | 9 | 46 | 46 | 45 | 2 | 5 | 10 | 0 | 24 | 1 |
| -4 | 6 | 8 | 199 | 158 | 23 | -2 | 16 | 8 | 81 | 87 | 17 | 4 | 5 | 9 | 138 | 176 | 30 | -3 | 16 | 9 | 56 | 69 | 56 | 3 | 5 | 10 | 251 | 254 | 17 |
| -3 | 6 | 8 | 261 | 246 | 10 | -1 | 16 | 8 | 230 | 204 | 12 | -4 | 6 | 9 | 91 | 37 | 31 | -2 | 16 | 9 | 219 | 235 | 10 | 4 | 5 | 10 | 91 | 80 | 43 |
| -2 | 6 | 8 | 301 | 303 | 14 | 0 | 16 | 8 | 207 | 204 | 13 | -3 | 6 | 9 | 219 | 218 | 9 | -1 | 16 | 9 | 223 | 218 | 12 | -4 | 6 | 10 | 79 | 75 | 41 |
| -1 | 6 | 8 | 184 | 217 | 9 | 1 | 16 | 8 | 222 | 204 | 21 | -2 | 6 | 9 | 78 | 59 | 20 | 0 | 16 | 9 | 126 | 113 | 11 | -3 | 6 | 10 | 294 | 283 | 8 |
| 0 | 6 | 8 | 516 | 526 | 15 | 2 | 16 | 8 | 107 | 87 | 19 | -1 | 6 | 9 | 42 | 45 | 41 | 1 | 16 | 9 | 216 | 218 | 13 | -2 | 6 | 10 | 269 | 256 | 13 |
| 1 | 6 | 8 | 200 | 215 | 6 | 3 | 16 | 8 | 24 | 51 | 23 | 0 | 6 | 9 | 413 | 441 | 12 | 2 | 16 | 9 | 248 | 235 | 18 | -1 | 6 | 10 | 433 | 491 | 26 |
| 2 | 6 | 8 | 308 | 304 | 22 | -3 | 17 | 8 | 146 | 137 | 23 | 1 | 6 | 9 | 66 | 47 | 12 | 3 | 16 | 9 | 66 | 68 | 29 | 0 | 6 | 10 | 951 | 909 | 29 |
| 3 | 6 | 8 | 252 | 246 | 12 | -2 | 17 | 8 | 161 | 162 | 11 | 2 | 6 | 9 | 57 | 59 | 56 | -3 | 17 | 9 | 0 | 63 | 1 | 1 | 6 | 10 | 445 | 491 | 26 |
| 4 | 6 | 8 | 99 | 158 | 45 | -1 | 17 | 8 | 260 | 251 | 14 | 3 | 6 | 9 | 228 | 219 | 12 | -2 | 17 | 9 | 114 | 123 | 17 | 2 | 6 | 10 | 255 | 257 | 22 |
| -4 | 7 | 8 | 76 | 65 | 44 | 0 | 17 | 8 | 0 | 35 | 1 | 4 | 6 | 9 | 0 | 37 | 1 | -1 | 17 | 9 | 311 | 266 | 14 | 3 | 6 | 10 | 293 | 282 | 22 |
| -3 | 7 | 8 | 213 | 181 | 9 | 1 | 17 | 8 | 248 | 251 | 14 | -4 | 7 | 9 | 102 | 108 | 27 | 0 | 17 | 9 | 32 | 30 | 32 | 4 | 6 | 10 | 83 | 75 | 54 |
| -2 | 7 | 8 | 329 | 326 | 15 | 2 | 17 | 8 | 166 | 161 | 13 | -3 | 7 | 9 | 248 | 252 | 12 | 1 | 17 | 9 | 319 | 266 | 20 | -4 | 7 | 10 | 113 | 128 | 42 |
| -1 | 7 | 8 | 70 | 51 | 23 | 3 | 17 | 8 | 145 | 138 | 14 | -2 | 7 | 9 | 305 | 333 | 16 | 2 | 17 | 9 | 121 | 123 | 18 | -3 | 7 | 10 | 23 | 49 | 23 |
| 0 | 7 | 8 | 117 | 117 | 12 | -3 | 18 | 8 | 68 | 34 | 48 | -1 | 7 | 9 | 216 | 230 | 9 | 3 | 17 | 9 | 35 | 63 | 35 | -2 | 7 | 10 | 455 | 473 | 12 |
| 1 | 7 | 8 | 42 | 51 | 42 | -2 | 18 | 8 | 156 | 142 | 10 | 0 | 7 | 9 | 468 | 496 | 14 | -3 | 18 | 9 | 0 | 47 | 1 | -1 | 7 | 10 | 73 | 97 | 26 |
| 2 | 7 | 8 | 335 | 327 | 23 | -1 | 18 | 8 | 237 | 251 | 10 | 1 | 7 | 9 | 195 | 230 | 9 | -2 | 18 | 9 | 262 | 260 | 10 | 0 | 7 | 10 | 827 | 837 | 29 |
| 3 | 7 | 8 | 193 | 182 | 15 | 0 | 18 | 8 | 218 | 187 | 10 | 2 | 7 | 9 | 359 | 333 | 21 | -1 | 18 | 9 | 56 | 3 | 43 | 1 | 7 | 10 | 72 | 96 | 21 |
| 4 | 7 | 8 | 54 | 65 | 54 | 1 | 18 | 8 | 205 | 251 | 14 | 3 | 7 | 9 | 262 | 252 | 16 | 0 | 18 | 9 | 415 | 419 | 14 | 2 | 7 | 10 | 479 | 473 | 31 |
| 3 | 7 | 10 | 81 | 51 | 24 | 1 | 18 | 10 | 159 | 189 | 20 | 3 | 7 | 11 | 136 | 136 | 14 | -2 | 19 | 11 | 76 | 67 | 20 | -3 | 8 | 12 | 162 | 171 | 11 |
| 4 | 7 | 10 | 103 | 127 | 17 | 2 | 18 | 10 | 57 | 70 | 56 | 4 | 7 | 11 | 67 | 67 | 28 | -1 | 19 | 11 | 156 | 153 | 19 | -2 | 8 | 12 | 339 | 356 | 9 |
| -4 | 8 | 10 | 0 | 30 | 1 | 3 | 18 | 10 | 88 | 96 | 21 | -4 | 8 | 11 | 109 | 70 | 24 | 0 | 19 | 11 | 158 | 117 | 13 | -1 | 8 | 12 | 457 | 456 | 16 |
| -3 | 8 | 10 | 124 | 100 | 19 | -2 | 19 | 10 | 168 | 164 | 10 | -3 | 8 | 11 | 133 | 122 | 21 | 1 | 19 | 11 | 146 | 153 | 39 | 0 | 8 | 12 | 456 | 465 | 16 |
| -2 | 8 | 10 | 300 | 301 | 9 | -1 | 19 | 10 | 115 | 141 | 34 | -2 | 8 | 11 | 216 | 171 | 33 | 2 | 19 | 11 | 76 | 67 | 76 | 1 | 8 | 12 | 453 | 455 | 20 |
| -1 | 8 | 10 | 196 | 192 | 10 | 0 | 19 | 10 | 120 | 114 | 16 | -1 | 8 | 11 | 141 | 125 | 11 | -2 | 20 | 11 | 186 | 173 | 10 | 2 | 8 | 12 | 352 | 356 | 11 |
| 0 | 8 | 10 | 170 | 198 | 8 | 1 | 19 | 10 | 107 | 141 | 23 | 0 | 8 | 11 | 102 | 84 | 13 | -1 | 20 | 11 | 127 | 145 | 15 | 3 | 8 | 12 | 182 | 171 | 10 |
| 1 | 8 | 10 | 176 | 195 | 13 | 2 | 19 | 10 | 139 | 164 | 16 | 1 | 8 | 11 | 127 | 126 | 14 | 0 | 20 | 11 | 179 | 199 | 21 | 4 | 8 | 12 | 96 | 122 | 23 |
| 2 | 8 | 10 | 329 | 301 | 12 | -2 | 20 | 10 | 81 | 51 | 16 | 2 | 8 | 11 | 184 | 171 | 11 | 1 | 20 | 11 | 141 | 145 | 22 | -4 | 9 | 12 | 120 | 123 | 19 |
| 3 | 8 | 10 | 94 | 100 | 21 | -1 | 20 | 10 | 23 | 30 | 22 | 3 | 8 | 11 | 128 | 122 | 16 | 2 | 20 | 11 | 172 | 172 | 20 | -3 | 9 | 12 | 0 | 16 | 1 |
| 4 | 8 | 10 | 33 | 30 | 32 | 0 | 20 | 10 | 41 | 26 | 41 | 4 | 8 | 11 | 87 | 70 | 20 | -2 | 21 | 11 | 0 | 37 | 1 | -2 | 9 | 12 | 84 | 65 | 13 |
| -4 | 9 | 10 | 70 | 36 | 48 | 1 | 20 | 10 | 0 | 30 | 1 | -4 | 9 | 11 | 96 | 58 | 23 | -1 | 21 | 11 | 71 | 64 | 22 | -1 | 9 | 12 | 472 | 445 | 17 |
| -3 | 9 | 10 | 103 | 76 | 22 | 2 | 20 | 10 | 84 | 51 | 31 | -3 | 9 | 11 | 219 | 212 | 27 | 0 | 21 | 11 | 0 | 82 | 1 | 0 | 9 | 12 | 146 | 143 | 11 |
| -2 | 9 | 10 | 214 | 217 | 14 | -2 | 21 | 10 | 0 | 19 | 1 | -2 | 9 | 11 | 43 | 36 | 43 | 1 | 21 | 11 | 86 | 63 | 20 | 1 | 9 | 12 | 472 | 444 | 21 |
| -1 | 9 | 10 | 203 | 194 | 10 | -1 | 21 | 10 | 82 | 110 | 17 | -1 | 9 | 11 | 123 | 124 | 13 | -1 | 22 | 11 | 77 | 83 | 21 | 2 | 9 | 12 | 73 | 64 | 20 |
| 0 | 9 | 10 | 193 | 190 | 10 | 0 | 21 | 10 | 70 | 60 | 40 | 0 | 9 | 11 | 403 | 400 | 15 | 0 | 22 | 11 | 0 | 50 | 1 | 3 | 9 | 12 | 0 | 17 | 1 |
| 1 | 9 | 10 | 171 | 193 | 14 | 1 | 21 | 10 | 98 | 110 | 18 | 1 | 9 | 11 | 118 | 123 | 21 | 1 | 22 | 11 | 61 | 84 | 30 | 4 | 9 | 12 | 112 | 122 | 12 |
| 2 | 9 | 10 | 212 | 217 | 9 | 2 | 21 | 10 | 55 | 19 | 55 | 2 | 9 | 11 | 89 | 36 | 15 | 0 | 23 | 11 | 29 | 56 | 29 | -4 | 10 | 12 | 87 | 92 | 29 |
| 3 | 9 | 10 | 96 | 77 | 21 | -1 | 22 | 10 | 0 | 60 | 1 | 3 | 9 | 11 | 231 | 212 | 10 | 0 | 0 | 12 | 153 | 174 | 16 | -3 | 10 | 12 | 43 | 14 | 42 |
| 4 | 9 | 10 | 65 | 36 | 36 | 0 | 22 | 10 | 27 | 4 | 27 | 4 | 9 | 11 | 82 | 59 | 21 | 1 | 0 | 12 | 968 | 1014 | 60 | -2 | 10 | 12 | 96 | 110 | 12 |
| -4 | 10 | 10 | 113 | 104 | 21 | 1 | 22 | 10 | 20 | 60 | 19 | -4 | 10 | 11 | 143 | 113 | 25 | 2 | 0 | 12 | 112 | 91 | 11 | -1 | 10 | 12 | 127 | 122 | 22 |
| -3 | 10 | 10 | 153 | 167 | 31 | 0 | 23 | 10 | 77 | 52 | 26 | -3 | 10 | 11 | 159 | 155 | 27 | 3 | 0 | 12 | 106 | 96 | 14 | 0 | 10 | 12 | 290 | 303 | 12 |
| -2 | 10 | 10 | 92 | 78 | 17 | 1 | 0 | 11 | 445 | 488 | 22 | -2 | 10 | 11 | 107 | 91 | 10 | 4 | 0 | 12 | 189 | 179 | 28 | 1 | 10 | 12 | 104 | 121 | 13 |
| -1 | 20 | 10 | 265 | 269 | 12 | 2 | 0 | 11 | 27 | 40 | 26 | -1 | 10 | 11 | 123 | 94 | 14 | -4 | 1 | 12 | 142 | 125 | 38 | 2 | 10 | 12 | 121 | 110 | 12 |
| 0 | 10 | 10 | 575 | 595 | 20 | 3 | 0 | 11 | 142 | 133 | 23 | 0 | 10 | 11 | 625 | 576 | 22 | -3 | 1 | 12 | 179 | 180 | 9 | 3 | 10 | 12 | 0 | 13 | 1 |
| 1 | 10 | 10 | 246 | 269 | 15 | 4 | 0 | 11 | 71 | 12 | 71 | 1 | 10 | 11 | 61 | 93 | 44 | -2 | 1 | 12 | 159 | 145 | 11 | 4 | 10 | 12 | 102 | 91 | 12 |
| 2 | 10 | 10 | 84 | 78 | 18 | -4 | 1 | 11 | 101 | 77 | 24 | 2 | 10 | 11 | 91 | 91 | 25 | -1 | 1 | 12 | 353 | 403 | 12 | -4 | 11 | 12 | 42 | 75 | 41 |
| 3 | 10 | 10 | 186 | 167 | 17 | -3 | 1 | 11 | 0 | 22 | 1 | 3 | 10 | 11 | 173 | 154 | 15 | 0 | 1 | 12 | 265 | 308 | 9 | -3 | 11 | 12 | 221 | 231 | 12 |
| 4 | 10 | 10 | 122 | 104 | 14 | -2 | 1 | 11 | 280 | 275 | 13 | 4 | 10 | 11 | 108 | 113 | 16 | 1 | 1 | 12 | 339 | 404 | 14 | -2 | 11 | 12 | 142 | 139 | 9 |
| -4 | 11 | 10 | 119 | 100 | 20 | -1 | 1 | 11 | 401 | 431 | 10 | -4 | 11 | 11 | 84 | 78 | 31 | 2 | 1 | 12 | 157 | 145 | 21 | -1 | 11 | 12 | 166 | 164 | 10 |
| -3 | 11 | 10 | 106 | 113 | 16 | 0 | 1 | 11 | 352 | 391 | 11 | -3 | 11 | 11 | 263 | 280 | 13 | 3 | 1 | 12 | 168 | 180 | 10 | 0 | 11 | 12 | 470 | 456 | 30 |
| -2 | 11 | 10 | 128 | 120 | 9 | 1 | 1 | 11 | 387 | 431 | 12 | -2 | 11 | 11 | 80 | 80 | 68 | 4 | 1 | 12 | 177 | 124 | 49 | 1 | 11 | 12 | 194 | 164 | 23 |
| -1 | 11 | 10 | 141 | 136 | 15 | 2 | 1 | 11 | 285 | 274 | 20 | -1 | 11 | 11 | 92 | 88 | 15 | -4 | 2 | 12 | 153 | 147 | 18 | 2 | 11 | 12 | 147 | 138 | 11 |
| 0 | 11 | 10 | 316 | 295 | 13 | 3 | 1 | 11 | 66 | 22 | 44 | 0 | 11 | 11 | 478 | 494 | 21 | -3 | 2 | 12 | 171 | 172 | 11 | 3 | 11 | 12 | 228 | 231 | 11 |
| 1 | 11 | 10 | 142 | 136 | 8 | 4 | 1 | 11 | 97 | 77 | 44 | 1 | 11 | 11 | 90 | 88 | 14 | -2 | 2 | 12 | 267 | 279 | 13 | 4 | 11 | 12 | 78 | 75 | 20 |
| 2 | 11 | 10 | 121 | 120 | 13 | -4 | 2 | 11 | 137 | 92 | 17 | 2 | 11 | 11 | 80 | 81 | 19 | -1 | 2 | 12 | 483 | 535 | 24 | -4 | 12 | 12 | 61 | 71 | 61 |
| 3 | 11 | 10 | 120 | 113 | 13 | -3 | 2 | 11 | 203 | 199 | 16 | 3 | 11 | 11 | 277 | 281 | 12 | 0 | 2 | 12 | 103 | 143 | 8 | -3 | 12 | 12 | 210 | 224 | 12 |
| 4 | 11 | 10 | 101 | 99 | 26 | -2 | 2 | 11 | 235 | 233 | 12 | 4 | 11 | 11 | 46 | 78 | 46 | 1 | 2 | 12 | 458 | 535 | 17 | -2 | 12 | 12 | 149 | 148 | 27 |
| -4 | 12 | 10 | 51 | 98 | 51 | -1 | 2 | 11 | 466 | 493 | 18 | -3 | 12 | 11 | 79 | 68 | 23 | 2 | 2 | 12 | 277 | 279 | 20 | -1 | 12 | 12 | 98 | 79 | 13 |
| -3 | 12 | 10 | 101 | 121 | 17 | 0 | 2 | 11 | 28 | 42 | 28 | -2 | 12 | 11 | 86 | 78 | 34 | 3 | 2 | 12 | 169 | 172 | 10 | 0 | 12 | 12 | 200 | 169 | 8 |
| -2 | 12 | 10 | 89 | 84 | 15 | 1 | 2 | 11 | 435 | 492 | 14 | -1 | 12 | 11 | 159 | 140 | 11 | 4 | 2 | 12 | 148 | 147 | 64 | 1 | 12 | 12 | 91 | 79 | 14 |
| -1 | 12 | 10 | 214 | 221 | 9 | 2 | 2 | 11 | 245 | 232 | 19 | 0 | 12 | 11 | 0 | 15 | 1 | -4 | 3 | 12 | 173 | 189 | 30 | 2 | 12 | 12 | 153 | 148 | 11 |
| 0 | 12 | 10 | 286 | 256 | 12 | 3 | 2 | 11 | 202 | 199 | 11 | 1 | 22 | 11 | 162 | 140 | 8 | -3 | 3 | 12 | 136 | 132 | 16 | 3 | 12 | 12 | 223 | 224 | 11 |
| 1 | 12 | 10 | 216 | 221 | 8 | 4 | 2 | 11 | 0 | 92 | 1 | 2 | 12 | 11 | 94 | 78 | 17 | -2 | 3 | 12 | 297 | 305 | 14 | 4 | 12 | 12 | 47 | 72 | 47 |
| 2 | 12 | 10 | 61 | 84 | 39 | -4 | 3 | 11 | 199 | 181 | 31 | 3 | 12 | 11 | 99 | 69 | 16 | -1 | 3 | 12 | 180 | 205 | 9 | -3 | 13 | 12 | 0 | 56 | 1 |
| 3 | 12 | 10 | 122 | 121 | 13 | -3 | 3 | 11 | 110 | 114 | 10 | 4 | 12 | 11 | 101 | 96 | 32 | 0 | 3 | 12 | 538 | 571 | 16 | -2 | 13 | 12 | 97 | 117 | 17 |
| 4 | 12 | 10 | 103 | 98 | 16 | -2 | 3 | 11 | 61 | 94 | 36 | -3 | 13 | 11 | 214 | 231 | 27 | 1 | 3 | 12 | 166 | 205 | 9 | -1 | 13 | 12 | 180 | 175 | 9 |
| -4 | 13 | 10 | 80 | 110 | 35 | -1 | 3 | 11 | 497 | 546 | 17 | -2 | 13 | 11 | 59 | 67 | 30 | 2 | 3 | 12 | 298 | 305 | 22 | 0 | 13 | 12 | 160 | 147 | 8 |
| -3 | 13 | 10 | 129 | 147 | 13 | 0 | 3 | 11 | 517 | 560 | 18 | -1 | 13 | 11 | 12 | 43 | 11 | 3 | 3 | 12 | 138 | 132 | 10 | 1 | 13 | 12 | 180 | 175 | 8 |
| -2 | 13 | 10 | 149 | 151 | 11 | 1 | 3 | 11 | 497 | 547 | 17 | 0 | 13 | 11 | 349 | 322 | 11 | 4 | 3 | 12 | 195 | 189 | 49 | 2 | 13 | 12 | 138 | 116 | 13 |
| -1 | 13 | 10 | 167 | 141 | 9 | 2 | 3 | 11 | 109 | 94 | 26 | 1 | 13 | 11 | 64 | 43 | 21 | -4 | 4 | 12 | 157 | 174 | 40 | 3 | 13 | 12 | 61 | 57 | 29 |
| 0 | 13 | 10 | 233 | 218 | 8 | 3 | 3 | 11 | 117 | 114 | 15 | 2 | 13 | 11 | 53 | 68 | 52 | -3 | 4 | 12 | 54 | 35 | 27 | -3 | 14 | 12 | 131 | 125 | 13 |
| 1 | 13 | 10 | 153 | 141 | 9 | 4 | 3 | 11 | 129 | 180 | 31 | 3 | 13 | 11 | 178 | 231 | 15 | -2 | 4 | 12 | 193 | 175 | 16 | -2 | 14 | 12 | 88 | 89 | 51 |
| 2 | 13 | 10 | 161 | 152 | 12 | -4 | 4 | 11 | 189 | 185 | 20 | -3 | 14 | 11 | 118 | 120 | 15 | -1 | 4 | 12 | 297 | 318 | 19 | -1 | 14 | 12 | 253 | 225 | 9 |
| 3 | 13 | 10 | 133 | 147 | 12 | -3 | 4 | 11 | 134 | 128 | 9 | -2 | 14 | 11 | 161 | 92 | 38 | 0 | 4 | 12 | 391 | 368 | 12 | 0 | 14 | 12 | 168 | 158 | 9 |
| -3 | 14 | 10 | 68 | 84 | 30 | -2 | 4 | 11 | 301 | 313 | 21 | -1 | 14 | 11 | 144 | 126 | 10 | 1 | 4 | 12 | 295 | 318 | 13 | 1 | 14 | 12 | 248 | 224 | 10 |
| -2 | 14 | 10 | 243 | 228 | 10 | -1 | 4 | 11 | 511 | 542 | 21 | 0 | 14 | 11 | 0 | 15 | 1 | 2 | 4 | 12 | 166 | 174 | 23 | 2 | 14 | 12 | 84 | 88 | 23 |
| -1 | 14 | 10 | 448 | 422 | 12 | 0 | 4 | 11 | 333 | 399 | 12 | 1 | 14 | 11 | 122 | 125 | 11 | 3 | 4 | 12 | 82 | 35 | 20 | 3 | 14 | 12 | 125 | 125 | 17 |
| 0 | 14 | 10 | 66 | 18 | 20 | 1 | 4 | 11 | 495 | 543 | 18 | 2 | 14 | 11 | 118 | 93 | 27 | 4 | 4 | 12 | 136 | 174 | 26 | -3 | 15 | 12 | 62 | 29 | 34 |
| 1 | 14 | 10 | 442 | 423 | 13 | 2 | 4 | 11 | 323 | 314 | 21 | 3 | 14 | 11 | 113 | 119 | 13 | -4 | 5 | 12 | 65 | 70 | 64 | -2 | 15 | 12 | 106 | 109 | 20 |
| 2 | 14 | 10 | 251 | 227 | 12 | 3 | 4 | 11 | 119 | 128 | 14 | -3 | 15 | 11 | 102 | 103 | 17 | -3 | 5 | 12 | 154 | 143 | 11 | -1 | 15 | 12 | 238 | 201 | 10 |
| 3 | 14 | 10 | 105 | 84 | 14 | 4 | 4 | 11 | 115 | 186 | 36 | -2 | 15 | 11 | 267 | 275 | 10 | -2 | 5 | 12 | 253 | 227 | 12 | 0 | 15 | 12 | 437 | 446 | 15 |
| -3 | 15 | 10 | 0 | 23 | 1 | -4 | 5 | 11 | 103 | 98 | 48 | -1 | 15 | 11 | 188 | 179 | 10 | -1 | 5 | 12 | 526 | 542 | 23 | 1 | 15 | 12 | 224 | 201 | 12 |
| -2 | 15 | 10 | 31 | 9 | 30 | -3 | 5 | 11 | 113 | 106 | 17 | 0 | 15 | 11 | 135 | 115 | 10 | 0 | 5 | 12 | 69 | 76 | 13 | 2 | 15 | 12 | 112 | 109 | 17 |
| -1 | 15 | 10 | 230 | 216 | 10 | -2 | 5 | 11 | 113 | 130 | 14 | 1 | 15 | 11 | 178 | 178 | 14 | 1 | 5 | 12 | 517 | 543 | 18 | 3 | 15 | 12 | 24 | 29 | 24 |
| 0 | 15 | 10 | 315 | 285 | 9 | -1 | 5 | 11 | 776 | 806 | 29 | 2 | 15 | 11 | 276 | 275 | 12 | 2 | 5 | 12 | 222 | 226 | 23 | -3 | 16 | 12 | 87 | 79 | 29 |
| 1 | 15 | 10 | 235 | 217 | 28 | 0 | 5 | 11 | 147 | 136 | 7 | 3 | 15 | 11 | 82 | 102 | 20 | 3 | 5 | 12 | 159 | 143 | 12 | -2 | 16 | 12 | 150 | 114 | 21 |
| 2 | 15 | 10 | 21 | 9 | 21 | 1 | 5 | 11 | 755 | 807 | 33 | -3 | 16 | 11 | 97 | 108 | 35 | 4 | 5 | 12 | 23 | 70 | 22 | -1 | 16 | 12 | 89 | 117 | 23 |
| 3 | 15 | 10 | 51 | 23 | 51 | 2 | 5 | 11 | 61 | 130 | 61 | -2 | 16 | 11 | 72 | 65 | 18 | -4 | 6 | 12 | 145 | 157 | 22 | 0 | 16 | 12 | 69 | 52 | 25 |
| -3 | 16 | 10 | 140 | 154 | 37 | 3 | 5 | 11 | 110 | 105 | 24 | -1 | 16 | 11 | 211 | 195 | 9 | -3 | 6 | 12 | 45 | 28 | 33 | 1 | 16 | 12 | 100 | 116 | 26 |
| -2 | 16 | 10 | 76 | 95 | 18 | 4 | 5 | 11 | 85 | 98 | 49 | 0 | 16 | 11 | 305 | 264 | 11 | -2 | 6 | 12 | 290 | 231 | 13 | 2 | 16 | 12 | 95 | 114 | 27 |
| -1 | 16 | 10 | 239 | 210 | 12 | -4 | 6 | 11 | 106 | 75 | 24 | 1 | 16 | 11 | 217 | 194 | 22 | -1 | 6 | 12 | 433 | 417 | 15 | 3 | 16 | 12 | 49 | 79 | 48 |
| 0 | 16 | 10 | 159 | 175 | 13 | -3 | 6 | 11 | 157 | 149 | 21 | 2 | 16 | 11 | 49 | 66 | 49 | 0 | 6 | 12 | 67 | 83 | 15 | -3 | 17 | 12 | 71 | 26 | 41 |
| 1 | 16 | 10 | 216 | 210 | 26 | -2 | 6 | 11 | 126 | 120 | 12 | 3 | 16 | 11 | 117 | 109 | 14 | 1 | 6 | 12 | 424 | 416 | 19 | -2 | 17 | 12 | 154 | 175 | 12 |
| 2 | 16 | 10 | 93 | 94 | 21 | -1 | 6 | 11 | 745 | 725 | 25 | -3 | 17 | 11 | 80 | 52 | 33 | 2 | 6 | 12 | 225 | 230 | 19 | -1 | 17 | 12 | 134 | 131 | 12 |
| 3 | 16 | 10 | 159 | 155 | 11 | 0 | 6 | 11 | 250 | 283 | 9 | -2 | 17 | 11 | 52 | 60 | 31 | 3 | 6 | 12 | 33 | 27 | 32 | 0 | 17 | 12 | 139 | 134 | 12 |
| -3 | 17 | 10 | 76 | 53 | 75 | 1 | 6 | 11 | 722 | 724 | 32 | -1 | 17 | 11 | 314 | 290 | 12 | 4 | 6 | 12 | 164 | 156 | 25 | 1 | 17 | 12 | 131 | 130 | 31 |
| -2 | 17 | 10 | 217 | 216 | 17 | 2 | 6 | 11 | 125 | 121 | 26 | -3 | 17 | 11 | 170 | 94 | 26 | -4 | 7 | 12 | 82 | 91 | 35 | 2 | 17 | 12 | 178 | 177 | 40 |
| -1 | 17 | 10 | 339 | 333 | 12 | 3 | 6 | 11 | 150 | 148 | 29 | 1 | 17 | 11 | 307 | 290 | 16 | -3 | 7 | 12 | 81 | 88 | 21 | 3 | 17 | 12 | 34 | 26 | 34 |
| 0 | 17 | 10 | 133 | 100 | 25 | 4 | 6 | 11 | 69 | 75 | 25 | 2 | 17 | 11 | 60 | 60 | 60 | -2 | 7 | 12 | 246 | 223 | 7 | -2 | 18 | 12 | 136 | 156 | 18 |
| 1 | 17 | 10 | 304 | 333 | 18 | -4 | 7 | 11 | 66 | 67 | 65 | 3 | 17 | 11 | 49 | 53 | 49 | -1 | 7 | 12 | 319 | 285 | 13 | -1 | 18 | 12 | 118 | 107 | 13 |
| 2 | 17 | 10 | 187 | 215 | 20 | -3 | 7 | 11 | 122 | 136 | 11 | -3 | 18 | 11 | 0 | 23 | 1 | 0 | 7 | 12 | 453 | 456 | 14 | 0 | 18 | 12 | 72 | 54 | 30 |
| 3 | 17 | 10 | 57 | 53 | 56 | -2 | 7 | 11 | 92 | 65 | 10 | -2 | 18 | 11 | 62 | 62 | 23 | 1 | 7 | 12 | 331 | 286 | 15 | 1 | 18 | 12 | 88 | 108 | 24 |
| -3 | 18 | 10 | 83 | 96 | 32 | -1 | 7 | 11 | 317 | 313 | 12 | -1 | 18 | 11 | 118 | 124 | 13 | 2 | 7 | 12 | 222 | 223 | 13 | 2 | 18 | 12 | 144 | 156 | 48 |
| -2 | 18 | 10 | 82 | 71 | 19 | 0 | 7 | 11 | 138 | 140 | 8 | 0 | 18 | 11 | 144 | 135 | 31 | 3 | 7 | 12 | 77 | 89 | 20 | -2 | 19 | 12 | 0 | 36 | 1 |
| -1 | 18 | 10 | 205 | 191 | 10 | 1 | 7 | 11 | 320 | 314 | 15 | 1 | 18 | 11 | 104 | 124 | 17 | 4 | 7 | 12 | 85 | 91 | 25 | -1 | 19 | 12 | 216 | 216 | 17 |
| 0 | 18 | 10 | 234 | 232 | 10 | 2 | 7 | 11 | 39 | 65 | 39 | 2 | 18 | 11 | 64 | 62 | 63 | -4 | 8 | 12 | 117 | 123 | 21 | 0 | 19 | 12 | 39 | 5 | 38 |
| 1 | 19 | 12 | 176 | 215 | 29 | 3 | 8 | 13 | 116 | 114 | 19 | -1 | 21 | 13 | 59 | 38 | 33 | 1 | 9 | 14 | 227 | 224 | 8 | -4 | 1 | 15 | 109 | 107 | 20 |
| 2 | 19 | 12 | 31 | 36 | 31 | 4 | 8 | 13 | 80 | 68 | 15 | 0 | 21 | 13 | 70 | 90 | 27 | 2 | 9 | 14 | 158 | 149 | 12 | -3 | 1 | 15 | 244 | 226 | 10 |
| -2 | 20 | 12 | 158 | 148 | 10 | -4 | 9 | 13 | 72 | 46 | 41 | 1 | 21 | 13 | 42 | 39 | 41 | 3 | 9 | 14 | 71 | 35 | 24 | -2 | 1 | 15 | 449 | 437 | 13 |
| -1 | 20 | 12 | 166 | 209 | 13 | -3 | 9 | 13 | 115 | 115 | 15 | -1 | 22 | 13 | 94 | 91 | 24 | 4 | 9 | 14 | 152 | 135 | 16 | -1 | 1 | 15 | 342 | 358 | 13 |
| 0 | 20 | 12 | 66 | 10 | 32 | -2 | 9 | 13 | 231 | 238 | 8 | 0 | 22 | 13 | 80 | 16 | 21 | -4 | 10 | 14 | 127 | 126 | 20 | 0 | 1 | 15 | 167 | 179 | 7 |
| 1 | 20 | 12 | 209 | 209 | 11 | -1 | 9 | 13 | 459 | 462 | 17 | 0 | 0 | 14 | 522 | 680 | 32 | -3 | 10 | 14 | 112 | 118 | 11 | 1 | 1 | 15 | 347 | 358 | 13 |
| 2 | 20 | 12 | 135 | 148 | 22 | 0 | 9 | 13 | 112 | 101 | 13 | 1 | 0 | 14 | 50 | 60 | 25 | -2 | 10 | 14 | 0 | 25 | 1 | 2 | 1 | 15 | 457 | 437 | 20 |
| -1 | 21 | 12 | 18 | 25 | 17 | 1 | 9 | 13 | 469 | 462 | 30 | 2 | 0 | 14 | 411 | 385 | 18 | -1 | 10 | 14 | 248 | 261 | 11 | 3 | 1 | 15 | 241 | 227 | 19 |
| 0 | 21 | 12 | 64 | 72 | 36 | 2 | 9 | 13 | 241 | 238 | 9 | 3 | 0 | 14 | 42 | 63 | 41 | 0 | 10 | 14 | 223 | 213 | 9 | 4 | 1 | 15 | 107 | 107 | 14 |
| 1 | 21 | 12 | 0 | 25 | 1 | 3 | 9 | 13 | 135 | 115 | 12 | 4 | 0 | 14 | 26 | 29 | 25 | 1 | 10 | 14 | 246 | 261 | 9 | -4 | 2 | 15 | 75 | 39 | 32 |
| -1 | 22 | 12 | 5 | 50 | 5 | 4 | 9 | 13 | 48 | 46 | 47 | -4 | 1 | 14 | 95 | 98 | 24 | 2 | 10 | 14 | 51 | 26 | 50 | -3 | 2 | 15 | 195 | 199 | 15 |
| 0 | 22 | 12 | 120 | 127 | 14 | -4 | 10 | 13 | 81 | 75 | 33 | -3 | 1 | 14 | 178 | 161 | 11 | 3 | 10 | 14 | 106 | 117 | 16 | -2 | 2 | 15 | 233 | 227 | 8 |
| 1 | 22 | 12 | 48 | 50 | 47 | -3 | 10 | 13 | 17 | 26 | 17 | -2 | 1 | 14 | 274 | 285 | 13 | 4 | 10 | 14 | 150 | 126 | 13 | -1 | 2 | 15 | 218 | 230 | 9 |
| 1 | 0 | 13 | 32 | 72 | 32 | -2 | 10 | 13 | 82 | 100 | 24 | -1 | 1 | 14 | 86 | 95 | 12 | -4 | 11 | 14 | 74 | 80 | 37 | 0 | 2 | 15 | 43 | 65 | 26 |
| 2 | 0 | 13 | 143 | 137 | 11 | -1 | 10 | 13 | 106 | 96 | 25 | 0 | 1 | 14 | 215 | 260 | 7 | -3 | 11 | 14 | 118 | 130 | 10 | 1 | 2 | 15 | 198 | 229 | 10 |
| 3 | 0 | 13 | 205 | 214 | 14 | 0 | 10 | 13 | 124 | 124 | 13 | 1 | 1 | 14 | 88 | 94 | 11 | -2 | 11 | 14 | 61 | 84 | 32 | 2 | 2 | 15 | 265 | 227 | 14 |
| 4 | 0 | 13 | 125 | 86 | 25 | 1 | 10 | 13 | 111 | 96 | 11 | 2 | 1 | 14 | 265 | 285 | 13 | -1 | 11 | 14 | 316 | 302 | 12 | 3 | 2 | 15 | 197 | 198 | 11 |
| -4 | 1 | 13 | 186 | 158 | 25 | 2 | 10 | 13 | 107 | 100 | 13 | 3 | 1 | 14 | 186 | 161 | 11 | 0 | 11 | 14 | 242 | 238 | 11 | 4 | 2 | 15 | 65 | 40 | 64 |
| -3 | 1 | 13 | 73 | 30 | 19 | 3 | 10 | 13 | 0 | 26 | 1 | 4 | 1 | 14 | 121 | 98 | 17 | 1 | 11 | 14 | 303 | 303 | 10 | -4 | 3 | 15 | 137 | 157 | 18 |
| -2 | 1 | 13 | 187 | 185 | 11 | 4 | 10 | 13 | 79 | 75 | 16 | -4 | 2 | 14 | 91 | 84 | 26 | 2 | 11 | 14 | 105 | 84 | 15 | -3 | 3 | 15 | 78 | 79 | 23 |
| -1 | 1 | 13 | 176 | 202 | 9 | -4 | 11 | 13 | 85 | 12 | 31 | -3 | 2 | 14 | 133 | 104 | 20 | 3 | 11 | 14 | 116 | 131 | 14 | -2 | 3 | 15 | 79 | 81 | 13 |
| 0 | 1 | 13 | 240 | 260 | 8 | -3 | 11 | 13 | 182 | 180 | 9 | -2 | 2 | 14 | 446 | 399 | 19 | 4 | 11 | 14 | 75 | 80 | 17 | -1 | 3 | 15 | 521 | 505 | 18 |
| 1 | 1 | 13 | 165 | 203 | 9 | -2 | 11 | 13 | 156 | 161 | 11 | -1 | 2 | 14 | 161 | 159 | 10 | -3 | 12 | 14 | 186 | 189 | 13 | 0 | 3 | 15 | 260 | 302 | 9 |
| 2 | 1 | 13 | 187 | 186 | 11 | -1 | 11 | 13 | 322 | 306 | 8 | 0 | 2 | 14 | 350 | 385 | 11 | -2 | 12 | 14 | 219 | 230 | 10 | 1 | 3 | 15 | 511 | 504 | 18 |
| 3 | 1 | 13 | 46 | 30 | 46 | 0 | 11 | 13 | 85 | 97 | 20 | 1 | 2 | 14 | 152 | 160 | 9 | -1 | 12 | 14 | 64 | 37 | 34 | 2 | 3 | 15 | 79 | 81 | 10 |
| 4 | 1 | 13 | 164 | 158 | 46 | 1 | 11 | 13 | 302 | 305 | 12 | 2 | 2 | 14 | 434 | 399 | 20 | 0 | 12 | 14 | 140 | 140 | 15 | 3 | 3 | 15 | 81 | 79 | 39 |
| -4 | 2 | 13 | 151 | 135 | 17 | 2 | 11 | 13 | 156 | 161 | 11 | 3 | 2 | 14 | 77 | 104 | 24 | 1 | 12 | 14 | 44 | 37 | 44 | 4 | 3 | 15 | 161 | 156 | 32 |
| -3 | 2 | 13 | 159 | 141 | 16 | 3 | 11 | 13 | 180 | 181 | 11 | 4 | 2 | 14 | 73 | 83 | 31 | 2 | 12 | 14 | 206 | 230 | 12 | -4 | 4 | 15 | 48 | 21 | 48 |
| -2 | 2 | 13 | 428 | 406 | 19 | 4 | 11 | 13 | 41 | 12 | 41 | -4 | 3 | 14 | 103 | 100 | 25 | 3 | 12 | 14 | 174 | 189 | 11 | -3 | 4 | 15 | 132 | 139 | 13 |
| -1 | 2 | 13 | 174 | 199 | 16 | -4 | 12 | 13 | 106 | 95 | 46 | -3 | 3 | 14 | 221 | 208 | 9 | -3 | 13 | 14 | 0 | 35 | 1 | -2 | 4 | 15 | 109 | 121 | 19 |
| 0 | 2 | 13 | 153 | 186 | 7 | -3 | 12 | 13 | 178 | 181 | 24 | -2 | 3 | 14 | 145 | 131 | 11 | -2 | 13 | 14 | 233 | 193 | 10 | -1 | 4 | 15 | 446 | 445 | 16 |
| 1 | 2 | 13 | 180 | 199 | 9 | -2 | 12 | 13 | 36 | 32 | 35 | -1 | 3 | 14 | 159 | 180 | 9 | -1 | 13 | 14 | 43 | 25 | 42 | 0 | 4 | 15 | 12 | 30 | 11 |
| 2 | 2 | 13 | 427 | 406 | 28 | -1 | 12 | 13 | 229 | 217 | 8 | 0 | 3 | 14 | 85 | 81 | 10 | 0 | 13 | 14 | 144 | 103 | 11 | 1 | 4 | 15 | 449 | 445 | 20 |
| 3 | 2 | 13 | 148 | 140 | 12 | 0 | 12 | 13 | 217 | 217 | 8 | 1 | 3 | 14 | 168 | 180 | 9 | 1 | 13 | 14 | 73 | 26 | 19 | 2 | 4 | 15 | 99 | 121 | 15 |
| 4 | 2 | 13 | 145 | 134 | 53 | 1 | 12 | 13 | 222 | 217 | 8 | 2 | 3 | 14 | 94 | 131 | 41 | 2 | 13 | 14 | 210 | 193 | 13 | 3 | 4 | 15 | 139 | 139 | 14 |
| -4 | 3 | 13 | 101 | 70 | 24 | 2 | 12 | 13 | 37 | 33 | 36 | 3 | 3 | 14 | 226 | 208 | 11 | 3 | 13 | 14 | 52 | 35 | 52 | 4 | 4 | 15 | 58 | 22 | 46 |
| -3 | 3 | 13 | 64 | 65 | 21 | 3 | 12 | 13 | 171 | 181 | 25 | 4 | 3 | 14 | 100 | 100 | 46 | -3 | 14 | 14 | 61 | 55 | 37 | -4 | 5 | 15 | 91 | 109 | 25 |
| -2 | 3 | 13 | 508 | 488 | 23 | -3 | 13 | 13 | 141 | 154 | 13 | -4 | 4 | 14 | 83 | 15 | 28 | -2 | 14 | 14 | 109 | 100 | 13 | -3 | 5 | 15 | 221 | 184 | 19 |
| -1 | 3 | 13 | 139 | 106 | 11 | -2 | 13 | 13 | 103 | 105 | 19 | -3 | 4 | 14 | 189 | 168 | 14 | -1 | 14 | 14 | 0 | 27 | 1 | -2 | 5 | 15 | 187 | 177 | 7 |
| 0 | 3 | 13 | 1000 | 1007 | 35 | -1 | 13 | 13 | 88 | 60 | 16 | -2 | 4 | 14 | 856 | 821 | 33 | 0 | 14 | 14 | 341 | 329 | 12 | -1 | 5 | 15 | 325 | 315 | 14 |
| 1 | 3 | 13 | 125 | 106 | 9 | 0 | 13 | 13 | 166 | 161 | 9 | -1 | 4 | 14 | 127 | 80 | 20 | 1 | 14 | 14 | 63 | 28 | 34 | 0 | 5 | 15 | 175 | 167 | 11 |
| 2 | 3 | 13 | 525 | 488 | 32 | 1 | 13 | 13 | 41 | 61 | 40 | 0 | 4 | 14 | 0 | 41 | 1 | 2 | 14 | 19 | 69 | 100 | 30 | 1 | 5 | 15 | 331 | 315 | 15 |
| 3 | 3 | 13 | 73 | 65 | 28 | 2 | 13 | 13 | 100 | 106 | 18 | 1 | 4 | 14 | 114 | 79 | 22 | 3 | 14 | 14 | 67 | 55 | 25 | 2 | 5 | 15 | 181 | 176 | 10 |
| 4 | 3 | 13 | 66 | 69 | 37 | 3 | 13 | 13 | 141 | 154 | 13 | 2 | 4 | 14 | 887 | 821 | 39 | -3 | 15 | 14 | 30 | 35 | 30 | 3 | 5 | 15 | 197 | 182 | 12 |
| -4 | 4 | 13 | 187 | 175 | 15 | -3 | 14 | 13 | 129 | 119 | 14 | 3 | 4 | 14 | 173 | 168 | 12 | -2 | 15 | 14 | 159 | 171 | 28 | 4 | 5 | 15 | 105 | 109 | 18 |
| -3 | 4 | 13 | 65 | 64 | 26 | -2 | 14 | 13 | 229 | 206 | 12 | 4 | 4 | 14 | 65 | 15 | 36 | -1 | 15 | 14 | 138 | 127 | 11 | -4 | 6 | 15 | 113 | 111 | 19 |
| -2 | 4 | 13 | 237 | 222 | 17 | -1 | 14 | 13 | 97 | 82 | 16 | -4 | 5 | 14 | 124 | 87 | 22 | 0 | 15 | 14 | 50 | 2 | 50 | -3 | 6 | 15 | 242 | 230 | 20 |
| -1 | 4 | 13 | 293 | 300 | 12 | 0 | 14 | 13 | 60 | 30 | 28 | -3 | 5 | 14 | 60 | 52 | 60 | 1 | 15 | 14 | 147 | 127 | 12 | -2 | 6 | 15 | 334 | 328 | 9 |
| 0 | 4 | 13 | 501 | 497 | 15 | 1 | 14 | 13 | 103 | 82 | 15 | -2 | 5 | 14 | 355 | 357 | 11 | 2 | 15 | 14 | 143 | 172 | 13 | -1 | 6 | 15 | 0 | 47 | 1 |
| 1 | 4 | 13 | 301 | 299 | 13 | 2 | 14 | 13 | 216 | 206 | 12 | -1 | 5 | 14 | 374 | 365 | 14 | 3 | 15 | 14 | 64 | 35 | 28 | 0 | 6 | 15 | 165 | 165 | 9 |
| 2 | 4 | 13 | 223 | 223 | 22 | 3 | 14 | 13 | 131 | 119 | 20 | 0 | 5 | 14 | 104 | 109 | 10 | -3 | 16 | 14 | 118 | 123 | 17 | 1 | 6 | 15 | 62 | 45 | 31 |
| 3 | 4 | 13 | 81 | 64 | 25 | -3 | 15 | 13 | 137 | 128 | 27 | 1 | 5 | 14 | 373 | 364 | 17 | -2 | 16 | 14 | 83 | 91 | 16 | 2 | 6 | 15 | 350 | 328 | 14 |
| 4 | 4 | 13 | 159 | 176 | 38 | -2 | 15 | 13 | 121 | 119 | 21 | 2 | 5 | 14 | 349 | 357 | 15 | -1 | 16 | 14 | 93 | 75 | 19 | 3 | 6 | 15 | 227 | 230 | 10 |
| -4 | 5 | 13 | 147 | 87 | 49 | -1 | 15 | 13 | 154 | 110 | 13 | 3 | 5 | 14 | 43 | 51 | 42 | 0 | 16 | 14 | 315 | 315 | 13 | 4 | 6 | 15 | 89 | 111 | 16 |
| -3 | 5 | 13 | 182 | 169 | 11 | 0 | 15 | 13 | 193 | 161 | 10 | 4 | 5 | 14 | 71 | 86 | 50 | 1 | 16 | 14 | 67 | 74 | 40 | -4 | 7 | 15 | 79 | 87 | 33 |
| -2 | 5 | 13 | 298 | 283 | 8 | 1 | 15 | 13 | 92 | 109 | 20 | -4 | 6 | 14 | 91 | 113 | 29 | 2 | 16 | 14 | 83 | 91 | 21 | -3 | 7 | 15 | 136 | 135 | 18 |
| -1 | 5 | 13 | 160 | 168 | 9 | 2 | 15 | 13 | 107 | 119 | 18 | -3 | 6 | 14 | 106 | 114 | 10 | 3 | 16 | 14 | 143 | 123 | 12 | -2 | 7 | 15 | 68 | 66 | 22 |
| 0 | 5 | 13 | 58 | 79 | 17 | 3 | 15 | 13 | 111 | 127 | 15 | -2 | 6 | 14 | 121 | 109 | 9 | -3 | 17 | 14 | 112 | 125 | 55 | -1 | 7 | 15 | 184 | 186 | 11 |
| 1 | 5 | 13 | 158 | 166 | 12 | -3 | 16 | 13 | 65 | 58 | 52 | -1 | 6 | 14 | 452 | 428 | 16 | -2 | 17 | 14 | 139 | 137 | 12 | 0 | 7 | 15 | 107 | 115 | 12 |
| 2 | 5 | 13 | 295 | 281 | 17 | -2 | 16 | 13 | 158 | 152 | 15 | 0 | 6 | 14 | 112 | 109 | 11 | -1 | 17 | 14 | 83 | 61 | 18 | 1 | 7 | 15 | 210 | 185 | 29 |
| 3 | 5 | 13 | 167 | 170 | 12 | -1 | 16 | 13 | 225 | 207 | 9 | 1 | 6 | 14 | 446 | 427 | 20 | 0 | 17 | 14 | 112 | 122 | 21 | 2 | 7 | 15 | 44 | 66 | 44 |
| 4 | 5 | 13 | 71 | 87 | 47 | 0 | 16 | 13 | 171 | 171 | 16 | 2 | 6 | 14 | 127 | 109 | 12 | 1 | 17 | 14 | 60 | 61 | 38 | 3 | 7 | 15 | 121 | 134 | 13 |
| -4 | 6 | 13 | 17 | 21 | 17 | 1 | 16 | 13 | 196 | 207 | 14 | 3 | 6 | 14 | 115 | 115 | 15 | 2 | 17 | 14 | 128 | 138 | 18 | 4 | 7 | 15 | 62 | 88 | 22 |
| -3 | 6 | 13 | 53 | 67 | 36 | 2 | 16 | 13 | 147 | 153 | 15 | 4 | 6 | 14 | 109 | 114 | 17 | 3 | 17 | 14 | 141 | 125 | 14 | -4 | 8 | 15 | 109 | 123 | 21 |
| -2 | 6 | 13 | 153 | 161 | 7 | 3 | 16 | 13 | 68 | 58 | 28 | -4 | 7 | 14 | 103 | 93 | 22 | -2 | 18 | 14 | 128 | 143 | 11 | -3 | 8 | 15 | 267 | 309 | 11 |
| -1 | 6 | 13 | 450 | 417 | 20 | -3 | 17 | 13 | 119 | 107 | 20 | -3 | 7 | 14 | 48 | 34 | 47 | -1 | 18 | 14 | 76 | 101 | 20 | -2 | 8 | 15 | 0 | 24 | 1 |
| 0 | 6 | 13 | 221 | 238 | 9 | -2 | 17 | 13 | 110 | 128 | 12 | -2 | 7 | 14 | 240 | 203 | 8 | 0 | 18 | 14 | 139 | 130 | 14 | -1 | 8 | 15 | 191 | 182 | 15 |
| 1 | 6 | 13 | 452 | 417 | 20 | -1 | 17 | 13 | 114 | 138 | 13 | -1 | 7 | 14 | 112 | 101 | 13 | 1 | 18 | 14 | 110 | 102 | 15 | 0 | 8 | 15 | 191 | 210 | 10 |
| 2 | 6 | 13 | 157 | 162 | 18 | 0 | 17 | 13 | 0 | 69 | 1 | 0 | 7 | 14 | 189 | 209 | 9 | 2 | 18 | 14 | 100 | 142 | 51 | 1 | 8 | 15 | 193 | 181 | 19 |
| 3 | 6 | 13 | 52 | 66 | 51 | 1 | 17 | 13 | 122 | 138 | 15 | 1 | 7 | 14 | 102 | 100 | 17 | -2 | 19 | 14 | 50 | 45 | 42 | 2 | 8 | 15 | 63 | 25 | 25 |
| 4 | 6 | 13 | 52 | 21 | 34 | 2 | 17 | 13 | 129 | 128 | 14 | 2 | 7 | 14 | 253 | 203 | 10 | -1 | 19 | 14 | 195 | 212 | 11 | 3 | 8 | 15 | 295 | 308 | 11 |
| -4 | 7 | 13 | 190 | 196 | 16 | 3 | 17 | 13 | 93 | 106 | 18 | 3 | 7 | 14 | 0 | 35 | 1 | 0 | 19 | 14 | 143 | 172 | 21 | 4 | 8 | 15 | 120 | 123 | 11 |
| -3 | 7 | 13 | 27 | 62 | 27 | -2 | 18 | 13 | 85 | 103 | 15 | 4 | 7 | 14 | 82 | 92 | 17 | 1 | 19 | 14 | 205 | 212 | 12 | -4 | 9 | 15 | 57 | 59 | 57 |
| -2 | 7 | 13 | 362 | 352 | 10 | -1 | 18 | 13 | 171 | 144 | 25 | -4 | 8 | 14 | 65 | 61 | 64 | 2 | 19 | 14 | 88 | 44 | 29 | -3 | 9 | 15 | 198 | 200 | 9 |
| -1 | 7 | 13 | 473 | 475 | 17 | 0 | 18 | 13 | 206 | 186 | 16 | -3 | 8 | 14 | 94 | 77 | 13 | -2 | 20 | 14 | 0 | 1 | 1 | -2 | 9 | 15 | 180 | 179 | 8 |
| 0 | 7 | 13 | 376 | 357 | 14 | 1 | 18 | 13 | 131 | 144 | 14 | -2 | 8 | 14 | 197 | 172 | 8 | -1 | 20 | 14 | 101 | 90 | 21 | -1 | 9 | 15 | 284 | 263 | 9 |
| 1 | 7 | 13 | 488 | 474 | 21 | 2 | 18 | 13 | 67 | 103 | 67 | -1 | 8 | 14 | 0 | 19 | 1 | 0 | 20 | 14 | 0 | 5 | 1 | 0 | 9 | 15 | 501 | 486 | 18 |
| 2 | 7 | 13 | 372 | 352 | 11 | -2 | 19 | 13 | 68 | 76 | 21 | 0 | 8 | 14 | 36 | 43 | 35 | 1 | 20 | 14 | 83 | 90 | 21 | 1 | 9 | 15 | 270 | 263 | 10 |
| 3 | 7 | 13 | 58 | 62 | 33 | -1 | 19 | 13 | 109 | 116 | 13 | 1 | 8 | 14 | 62 | 19 | 62 | 2 | 20 | 14 | 55 | 2 | 55 | 2 | 9 | 15 | 173 | 180 | 10 |
| 4 | 7 | 13 | 199 | 197 | 11 | 0 | 19 | 13 | 209 | 223 | 35 | 2 | 8 | 14 | 205 | 172 | 9 | -1 | 21 | 14 | 95 | 72 | 17 | 3 | 9 | 15 | 205 | 201 | 13 |
| -4 | 8 | 13 | 91 | 68 | 29 | 1 | 19 | 13 | 120 | 116 | 14 | 3 | 8 | 14 | 160 | 77 | 32 | 0 | 21 | 14 | 0 | 6 | 1 | 4 | 9 | 15 | 41 | 59 | 41 |
| -3 | 8 | 13 | 139 | 114 | 41 | 2 | 19 | 13 | 51 | 75 | 50 | 4 | 8 | 14 | 42 | 61 | 42 | 1 | 21 | 14 | 79 | 72 | 21 | -4 | 10 | 15 | 76 | 60 | 36 |
| -2 | 8 | 13 | 260 | 239 | 8 | -2 | 20 | 13 | 0 | 51 | 1 | -4 | 9 | 14 | 117 | 135 | 21 | 0 | 22 | 14 | 67 | 23 | 27 | -3 | 10 | 15 | 23 | 17 | 23 |
| -1 | 8 | 13 | 79 | 60 | 19 | -1 | 20 | 13 | 71 | 42 | 22 | -3 | 9 | 14 | 27 | 35 | 27 | 1 | 0 | 15 | 42 | 69 | 42 | -2 | 10 | 15 | 198 | 198 | 15 |
| 0 | 8 | 13 | 141 | 134 | 10 | 0 | 20 | 13 | 63 | 58 | 37 | -2 | 9 | 14 | 135 | 149 | 9 | 2 | 0 | 15 | 194 | 162 | 12 | -1 | 10 | 15 | 246 | 227 | 7 |
| 1 | 8 | 13 | 81 | 60 | 23 | 1 | 20 | 13 | 78 | 42 | 59 | -1 | 9 | 14 | 224 | 224 | 11 | 3 | 0 | 15 | 233 | 215 | 9 | 0 | 10 | 15 | 30 | 1 | 30 |
| 2 | 8 | 13 | 248 | 239 | 9 | 2 | 20 | 13 | 29 | 51 | 28 | 0 | 9 | 14 | 101 | 106 | 14 | 4 | 0 | 15 | 261 | 249 | 21 | 1 | 10 | 15 | 251 | 227 | 8 |
| 2 | 10 | 15 | 189 | 197 | 11 | 0 | 2 | 16 | 284 | 284 | 9 | 0 | 12 | 16 | 136 | 146 | 14 | -1 | 4 | 17 | 264 | 247 | 12 | -2 | 15 | 17 | 118 | 128 | 17 |
| 3 | 10 | 15 | 0 | 16 | 1 | 1 | 2 | 16 | 391 | 412 | 17 | 1 | 12 | 16 | 206 | 214 | 9 | 0 | 4 | 17 | 148 | 138 | 9 | -1 | 15 | 17 | 108 | 71 | 14 |
| 4 | 10 | 15 | 62 | 60 | 22 | 2 | 2 | 16 | 292 | 314 | 11 | 2 | 12 | 16 | 151 | 83 | 49 | 1 | 4 | 17 | 264 | 247 | 13 | 0 | 15 | 17 | 47 | 12 | 47 |
| -4 | 11 | 15 | 0 | 26 | 1 | 3 | 2 | 16 | 119 | 124 | 15 | 3 | 12 | 16 | 48 | 68 | 47 | 2 | 4 | 17 | 73 | 47 | 12 | 1 | 15 | 17 | 89 | 71 | 19 |
| -3 | 11 | 15 | 109 | 91 | 11 | 4 | 2 | 16 | 57 | 52 | 56 | -3 | 13 | 16 | 107 | 97 | 11 | 3 | 4 | 17 | 27 | 67 | 26 | 2 | 15 | 17 | 78 | 127 | 23 |
| -2 | 11 | 15 | 79 | 84 | 21 | -4 | 3 | 16 | 61 | 69 | 61 | -2 | 13 | 16 | 238 | 241 | 14 | 4 | 4 | 17 | 95 | 121 | 20 | 3 | 15 | 17 | 0 | 51 | 1 |
| -1 | 11 | 15 | 225 | 200 | 8 | -3 | 3 | 16 | 150 | 154 | 13 | -1 | 13 | 16 | 194 | 197 | 9 | -4 | 5 | 17 | 0 | 23 | 1 | -3 | 16 | 17 | 0 | 53 | 1 |
| 0 | 11 | 15 | 184 | 168 | 8 | -2 | 3 | 16 | 375 | 368 | 10 | 0 | 13 | 16 | 259 | 266 | 12 | -3 | 5 | 17 | 244 | 256 | 12 | -2 | 16 | 17 | 184 | 208 | 23 |
| 1 | 11 | 15 | 223 | 200 | 8 | -1 | 3 | 16 | 266 | 278 | 11 | 1 | 13 | 16 | 190 | 198 | 10 | -2 | 5 | 17 | 185 | 177 | 8 | -1 | 16 | 17 | 114 | 38 | 24 |
| 2 | 11 | 15 | 96 | 83 | 19 | 0 | 3 | 16 | 219 | 223 | 8 | 2 | 13 | 16 | 239 | 241 | 12 | -1 | 5 | 17 | 156 | 166 | 16 | 0 | 16 | 17 | 99 | 48 | 23 |
| 3 | 11 | 15 | 73 | 91 | 21 | 1 | 3 | 16 | 265 | 277 | 13 | 3 | 13 | 16 | 102 | 97 | 15 | 0 | 5 | 17 | 715 | 711 | 31 | 1 | 16 | 17 | 79 | 38 | 23 |
| -3 | 12 | 15 | 125 | 127 | 13 | 2 | 3 | 16 | 368 | 368 | 11 | -3 | 14 | 16 | 126 | 115 | 14 | 1 | 5 | 17 | 150 | 166 | 13 | 2 | 16 | 17 | 153 | 207 | 19 |
| -2 | 12 | 15 | 124 | 114 | 20 | 3 | 3 | 16 | 166 | 154 | 12 | -2 | 14 | 16 | 137 | 152 | 14 | 2 | 5 | 17 | 183 | 176 | 9 | -2 | 17 | 17 | 119 | 139 | 16 |
| -1 | 12 | 15 | 171 | 169 | 13 | 4 | 3 | 16 | 75 | 68 | 19 | -1 | 14 | 16 | 261 | 240 | 9 | 3 | 5 | 17 | 255 | 255 | 12 | -1 | 17 | 17 | 141 | 137 | 10 |
| 0 | 12 | 15 | 233 | 215 | 10 | -4 | 4 | 16 | 132 | 138 | 17 | 0 | 14 | 16 | 0 | 10 | 1 | 4 | 5 | 17 | 0 | 23 | 1 | 0 | 17 | 17 | 82 | 54 | 53 |
| 1 | 12 | 15 | 192 | 170 | 8 | -3 | 4 | 16 | 88 | 107 | 20 | 1 | 14 | 16 | 257 | 239 | 12 | -4 | 6 | 17 | 108 | 134 | 19 | 1 | 17 | 17 | 150 | 137 | 12 |
| 2 | 12 | 15 | 114 | 113 | 16 | -2 | 4 | 16 | 199 | 198 | 9 | 2 | 14 | 16 | 136 | 152 | 14 | -3 | 6 | 17 | 174 | 190 | 12 | 2 | 17 | 17 | 129 | 139 | 84 |
| 3 | 12 | 15 | 121 | 128 | 12 | -1 | 4 | 16 | 420 | 415 | 16 | 3 | 14 | 16 | 91 | 115 | 17 | -2 | 6 | 17 | 69 | 69 | 18 | -2 | 18 | 17 | 146 | 148 | 15 |
| -3 | 13 | 15 | 101 | 89 | 36 | 0 | 4 | 16 | 426 | 432 | 15 | -3 | 15 | 16 | 62 | 40 | 62 | -1 | 6 | 17 | 34 | 48 | 33 | -1 | 18 | 17 | 48 | 54 | 48 |
| -2 | 13 | 15 | 143 | 128 | 11 | 1 | 4 | 16 | 439 | 415 | 18 | -2 | 15 | 16 | 87 | 107 | 26 | 0 | 6 | 17 | 127 | 128 | 17 | 0 | 18 | 17 | 0 | 40 | 1 |
| -1 | 13 | 15 | 89 | 71 | 16 | 2 | 4 | 16 | 193 | 199 | 8 | -1 | 15 | 16 | 172 | 142 | 10 | 1 | 6 | 17 | 74 | 49 | 25 | 1 | 18 | 17 | 39 | 55 | 38 |
| 0 | 13 | 15 | 185 | 163 | 10 | 3 | 4 | 16 | 104 | 108 | 18 | 0 | 15 | 16 | 0 | 15 | 1 | 2 | 6 | 17 | 79 | 69 | 28 | 2 | 18 | 17 | 170 | 149 | 17 |
| 1 | 13 | 15 | 94 | 72 | 18 | 4 | 4 | 16 | 124 | 138 | 18 | 1 | 15 | 16 | 155 | 141 | 13 | 3 | 6 | 17 | 176 | 190 | 10 | -2 | 19 | 17 | 5 | 58 | 4 |
| 2 | 13 | 15 | 116 | 128 | 17 | -4 | 5 | 16 | 76 | 41 | 31 | 2 | 15 | 16 | 96 | 107 | 21 | 4 | 6 | 17 | 139 | 135 | 22 | -1 | 19 | 17 | 63 | 18 | 23 |
| 3 | 13 | 15 | 83 | 89 | 17 | -3 | 5 | 16 | 223 | 216 | 14 | 3 | 15 | 16 | 57 | 40 | 38 | -4 | 7 | 17 | 67 | 55 | 38 | 0 | 19 | 17 | 60 | 91 | 34 |
| -3 | 14 | 15 | 87 | 91 | 21 | -2 | 5 | 16 | 45 | 40 | 45 | -3 | 16 | 16 | 38 | 49 | 37 | -3 | 7 | 17 | 122 | 135 | 10 | 1 | 19 | 17 | 0 | 19 | 1 |
| -2 | 14 | 15 | 238 | 263 | 12 | -1 | 5 | 16 | 220 | 212 | 10 | -2 | 16 | 16 | 133 | 120 | 21 | -2 | 7 | 17 | 194 | 185 | 8 | 2 | 19 | 17 | 67 | 58 | 54 |
| -1 | 14 | 15 | 226 | 210 | 9 | 0 | 5 | 16 | 39 | 19 | 39 | -1 | 16 | 16 | 274 | 273 | 10 | -1 | 7 | 17 | 106 | 116 | 16 | -1 | 20 | 17 | 51 | 32 | 50 |
| 0 | 14 | 15 | 215 | 182 | 15 | 1 | 5 | 16 | 213 | 213 | 17 | 0 | 16 | 16 | 61 | 59 | 44 | 0 | 7 | 17 | 468 | 487 | 17 | 0 | 20 | 17 | 139 | 130 | 12 |
| 1 | 14 | 15 | 215 | 209 | 15 | 2 | 5 | 16 | 61 | 39 | 61 | 1 | 16 | 16 | 280 | 272 | 15 | 1 | 7 | 17 | 116 | 116 | 9 | 1 | 20 | 17 | 45 | 32 | 45 |
| 2 | 14 | 15 | 237 | 263 | 16 | 3 | 5 | 16 | 210 | 216 | 12 | 2 | 16 | 16 | 91 | 120 | 30 | 2 | 7 | 17 | 188 | 184 | 9 | 0 | 21 | 17 | 0 | 3 | 1 |
| 3 | 14 | 15 | 113 | 90 | 17 | 4 | 5 | 16 | 60 | 41 | 38 | 3 | 16 | 16 | 58 | 49 | 42 | 3 | 7 | 17 | 151 | 135 | 10 | 0 | 0 | 18 | 125 | 87 | 18 |
| -3 | 15 | 15 | 76 | 54 | 32 | -4 | 6 | 16 | 109 | 110 | 18 | -2 | 17 | 16 | 85 | 97 | 15 | 4 | 7 | 17 | 33 | 55 | 32 | 1 | 0 | 18 | 64 | 78 | 23 |
| -2 | 15 | 15 | 95 | 65 | 13 | -3 | 6 | 16 | 290 | 283 | 13 | -1 | 17 | 16 | 70 | 73 | 21 | -4 | 8 | 17 | 50 | 69 | 50 | 2 | 0 | 18 | 225 | 235 | 7 |
| -1 | 15 | 15 | 131 | 112 | 15 | -2 | 6 | 16 | 115 | 116 | 11 | 0 | 17 | 16 | 0 | 7 | 1 | -3 | 8 | 17 | 204 | 195 | 16 | 3 | 0 | 18 | 210 | 173 | 10 |
| 0 | 15 | 15 | 295 | 251 | 16 | -1 | 6 | 16 | 349 | 355 | 13 | 1 | 17 | 16 | 85 | 74 | 20 | -2 | 8 | 17 | 164 | 167 | 9 | 4 | 0 | 18 | 142 | 217 | 20 |
| 1 | 15 | 15 | 145 | 113 | 15 | 0 | 6 | 16 | 189 | 189 | 9 | 2 | 17 | 16 | 104 | 98 | 40 | -1 | 8 | 17 | 181 | 166 | 16 | -4 | 1 | 18 | 110 | 121 | 23 |
| 2 | 15 | 15 | 74 | 65 | 73 | 1 | 6 | 16 | 356 | 355 | 16 | -2 | 18 | 16 | 18 | 21 | 17 | 0 | 8 | 17 | 193 | 193 | 15 | -3 | 1 | 18 | 313 | 294 | 22 |
| 3 | 15 | 15 | 96 | 54 | 16 | 2 | 6 | 16 | 104 | 117 | 15 | -1 | 18 | 16 | 90 | 119 | 14 | 1 | 8 | 17 | 177 | 165 | 8 | -2 | 1 | 18 | 98 | 95 | 10 |
| -3 | 16 | 15 | 118 | 102 | 20 | 3 | 6 | 16 | 302 | 283 | 14 | 0 | 18 | 16 | 115 | 109 | 14 | 2 | 8 | 17 | 180 | 167 | 9 | -1 | 1 | 18 | 51 | 55 | 34 |
| -2 | 16 | 15 | 180 | 224 | 22 | 4 | 6 | 16 | 109 | 111 | 21 | 1 | 18 | 16 | 100 | 119 | 17 | 3 | 8 | 17 | 216 | 195 | 10 | 0 | 1 | 18 | 139 | 126 | 9 |
| -1 | 16 | 15 | 240 | 232 | 9 | -4 | 7 | 16 | 47 | 67 | 47 | 2 | 18 | 16 | 0 | 21 | 1 | 4 | 8 | 17 | 40 | 69 | 39 | 1 | 1 | 18 | 77 | 55 | 33 |
| 0 | 16 | 15 | 43 | 18 | 43 | -3 | 7 | 16 | 310 | 313 | 11 | -2 | 19 | 16 | 74 | 97 | 19 | -4 | 9 | 17 | 27 | 44 | 27 | 2 | 1 | 18 | 98 | 94 | 9 |
| 1 | 16 | 15 | 229 | 232 | 14 | -2 | 7 | 16 | 175 | 171 | 8 | -1 | 19 | 16 | 124 | 134 | 13 | -3 | 9 | 17 | 288 | 308 | 12 | 3 | 1 | 18 | 303 | 295 | 12 |
| 2 | 16 | 15 | 187 | 224 | 31 | -1 | 7 | 16 | 88 | 102 | 18 | 0 | 19 | 16 | 76 | 82 | 30 | -2 | 9 | 17 | 54 | 69 | 51 | 4 | 1 | 18 | 126 | 121 | 12 |
| 3 | 16 | 15 | 97 | 102 | 17 | 0 | 7 | 16 | 158 | 176 | 10 | 1 | 19 | 16 | 158 | 135 | 18 | -1 | 9 | 17 | 105 | 88 | 19 | -4 | 2 | 18 | 120 | 135 | 31 |
| -2 | 17 | 15 | 86 | 86 | 14 | 1 | 7 | 16 | 109 | 101 | 17 | 2 | 19 | 16 | 99 | 96 | 26 | 0 | 9 | 17 | 465 | 487 | 15 | -3 | 2 | 18 | 185 | 176 | 17 |
| -1 | 17 | 15 | 0 | 6 | 1 | 2 | 7 | 16 | 173 | 170 | 9 | -1 | 20 | 16 | 82 | 78 | 19 | 1 | 9 | 17 | 104 | 89 | 11 | -2 | 2 | 18 | 103 | 108 | 10 |
| 0 | 17 | 15 | 313 | 305 | 13 | 3 | 7 | 16 | 315 | 314 | 12 | 0 | 20 | 16 | 82 | 97 | 21 | 2 | 9 | 17 | 85 | 70 | 36 | -1 | 2 | 18 | 189 | 202 | 10 |
| 1 | 17 | 15 | 46 | 6 | 46 | 4 | 7 | 16 | 53 | 67 | 28 | 1 | 20 | 16 | 71 | 78 | 36 | 3 | 9 | 17 | 305 | 308 | 14 | 0 | 2 | 18 | 769 | 776 | 27 |
| 2 | 17 | 15 | 54 | 86 | 54 | -4 | 8 | 16 | 87 | 64 | 28 | -1 | 21 | 16 | 23 | 24 | 22 | 4 | 9 | 17 | 43 | 44 | 42 | 1 | 2 | 18 | 196 | 202 | 12 |
| -2 | 18 | 15 | 66 | 94 | 21 | -3 | 8 | 16 | 223 | 232 | 9 | 0 | 21 | 16 | 0 | 16 | 1 | -3 | 10 | 17 | 101 | 89 | 12 | 2 | 2 | 18 | 113 | 108 | 7 |
| -1 | 18 | 15 | 98 | 112 | 13 | -2 | 8 | 16 | 163 | 153 | 8 | 1 | 21 | 16 | 49 | 24 | 49 | -2 | 10 | 17 | 121 | 124 | 13 | 3 | 2 | 18 | 190 | 176 | 9 |
| 0 | 18 | 15 | 269 | 277 | 22 | -1 | 8 | 16 | 209 | 226 | 10 | 1 | 0 | 17 | 101 | 96 | 14 | -1 | 10 | 17 | 178 | 177 | 9 | 4 | 2 | 18 | 146 | 135 | 14 |
| 1 | 18 | 15 | 123 | 112 | 14 | 0 | 8 | 16 | 205 | 204 | 10 | 2 | 0 | 17 | 274 | 268 | 8 | 0 | 10 | 17 | 147 | 205 | 17 | -4 | 3 | 18 | 97 | 95 | 21 |
| 2 | 18 | 15 | 79 | 94 | 27 | 1 | 8 | 16 | 225 | 227 | 10 | 3 | 0 | 17 | 78 | 74 | 16 | 1 | 10 | 17 | 180 | 177 | 8 | -3 | 3 | 18 | 249 | 218 | 16 |
| -2 | 19 | 15 | 57 | 38 | 27 | 2 | 8 | 16 | 161 | 152 | 10 | 4 | 0 | 17 | 68 | 12 | 38 | 2 | 10 | 17 | 103 | 124 | 17 | -2 | 3 | 18 | 239 | 247 | 8 |
| -1 | 19 | 15 | 47 | 59 | 47 | 3 | 8 | 16 | 208 | 232 | 10 | -4 | 1 | 17 | 136 | 156 | 26 | 3 | 10 | 17 | 72 | 88 | 24 | -1 | 3 | 18 | 207 | 227 | 12 |
| 0 | 19 | 15 | 68 | 44 | 26 | 4 | 8 | 16 | 61 | 64 | 24 | -3 | 1 | 17 | 58 | 59 | 27 | -3 | 11 | 17 | 173 | 179 | 18 | 0 | 3 | 18 | 216 | 238 | 10 |
| 1 | 19 | 15 | 73 | 58 | 24 | -4 | 9 | 16 | 59 | 69 | 58 | -2 | 1 | 17 | 151 | 145 | 7 | -2 | 11 | 17 | 88 | 28 | 19 | 1 | 3 | 18 | 202 | 226 | 12 |
| 2 | 19 | 15 | 0 | 37 | 1 | -3 | 9 | 16 | 97 | 91 | 12 | -1 | 1 | 17 | 193 | 190 | 13 | -1 | 11 | 17 | 145 | 132 | 9 | 2 | 3 | 18 | 229 | 247 | 7 |
| -2 | 20 | 15 | 161 | 108 | 35 | -2 | 9 | 16 | 129 | 134 | 13 | 0 | 1 | 17 | 220 | 259 | 8 | 0 | 11 | 17 | 56 | 72 | 56 | 3 | 3 | 18 | 233 | 218 | 10 |
| -1 | 20 | 15 | 92 | 104 | 16 | -1 | 9 | 16 | 143 | 118 | 8 | 1 | 1 | 17 | 193 | 189 | 14 | 1 | 11 | 17 | 139 | 132 | 10 | 4 | 3 | 18 | 100 | 95 | 11 |
| 0 | 20 | 15 | 0 | 15 | 1 | 0 | 9 | 16 | 94 | 73 | 14 | 2 | 1 | 17 | 150 | 145 | 6 | 2 | 11 | 17 | 25 | 28 | 25 | -4 | 4 | 18 | 51 | 80 | 50 |
| 1 | 20 | 15 | 82 | 104 | 37 | 1 | 9 | 16 | 127 | 119 | 10 | 3 | 1 | 17 | 0 | 59 | 1 | 3 | 11 | 17 | 133 | 180 | 13 | -3 | 4 | 18 | 174 | 145 | 12 |
| -1 | 21 | 15 | 27 | 7 | 27 | 2 | 9 | 16 | 141 | 133 | 11 | 4 | 1 | 17 | 154 | 155 | 11 | -3 | 12 | 17 | 73 | 56 | 28 | -2 | 4 | 18 | 252 | 239 | 9 |
| 0 | 21 | 15 | 8 | 21 | 8 | 3 | 9 | 16 | 71 | 92 | 23 | -4 | 2 | 17 | 223 | 243 | 22 | -2 | 12 | 17 | 115 | 93 | 14 | -1 | 4 | 18 | 283 | 293 | 14 |
| 1 | 21 | 15 | 34 | 6 | 34 | 4 | 9 | 16 | 70 | 69 | 26 | -3 | 2 | 17 | 280 | 273 | 18 | -1 | 12 | 17 | 132 | 114 | 11 | 0 | 4 | 18 | 172 | 167 | 9 |
| 0 | 22 | 15 | 31 | 24 | 31 | -4 | 10 | 16 | 98 | 100 | 25 | -2 | 2 | 17 | 120 | 105 | 9 | 0 | 12 | 17 | 35 | 30 | 34 | 1 | 4 | 18 | 278 | 292 | 15 |
| 0 | 0 | 16 | 163 | 155 | 11 | -3 | 10 | 16 | 0 | 58 | 1 | -1 | 2 | 17 | 127 | 145 | 11 | 1 | 12 | 17 | 111 | 113 | 14 | 2 | 4 | 18 | 234 | 239 | 9 |
| 1 | 0 | 16 | 259 | 267 | 11 | -2 | 10 | 16 | 160 | 165 | 11 | 0 | 2 | 17 | 315 | 318 | 12 | 2 | 12 | 17 | 97 | 94 | 19 | 3 | 4 | 18 | 172 | 146 | 19 |
| 2 | 0 | 16 | 37 | 6 | 37 | -1 | 10 | 16 | 98 | 56 | 13 | 1 | 2 | 17 | 118 | 145 | 39 | 3 | 12 | 17 | 81 | 57 | 18 | 4 | 4 | 18 | 62 | 81 | 61 |
| 3 | 0 | 16 | 32 | 57 | 31 | 0 | 10 | 16 | 291 | 295 | 12 | 2 | 2 | 17 | 104 | 106 | 8 | -3 | 13 | 17 | 0 | 48 | 1 | -4 | 5 | 18 | 53 | 54 | 52 |
| 4 | 0 | 16 | 157 | 198 | 31 | 1 | 10 | 16 | 72 | 57 | 17 | 3 | 2 | 17 | 296 | 274 | 11 | -2 | 13 | 17 | 186 | 184 | 12 | -3 | 5 | 18 | 144 | 153 | 12 |
| -4 | 1 | 16 | 62 | 84 | 54 | 2 | 10 | 16 | 160 | 164 | 18 | 4 | 2 | 17 | 262 | 243 | 19 | -1 | 13 | 17 | 419 | 404 | 13 | -2 | 5 | 18 | 96 | 93 | 20 |
| -3 | 1 | 16 | 71 | 63 | 19 | 3 | 10 | 16 | 84 | 59 | 19 | -4 | 3 | 17 | 110 | 121 | 20 | 0 | 13 | 17 | 99 | 54 | 24 | -1 | 5 | 18 | 182 | 197 | 11 |
| -2 | 1 | 16 | 308 | 287 | 9 | 4 | 10 | 16 | 94 | 101 | 22 | -3 | 3 | 17 | 213 | 183 | 31 | 1 | 13 | 17 | 433 | 403 | 17 | 0 | 5 | 18 | 271 | 283 | 17 |
| -1 | 1 | 16 | 140 | 136 | 10 | -3 | 11 | 16 | 84 | 99 | 14 | -2 | 3 | 17 | 219 | 194 | 7 | 2 | 13 | 17 | 164 | 184 | 12 | 1 | 5 | 18 | 199 | 197 | 14 |
| 0 | 1 | 16 | 39 | 28 | 38 | -2 | 11 | 16 | 0 | 9 | 1 | -1 | 3 | 17 | 311 | 316 | 12 | 3 | 13 | 17 | 32 | 48 | 32 | 2 | 5 | 18 | 92 | 93 | 15 |
| 1 | 1 | 16 | 135 | 137 | 12 | -1 | 11 | 16 | 60 | 39 | 26 | 0 | 3 | 17 | 155 | 141 | 9 | -3 | 14 | 17 | 71 | 59 | 27 | 3 | 5 | 18 | 143 | 152 | 11 |
| 2 | 1 | 16 | 318 | 286 | 13 | 0 | 11 | 16 | 214 | 205 | 10 | 1 | 3 | 17 | 309 | 315 | 15 | -2 | 14 | 17 | 99 | 80 | 13 | 4 | 5 | 18 | 0 | 54 | 1 |
| 3 | 1 | 16 | 74 | 62 | 19 | 1 | 11 | 16 | 56 | 39 | 26 | 2 | 3 | 17 | 195 | 195 | 7 | -1 | 14 | 17 | 273 | 267 | 10 | -4 | 6 | 18 | 35 | 72 | 34 |
| 4 | 1 | 16 | 100 | 84 | 15 | 2 | 11 | 16 | 48 | 9 | 47 | 3 | 3 | 17 | 198 | 183 | 11 | 0 | 14 | 17 | 280 | 243 | 24 | -3 | 6 | 18 | 238 | 264 | 12 |
| -4 | 2 | 16 | 67 | 51 | 40 | 3 | 11 | 16 | 83 | 98 | 17 | 4 | 3 | 17 | 139 | 121 | 22 | 1 | 14 | 17 | 281 | 268 | 12 | -2 | 6 | 18 | 121 | 130 | 23 |
| -3 | 2 | 16 | 116 | 124 | 15 | -3 | 12 | 16 | 62 | 68 | 20 | -4 | 4 | 17 | 91 | 122 | 30 | 2 | 14 | 17 | 47 | 81 | 46 | -1 | 6 | 18 | 133 | 123 | 12 |
| -2 | 2 | 16 | 291 | 314 | 8 | -2 | 12 | 16 | 151 | 84 | 28 | -3 | 4 | 17 | 0 | 67 | 1 | 3 | 14 | 17 | 70 | 60 | 23 | 0 | 6 | 18 | 52 | 53 | 51 |
| -1 | 2 | 16 | 406 | 410 | 19 | -1 | 12 | 16 | 226 | 213 | 8 | -2 | 4 | 17 | 26 | 46 | 26 | -3 | 15 | 17 | 62 | 51 | 62 | 1 | 6 | 18 | 123 | 122 | 9 |
| 2 | 6 | 18 | 124 | 129 | 11 | 1 | 19 | 18 | 56 | 60 | 39 | -3 | 10 | 19 | 54 | 40 | 29 | -3 | 3 | 20 | 182 | 204 | 12 | -2 | 14 | 20 | 71 | 73 | 21 |
| 3 | 6 | 18 | 269 | 264 | 15 | -1 | 20 | 18 | 65 | 64 | 22 | -2 | 10 | 19 | 130 | 123 | 16 | -2 | 3 | 20 | 44 | 49 | 44 | -1 | 14 | 20 | 136 | 127 | 12 |
| 4 | 6 | 18 | 69 | 71 | 31 | 0 | 20 | 18 | 72 | 40 | 24 | -1 | 10 | 19 | 111 | 111 | 13 | -1 | 3 | 20 | 55 | 56 | 47 | 0 | 14 | 20 | 289 | 258 | 14 |
| -4 | 7 | 18 | 71 | 76 | 37 | 1 | 20 | 18 | 48 | 64 | 48 | 0 | 10 | 19 | 195 | 184 | 10 | 0 | 3 | 20 | 595 | 592 | 37 | 1 | 14 | 20 | 141 | 127 | 17 |
| -3 | 7 | 18 | 230 | 231 | 12 | 0 | 21 | 18 | 63 | 54 | 28 | 1 | 10 | 19 | 136 | 112 | 9 | 1 | 3 | 20 | 77 | 57 | 14 | 2 | 14 | 20 | 0 | 73 | 1 |
| -2 | 7 | 18 | 110 | 106 | 15 | 1 | 0 | 19 | 105 | 70 | 17 | 2 | 10 | 19 | 203 | 123 | 32 | 2 | 3 | 20 | 45 | 48 | 25 | 3 | 14 | 20 | 64 | 62 | 27 |
| -1 | 7 | 18 | 62 | 53 | 43 | 2 | 0 | 19 | 164 | 176 | 7 | 3 | 10 | 19 | 31 | 40 | 30 | 3 | 3 | 20 | 200 | 204 | 9 | -2 | 15 | 20 | 118 | 124 | 13 |
| 0 | 7 | 18 | 110 | 69 | 17 | 3 | 0 | 19 | 66 | 19 | 30 | -3 | 11 | 19 | 91 | 105 | 14 | 4 | 3 | 20 | 86 | 91 | 13 | -1 | 15 | 20 | 124 | 122 | 18 |
| 1 | 7 | 18 | 62 | 54 | 37 | 4 | 0 | 19 | 64 | 37 | 37 | -2 | 11 | 19 | 157 | 157 | 11 | -4 | 4 | 20 | 109 | 113 | 19 | 0 | 15 | 20 | 469 | 461 | 19 |
| 2 | 7 | 18 | 103 | 106 | 14 | -4 | 1 | 19 | 51 | 79 | 51 | -1 | 11 | 19 | 212 | 230 | 8 | -3 | 4 | 20 | 170 | 178 | 13 | 1 | 15 | 20 | 99 | 122 | 18 |
| 3 | 7 | 18 | 243 | 232 | 10 | -3 | 1 | 19 | 266 | 268 | 17 | 0 | 11 | 19 | 116 | 91 | 19 | -2 | 4 | 20 | 116 | 114 | 13 | 2 | 15 | 20 | 104 | 124 | 20 |
| 4 | 7 | 18 | 83 | 76 | 14 | -2 | 1 | 19 | 80 | 71 | 22 | 1 | 11 | 19 | 240 | 230 | 11 | -1 | 4 | 20 | 281 | 261 | 18 | -2 | 16 | 20 | 99 | 78 | 23 |
| -4 | 8 | 18 | 110 | 115 | 20 | -1 | 1 | 19 | 42 | 71 | 42 | 2 | 11 | 19 | 138 | 158 | 20 | 0 | 4 | 20 | 80 | 33 | 40 | -1 | 16 | 20 | 155 | 170 | 10 |
| -3 | 8 | 18 | 172 | 156 | 25 | 0 | 1 | 19 | 205 | 231 | 12 | 3 | 11 | 19 | 101 | 106 | 14 | 1 | 4 | 20 | 276 | 261 | 10 | 0 | 16 | 20 | 211 | 253 | 13 |
| -2 | 8 | 18 | 270 | 227 | 10 | 1 | 1 | 19 | 95 | 71 | 18 | -3 | 12 | 19 | 75 | 79 | 16 | 2 | 4 | 20 | 119 | 114 | 13 | 1 | 16 | 20 | 157 | 170 | 15 |
| -1 | 8 | 18 | 174 | 150 | 10 | 2 | 1 | 19 | 91 | 71 | 10 | -2 | 12 | 19 | 202 | 183 | 9 | 3 | 4 | 20 | 208 | 179 | 16 | 2 | 16 | 20 | 74 | 77 | 73 |
| 0 | 8 | 18 | 351 | 339 | 12 | 3 | 1 | 19 | 267 | 269 | 11 | -1 | 12 | 19 | 314 | 301 | 10 | 4 | 4 | 20 | 89 | 113 | 16 | -2 | 17 | 20 | 62 | 55 | 25 |
| 1 | 8 | 18 | 181 | 151 | 8 | 4 | 1 | 19 | 89 | 79 | 17 | 0 | 12 | 19 | 209 | 180 | 13 | -4 | 5 | 20 | 113 | 96 | 19 | -1 | 17 | 20 | 45 | 64 | 44 |
| 2 | 8 | 18 | 257 | 227 | 17 | -4 | 2 | 19 | 69 | 30 | 33 | 1 | 12 | 19 | 312 | 301 | 12 | -3 | 5 | 20 | 133 | 138 | 13 | 0 | 17 | 20 | 59 | 87 | 59 |
| 3 | 8 | 18 | 139 | 156 | 14 | -3 | 2 | 19 | 298 | 256 | 22 | 2 | 12 | 19 | 148 | 183 | 13 | -2 | 5 | 20 | 259 | 233 | 13 | 1 | 17 | 20 | 61 | 64 | 32 |
| 4 | 8 | 18 | 117 | 116 | 25 | -2 | 2 | 19 | 105 | 103 | 11 | 3 | 12 | 19 | 76 | 79 | 19 | -1 | 5 | 20 | 141 | 130 | 12 | 2 | 17 | 20 | 81 | 55 | 80 |
| -3 | 9 | 18 | 66 | 60 | 20 | -1 | 2 | 19 | 86 | 94 | 16 | -3 | 13 | 19 | 154 | 148 | 17 | 0 | 5 | 20 | 174 | 154 | 13 | -2 | 18 | 20 | 59 | 46 | 31 |
| -2 | 9 | 18 | 87 | 65 | 23 | 0 | 2 | 19 | 504 | 514 | 22 | -2 | 13 | 19 | 126 | 136 | 20 | 1 | 5 | 20 | 118 | 130 | 10 | -1 | 18 | 20 | 17 | 67 | 16 |
| -1 | 9 | 18 | 210 | 178 | 8 | 1 | 2 | 19 | 84 | 94 | 22 | -1 | 13 | 19 | 89 | 88 | 18 | 2 | 5 | 20 | 251 | 233 | 10 | 0 | 18 | 20 | 32 | 9 | 31 |
| 0 | 9 | 18 | 0 | 38 | 1 | 2 | 2 | 19 | 113 | 103 | 11 | 0 | 13 | 19 | 0 | 56 | 1 | 3 | 5 | 20 | 141 | 138 | 9 | 1 | 18 | 20 | 0 | 67 | 1 |
| 1 | 9 | 18 | 195 | 179 | 8 | 3 | 2 | 19 | 274 | 256 | 11 | 1 | 13 | 19 | 107 | 88 | 17 | 4 | 5 | 20 | 86 | 96 | 14 | -1 | 19 | 20 | 108 | 80 | 12 |
| 2 | 9 | 18 | 52 | 64 | 52 | 4 | 2 | 19 | 36 | 32 | 36 | 2 | 13 | 19 | 91 | 136 | 17 | -4 | 6 | 20 | 58 | 86 | 57 | 0 | 19 | 20 | 23 | 13 | 22 |
| 3 | 9 | 18 | 71 | 59 | 20 | -4 | 3 | 29 | 96 | 115 | 20 | 3 | 13 | 19 | 120 | 148 | 13 | -3 | 6 | 20 | 79 | 90 | 19 | 1 | 19 | 20 | 61 | 80 | 60 |
| -3 | 10 | 18 | 184 | 180 | 8 | -3 | 3 | 19 | 270 | 277 | 14 | -3 | 14 | 19 | 94 | 96 | 24 | -2 | 6 | 20 | 105 | 95 | 15 | 0 | 20 | 20 | 42 | 75 | 42 |
| -2 | 10 | 18 | 147 | 140 | 12 | -2 | 3 | 19 | 70 | 76 | 17 | -2 | 14 | 19 | 144 | 133 | 19 | -1 | 6 | 20 | 94 | 77 | 13 | 1 | 0 | 21 | 0 | 49 | 1 |
| -1 | 10 | 18 | 96 | 83 | 14 | -1 | 3 | 19 | 191 | 205 | 10 | -1 | 14 | 19 | 202 | 216 | 9 | 0 | 6 | 20 | 123 | 123 | 12 | 2 | 0 | 21 | 44 | 18 | 43 |
| 0 | 10 | 18 | 307 | 295 | 12 | 0 | 3 | 19 | 248 | 244 | 20 | 0 | 14 | 19 | 499 | 461 | 20 | 1 | 6 | 20 | 124 | 76 | 15 | 3 | 0 | 21 | 75 | 75 | 21 |
| 1 | 10 | 18 | 83 | 84 | 23 | 1 | 3 | 19 | 190 | 205 | 18 | 1 | 14 | 19 | 192 | 215 | 12 | 2 | 6 | 20 | 109 | 95 | 13 | 4 | 0 | 21 | 0 | 37 | 1 |
| 2 | 10 | 18 | 143 | 141 | 13 | 2 | 3 | 19 | 69 | 77 | 12 | 2 | 14 | 19 | 120 | 134 | 15 | 3 | 6 | 20 | 68 | 91 | 50 | -4 | 1 | 21 | 52 | 46 | 52 |
| 3 | 10 | 18 | 142 | 181 | 11 | 3 | 3 | 19 | 297 | 278 | 11 | 3 | 14 | 19 | 56 | 96 | 35 | 4 | 6 | 20 | 92 | 86 | 18 | -3 | 1 | 21 | 95 | 96 | 40 |
| -3 | 11 | 18 | 97 | 57 | 12 | 4 | 3 | 19 | 121 | 116 | 10 | -3 | 15 | 19 | 98 | 91 | 67 | -4 | 7 | 20 | 153 | 117 | 27 | -2 | 1 | 21 | 185 | 180 | 8 |
| -2 | 11 | 18 | 66 | 63 | 28 | -4 | 4 | 19 | 70 | 40 | 30 | -2 | 15 | 19 | 184 | 175 | 17 | -3 | 7 | 20 | 97 | 84 | 15 | -1 | 1 | 21 | 96 | 67 | 29 |
| -1 | 11 | 18 | 143 | 136 | 10 | -3 | 4 | 19 | 193 | 183 | 12 | -1 | 15 | 19 | 39 | 12 | 38 | -2 | 7 | 20 | 38 | 57 | 38 | 0 | 1 | 21 | 315 | 327 | 11 |
| 0 | 11 | 18 | 51 | 7 | 50 | -2 | 4 | 19 | 165 | 156 | 10 | 0 | 15 | 19 | 356 | 321 | 15 | -1 | 7 | 20 | 116 | 108 | 11 | 1 | 1 | 21 | 80 | 66 | 23 |
| 1 | 11 | 18 | 131 | 135 | 10 | -1 | 4 | 19 | 210 | 209 | 28 | 1 | 15 | 19 | 66 | 12 | 30 | 0 | 7 | 20 | 216 | 197 | 13 | 2 | 1 | 21 | 178 | 181 | 7 |
| 2 | 11 | 18 | 22 | 63 | 21 | 0 | 4 | 19 | 166 | 144 | 12 | 2 | 15 | 19 | 173 | 175 | 48 | 1 | 7 | 20 | 105 | 109 | 12 | 3 | 1 | 21 | 111 | 97 | 14 |
| 3 | 11 | 18 | 66 | 57 | 36 | 1 | 4 | 19 | 232 | 209 | 9 | 3 | 15 | 19 | 73 | 90 | 26 | 2 | 7 | 20 | 17 | 57 | 16 | 4 | 1 | 21 | 88 | 46 | 22 |
| -3 | 12 | 18 | 82 | 63 | 14 | 2 | 4 | 19 | 164 | 156 | 6 | -2 | 16 | 19 | 81 | 93 | 24 | 3 | 7 | 20 | 73 | 84 | 20 | -4 | 2 | 21 | 106 | 133 | 23 |
| -2 | 12 | 18 | 179 | 177 | 13 | 3 | 4 | 19 | 207 | 183 | 9 | -1 | 16 | 19 | 62 | 33 | 34 | 4 | 7 | 20 | 103 | 117 | 17 | -3 | 2 | 21 | 210 | 218 | 12 |
| -1 | 12 | 18 | 126 | 126 | 11 | 4 | 4 | 19 | 0 | 40 | 1 | 0 | 16 | 19 | 300 | 288 | 28 | -3 | 8 | 20 | 85 | 93 | 15 | -2 | 2 | 21 | 11 | 31 | 11 |
| 0 | 12 | 18 | 101 | 75 | 18 | -4 | 5 | 19 | 43 | 52 | 43 | 1 | 16 | 19 | 54 | 33 | 53 | -2 | 8 | 20 | 165 | 139 | 16 | -1 | 2 | 21 | 40 | 89 | 40 |
| 1 | 12 | 18 | 139 | 127 | 16 | -3 | 5 | 19 | 73 | 70 | 23 | 2 | 16 | 19 | 87 | 94 | 75 | -1 | 8 | 20 | 207 | 216 | 8 | 0 | 2 | 21 | 92 | 74 | 14 |
| 2 | 12 | 18 | 161 | 177 | 12 | -2 | 5 | 19 | 258 | 253 | 8 | -2 | 17 | 19 | 52 | 69 | 51 | 0 | 8 | 20 | 86 | 83 | 16 | 1 | 2 | 21 | 90 | 88 | 13 |
| 3 | 12 | 18 | 42 | 63 | 41 | -1 | 5 | 19 | 142 | 123 | 10 | -1 | 17 | 19 | 0 | 6 | 1 | 1 | 8 | 20 | 212 | 215 | 11 | 2 | 2 | 21 | 53 | 31 | 21 |
| -3 | 13 | 18 | 62 | 69 | 41 | 0 | 5 | 19 | 0 | 59 | 1 | 0 | 17 | 19 | 105 | 94 | 18 | 2 | 8 | 20 | 140 | 139 | 12 | 3 | 2 | 21 | 206 | 218 | 24 |
| -2 | 13 | 18 | 198 | 198 | 11 | 1 | 5 | 19 | 129 | 123 | 9 | 1 | 17 | 19 | 0 | 6 | 1 | 3 | 8 | 20 | 67 | 93 | 20 | 4 | 2 | 21 | 122 | 133 | 10 |
| -1 | 13 | 18 | 282 | 267 | 10 | 2 | 5 | 19 | 256 | 253 | 9 | 2 | 17 | 19 | 82 | 69 | 34 | -3 | 9 | 20 | 118 | 109 | 10 | -4 | 3 | 21 | 97 | 99 | 22 |
| 0 | 13 | 18 | 105 | 104 | 21 | 3 | 5 | 19 | 48 | 70 | 47 | -2 | 18 | 19 | 0 | 26 | 1 | -2 | 9 | 20 | 178 | 168 | 10 | -3 | 3 | 21 | 104 | 107 | 29 |
| 1 | 13 | 18 | 289 | 267 | 13 | 4 | 5 | 19 | 73 | 52 | 27 | -1 | 18 | 19 | 0 | 5 | 1 | -1 | 9 | 20 | 274 | 263 | 9 | -2 | 3 | 21 | 137 | 126 | 11 |
| 2 | 13 | 18 | 152 | 198 | 13 | -4 | 6 | 19 | 70 | 67 | 38 | 0 | 18 | 19 | 173 | 164 | 11 | 0 | 9 | 20 | 35 | 25 | 34 | -1 | 3 | 21 | 71 | 63 | 19 |
| 3 | 13 | 18 | 70 | 70 | 22 | -3 | 6 | 19 | 101 | 131 | 16 | 1 | 18 | 19 | 41 | 5 | 41 | 1 | 9 | 20 | 270 | 264 | 9 | 0 | 3 | 21 | 177 | 170 | 15 |
| -3 | 14 | 18 | 66 | 62 | 43 | -2 | 6 | 19 | 135 | 140 | 10 | 2 | 18 | 19 | 53 | 26 | 53 | 2 | 9 | 20 | 145 | 169 | 13 | 1 | 3 | 21 | 91 | 64 | 22 |
| -2 | 14 | 18 | 76 | 81 | 29 | -1 | 6 | 19 | 271 | 277 | 13 | -1 | 19 | 19 | 57 | 71 | 35 | 3 | 9 | 20 | 68 | 108 | 20 | 2 | 3 | 21 | 131 | 125 | 7 |
| -1 | 14 | 18 | 179 | 162 | 10 | 0 | 6 | 19 | 488 | 460 | 23 | 0 | 19 | 19 | 71 | 86 | 24 | -3 | 10 | 20 | 101 | 85 | 22 | 3 | 3 | 21 | 91 | 107 | 16 |
| 0 | 14 | 18 | 250 | 224 | 13 | 1 | 6 | 19 | 277 | 277 | 9 | 1 | 19 | 19 | 60 | 71 | 59 | -2 | 10 | 20 | 95 | 71 | 17 | 4 | 3 | 21 | 109 | 99 | 10 |
| 1 | 14 | 18 | 177 | 162 | 14 | 2 | 6 | 19 | 127 | 140 | 12 | -1 | 20 | 19 | 97 | 107 | 13 | -1 | 10 | 20 | 163 | 141 | 10 | -4 | 4 | 21 | 0 | 54 | 1 |
| 2 | 14 | 18 | 68 | 81 | 26 | 3 | 6 | 19 | 131 | 131 | 11 | 0 | 20 | 19 | 88 | 82 | 22 | 0 | 10 | 20 | 144 | 145 | 23 | -3 | 4 | 21 | 34 | 49 | 33 |
| 3 | 14 | 18 | 64 | 62 | 26 | 4 | 6 | 19 | 59 | 67 | 32 | 1 | 20 | 19 | 90 | 106 | 39 | 1 | 10 | 20 | 161 | 141 | 10 | -2 | 4 | 21 | 165 | 152 | 11 |
| -3 | 15 | 18 | 66 | 104 | 47 | -4 | 7 | 19 | 184 | 177 | 19 | 0 | 0 | 20 | 468 | 470 | 30 | 2 | 10 | 20 | 70 | 71 | 28 | -1 | 4 | 21 | 115 | 97 | 15 |
| -2 | 15 | 18 | 240 | 264 | 19 | -3 | 7 | 19 | 101 | 102 | 15 | 1 | 0 | 20 | 136 | 119 | 18 | 3 | 10 | 20 | 94 | 85 | 19 | 0 | 4 | 21 | 71 | 61 | 21 |
| -1 | 15 | 18 | 116 | 108 | 17 | -2 | 7 | 19 | 242 | 228 | 10 | 2 | 0 | 20 | 230 | 227 | 8 | -3 | 11 | 20 | 83 | 67 | 15 | 1 | 4 | 21 | 116 | 97 | 11 |
| 0 | 15 | 18 | 156 | 131 | 15 | -1 | 7 | 19 | 181 | 169 | 7 | 3 | 0 | 20 | 321 | 299 | 14 | -2 | 11 | 20 | 70 | 64 | 23 | 2 | 4 | 21 | 163 | 152 | 7 |
| 1 | 15 | 18 | 131 | 107 | 16 | 0 | 7 | 19 | 102 | 93 | 13 | 4 | 0 | 20 | 0 | 38 | 1 | -1 | 11 | 20 | 105 | 113 | 17 | 3 | 4 | 21 | 68 | 49 | 21 |
| 2 | 15 | 18 | 254 | 263 | 23 | 1 | 7 | 19 | 161 | 168 | 10 | -4 | 1 | 20 | 87 | 70 | 26 | 0 | 11 | 20 | 234 | 234 | 14 | 4 | 4 | 21 | 61 | 54 | 20 |
| 3 | 15 | 18 | 90 | 104 | 17 | 2 | 7 | 19 | 229 | 228 | 10 | -3 | 1 | 20 | 160 | 138 | 17 | 1 | 11 | 20 | 129 | 114 | 13 | -4 | 5 | 21 | 39 | 40 | 39 |
| -2 | 16 | 18 | 108 | 96 | 16 | 3 | 7 | 19 | 100 | 101 | 16 | -2 | 1 | 20 | 76 | 74 | 15 | 2 | 11 | 20 | 68 | 63 | 26 | -3 | 5 | 21 | 104 | 122 | 14 |
| -1 | 16 | 18 | 118 | 115 | 12 | 4 | 7 | 19 | 181 | 177 | 11 | -1 | 1 | 20 | 108 | 101 | 14 | 3 | 11 | 20 | 61 | 67 | 25 | -2 | 5 | 21 | 86 | 98 | 19 |
| 0 | 16 | 18 | 168 | 183 | 14 | -4 | 8 | 19 | 138 | 153 | 17 | 0 | 1 | 20 | 310 | 319 | 15 | -3 | 12 | 20 | 99 | 119 | 13 | -1 | 5 | 21 | 347 | 332 | 10 |
| 1 | 16 | 18 | 90 | 116 | 27 | -3 | 8 | 19 | 61 | 46 | 21 | 1 | 1 | 20 | 130 | 102 | 11 | -2 | 12 | 20 | 183 | 201 | 14 | 0 | 5 | 21 | 94 | 55 | 26 |
| 2 | 16 | 18 | 84 | 96 | 24 | -2 | 8 | 19 | 93 | 89 | 17 | 2 | 1 | 20 | 101 | 73 | 15 | -1 | 12 | 20 | 23 | 29 | 23 | 1 | 5 | 21 | 346 | 332 | 12 |
| -2 | 17 | 18 | 88 | 82 | 14 | -1 | 8 | 19 | 109 | 73 | 12 | 3 | 1 | 20 | 173 | 139 | 11 | 0 | 12 | 20 | 229 | 207 | 13 | 2 | 5 | 21 | 96 | 98 | 10 |
| -1 | 17 | 18 | 135 | 128 | 11 | 0 | 8 | 19 | 218 | 211 | 14 | 4 | 1 | 20 | 62 | 70 | 41 | 1 | 12 | 20 | 27 | 29 | 26 | 3 | 5 | 21 | 99 | 123 | 11 |
| 0 | 17 | 18 | 259 | 279 | 29 | 1 | 8 | 19 | 101 | 73 | 14 | -4 | 2 | 20 | 57 | 35 | 56 | 2 | 12 | 20 | 175 | 201 | 16 | 4 | 5 | 21 | 22 | 40 | 22 |
| 1 | 17 | 18 | 121 | 127 | 17 | 2 | 8 | 19 | 73 | 89 | 22 | -3 | 2 | 20 | 185 | 181 | 24 | 3 | 12 | 20 | 94 | 119 | 14 | -3 | 6 | 21 | 92 | 108 | 16 |
| 2 | 17 | 18 | 109 | 82 | 27 | 3 | 8 | 19 | 51 | 46 | 34 | -2 | 2 | 20 | 144 | 148 | 9 | -3 | 13 | 20 | 179 | 177 | 12 | -2 | 6 | 21 | 164 | 136 | 11 |
| -2 | 18 | 18 | 67 | 36 | 21 | -3 | 9 | 19 | 96 | 89 | 13 | -1 | 2 | 20 | 132 | 114 | 16 | -2 | 13 | 20 | 0 | 45 | 1 | -1 | 6 | 21 | 123 | 110 | 11 |
| -1 | 18 | 18 | 104 | 86 | 13 | -2 | 9 | 19 | 97 | 63 | 17 | 0 | 2 | 20 | 503 | 539 | 32 | -1 | 13 | 20 | 266 | 252 | 9 | 0 | 6 | 21 | 62 | 60 | 22 |
| 0 | 18 | 18 | 43 | 37 | 43 | -1 | 9 | 19 | 137 | 118 | 10 | 1 | 2 | 20 | 132 | 113 | 10 | 0 | 13 | 20 | 61 | 71 | 60 | 1 | 6 | 21 | 122 | 110 | 24 |
| 1 | 18 | 18 | 95 | 86 | 31 | 0 | 9 | 19 | 0 | 24 | 1 | 2 | 2 | 20 | 150 | 148 | 7 | 1 | 13 | 20 | 246 | 252 | 13 | 2 | 6 | 21 | 149 | 136 | 11 |
| 2 | 18 | 18 | 58 | 36 | 57 | 1 | 9 | 19 | 130 | 118 | 10 | 3 | 2 | 20 | 185 | 181 | 9 | 2 | 13 | 20 | 44 | 45 | 43 | 3 | 6 | 21 | 77 | 107 | 17 |
| -1 | 19 | 18 | 44 | 60 | 43 | 2 | 9 | 19 | 80 | 63 | 20 | 4 | 2 | 20 | 0 | 34 | 1 | 3 | 13 | 20 | 136 | 178 | 12 | -3 | 7 | 21 | 0 | 41 | 1 |
| 0 | 19 | 18 | 23 | 38 | 23 | 3 | 9 | 19 | 83 | 90 | 16 | -4 | 3 | 20 | 96 | 91 | 25 | -3 | 14 | 20 | 79 | 63 | 29 | -2 | 7 | 21 | 216 | 212 | 10 |
| -1 | 7 | 21 | 175 | 156 | 8 | 4 | 1 | 22 | 64 | 68 | 38 | 1 | 13 | 22 | 222 | 224 | 12 | 3 | 8 | 23 | 37 | 53 | 37 | 3 | 4 | 24 | 44 | 70 | 44 |
| 0 | 7 | 21 | 106 | 97 | 12 | -4 | 2 | 22 | 85 | 77 | 33 | 2 | 13 | 22 | 153 | 163 | 14 | -3 | 9 | 23 | 161 | 147 | 11 | -3 | 5 | 24 | 63 | 90 | 28 |
| 1 | 7 | 21 | 162 | 157 | 9 | -3 | 2 | 22 | 35 | 76 | 34 | 3 | 13 | 22 | 86 | 73 | 36 | -2 | 9 | 23 | 84 | 98 | 21 | -2 | 5 | 24 | 99 | 88 | 14 |
| 2 | 7 | 21 | 187 | 212 | 11 | -2 | 2 | 22 | 149 | 134 | 10 | -2 | 14 | 22 | 77 | 78 | 17 | -1 | 9 | 23 | 233 | 221 | 9 | -1 | 5 | 24 | 86 | 54 | 20 |
| 3 | 7 | 21 | 0 | 41 | 1 | -1 | 2 | 22 | 18 | 49 | 17 | -1 | 14 | 22 | 111 | 124 | 13 | 0 | 9 | 23 | 254 | 229 | 15 | 0 | 5 | 24 | 150 | 118 | 11 |
| -3 | 8 | 21 | 95 | 117 | 16 | 0 | 2 | 22 | 49 | 3 | 49 | 0 | 14 | 22 | 333 | 317 | 15 | 1 | 9 | 23 | 232 | 221 | 12 | 1 | 5 | 24 | 75 | 54 | 24 |
| -2 | 8 | 21 | 0 | 27 | 1 | 1 | 2 | 22 | 70 | 48 | 20 | 1 | 14 | 22 | 133 | 123 | 12 | 2 | 9 | 23 | 117 | 98 | 19 | 2 | 5 | 24 | 102 | 88 | 17 |
| -1 | 8 | 21 | 64 | 28 | 25 | 2 | 2 | 22 | 136 | 134 | 9 | 2 | 14 | 22 | 105 | 78 | 20 | 3 | 9 | 23 | 118 | 146 | 12 | 3 | 5 | 24 | 58 | 90 | 25 |
| 0 | 8 | 21 | 100 | 96 | 16 | 3 | 2 | 22 | 78 | 76 | 21 | -2 | 15 | 22 | 307 | 318 | 22 | -3 | 10 | 23 | 95 | 84 | 16 | -3 | 6 | 24 | 47 | 18 | 46 |
| 1 | 8 | 21 | 0 | 28 | 1 | 4 | 2 | 22 | 73 | 77 | 33 | -1 | 15 | 22 | 86 | 19 | 16 | -2 | 10 | 23 | 106 | 77 | 24 | -2 | 6 | 24 | 134 | 112 | 11 |
| 2 | 8 | 21 | 0 | 27 | 1 | -4 | 3 | 22 | 83 | 96 | 25 | 0 | 15 | 22 | 114 | 129 | 16 | -1 | 10 | 23 | 37 | 12 | 37 | -1 | 6 | 24 | 112 | 110 | 18 |
| 3 | 8 | 21 | 92 | 117 | 13 | -3 | 3 | 22 | 88 | 112 | 21 | 1 | 15 | 22 | 0 | 19 | 1 | 0 | 10 | 23 | 98 | 101 | 55 | 0 | 6 | 24 | 131 | 146 | 12 |
| -3 | 9 | 21 | 150 | 159 | 13 | -2 | 3 | 22 | 89 | 85 | 21 | 2 | 15 | 22 | 345 | 318 | 17 | 1 | 10 | 23 | 74 | 12 | 49 | 1 | 6 | 24 | 122 | 110 | 17 |
| -2 | 9 | 21 | 241 | 213 | 10 | -1 | 3 | 22 | 124 | 99 | 11 | -2 | 16 | 22 | 34 | 29 | 34 | 2 | 10 | 23 | 82 | 77 | 20 | 2 | 6 | 24 | 138 | 112 | 13 |
| -1 | 9 | 21 | 174 | 127 | 9 | 0 | 3 | 22 | 162 | 174 | 9 | -1 | 16 | 22 | 115 | 110 | 12 | 3 | 10 | 23 | 81 | 84 | 22 | 3 | 6 | 24 | 0 | 18 | 1 |
| 0 | 9 | 21 | 155 | 152 | 11 | 1 | 3 | 22 | 105 | 98 | 13 | 0 | 16 | 22 | 125 | 114 | 16 | -3 | 11 | 23 | 108 | 114 | 34 | -3 | 7 | 24 | 67 | 69 | 24 |
| 1 | 9 | 21 | 151 | 126 | 11 | 2 | 3 | 22 | 97 | 85 | 18 | 1 | 16 | 22 | 146 | 110 | 17 | -2 | 11 | 23 | 29 | 17 | 29 | -2 | 7 | 24 | 91 | 81 | 15 |
| 2 | 9 | 21 | 195 | 212 | 13 | 3 | 3 | 22 | 92 | 112 | 14 | 2 | 16 | 22 | 59 | 29 | 59 | -1 | 11 | 23 | 206 | 176 | 9 | -1 | 7 | 24 | 130 | 111 | 15 |
| 3 | 9 | 21 | 133 | 159 | 11 | 4 | 3 | 22 | 100 | 96 | 16 | -2 | 17 | 22 | 100 | 109 | 23 | 0 | 11 | 23 | 108 | 83 | 40 | 0 | 7 | 24 | 154 | 165 | 10 |
| -3 | 10 | 21 | 92 | 81 | 13 | -4 | 4 | 22 | 61 | 37 | 61 | -1 | 17 | 22 | 118 | 143 | 11 | 1 | 11 | 23 | 202 | 176 | 12 | 1 | 7 | 24 | 104 | 111 | 17 |
| -2 | 10 | 21 | 110 | 77 | 14 | -3 | 4 | 22 | 59 | 59 | 28 | 0 | 17 | 22 | 35 | 33 | 35 | 2 | 11 | 23 | 0 | 17 | 1 | 2 | 7 | 24 | 70 | 80 | 26 |
| -1 | 10 | 21 | 210 | 190 | 9 | -2 | 4 | 22 | 114 | 102 | 14 | 1 | 17 | 22 | 177 | 143 | 14 | 3 | 11 | 23 | 114 | 114 | 18 | 3 | 7 | 24 | 76 | 69 | 16 |
| 0 | 10 | 21 | 46 | 60 | 46 | -1 | 4 | 22 | 75 | 70 | 26 | -1 | 18 | 22 | 35 | 54 | 35 | -3 | 12 | 23 | 78 | 78 | 15 | -3 | 8 | 24 | 51 | 52 | 51 |
| 1 | 10 | 21 | 224 | 190 | 12 | 0 | 4 | 22 | 85 | 60 | 17 | 0 | 18 | 22 | 0 | 63 | 1 | -2 | 12 | 23 | 137 | 139 | 11 | -2 | 8 | 24 | 142 | 145 | 12 |
| 2 | 10 | 21 | 55 | 77 | 55 | 1 | 4 | 22 | 68 | 70 | 20 | 1 | 18 | 22 | 60 | 54 | 59 | -1 | 12 | 23 | 345 | 336 | 11 | -1 | 8 | 24 | 170 | 152 | 11 |
| 3 | 10 | 21 | 49 | 81 | 49 | 2 | 4 | 22 | 86 | 102 | 13 | -1 | 19 | 22 | 0 | 9 | 1 | 0 | 12 | 23 | 126 | 122 | 20 | 0 | 8 | 24 | 85 | 72 | 21 |
| -3 | 11 | 21 | 44 | 18 | 43 | 3 | 4 | 22 | 0 | 60 | 1 | 0 | 19 | 22 | 24 | 13 | 23 | 1 | 12 | 23 | 362 | 336 | 24 | 1 | 8 | 24 | 174 | 152 | 29 |
| -2 | 11 | 21 | 91 | 100 | 19 | -3 | 5 | 22 | 98 | 86 | 15 | 1 | 0 | 23 | 84 | 17 | 17 | 2 | 12 | 23 | 151 | 139 | 12 | 2 | 8 | 24 | 150 | 145 | 14 |
| -1 | 11 | 21 | 62 | 47 | 30 | -2 | 5 | 22 | 50 | 41 | 49 | 2 | 0 | 23 | 175 | 163 | 9 | 3 | 12 | 23 | 63 | 78 | 30 | 3 | 8 | 24 | 59 | 51 | 25 |
| 0 | 11 | 21 | 424 | 384 | 21 | -1 | 5 | 22 | 297 | 271 | 15 | 3 | 0 | 23 | 73 | 22 | 43 | -2 | 13 | 23 | 95 | 107 | 15 | -3 | 9 | 24 | 40 | 19 | 40 |
| 1 | 11 | 21 | 0 | 47 | 1 | 0 | 5 | 22 | 31 | 38 | 30 | -4 | 1 | 23 | 78 | 72 | 77 | -1 | 13 | 23 | 304 | 282 | 9 | -2 | 9 | 24 | 80 | 105 | 80 |
| 2 | 11 | 21 | 99 | 100 | 15 | 1 | 5 | 22 | 288 | 271 | 11 | -3 | 1 | 23 | 99 | 101 | 43 | 0 | 13 | 23 | 223 | 229 | 15 | -1 | 9 | 24 | 102 | 86 | 15 |
| 3 | 11 | 21 | 41 | 19 | 41 | 2 | 5 | 22 | 55 | 40 | 21 | -2 | 1 | 23 | 114 | 112 | 12 | 1 | 13 | 23 | 283 | 282 | 35 | 0 | 9 | 24 | 126 | 97 | 21 |
| -3 | 12 | 21 | 43 | 26 | 42 | 3 | 5 | 22 | 67 | 86 | 20 | -1 | 1 | 23 | 103 | 92 | 24 | 2 | 13 | 23 | 119 | 107 | 18 | 1 | 9 | 24 | 128 | 85 | 22 |
| -2 | 12 | 21 | 43 | 51 | 43 | -3 | 6 | 22 | 108 | 109 | 16 | 0 | 1 | 23 | 66 | 102 | 54 | -2 | 14 | 23 | 103 | 105 | 14 | 2 | 9 | 24 | 108 | 105 | 20 |
| -1 | 12 | 21 | 69 | 67 | 35 | -2 | 6 | 22 | 51 | 41 | 50 | 1 | 1 | 23 | 117 | 93 | 12 | -1 | 14 | 23 | 131 | 143 | 21 | 3 | 9 | 24 | 0 | 18 | 1 |
| 0 | 12 | 21 | 124 | 120 | 17 | -1 | 6 | 22 | 22 | 49 | 21 | 2 | 1 | 23 | 119 | 112 | 11 | 0 | 14 | 23 | 74 | 78 | 30 | -3 | 10 | 24 | 87 | 103 | 29 |
| 1 | 12 | 21 | 96 | 67 | 27 | 0 | 6 | 22 | 230 | 213 | 8 | 3 | 1 | 23 | 100 | 101 | 14 | 1 | 14 | 23 | 128 | 142 | 19 | -2 | 10 | 24 | 105 | 122 | 14 |
| 2 | 12 | 21 | 43 | 50 | 43 | 1 | 6 | 22 | 80 | 49 | 33 | 4 | 1 | 23 | 124 | 71 | 28 | 2 | 14 | 23 | 125 | 105 | 18 | -1 | 10 | 24 | 197 | 168 | 12 |
| 3 | 12 | 21 | 26 | 26 | 25 | 2 | 6 | 22 | 0 | 41 | 1 | -3 | 2 | 23 | 60 | 51 | 30 | -2 | 15 | 23 | 63 | 76 | 24 | 0 | 10 | 24 | 205 | 179 | 18 |
| -3 | 13 | 21 | 45 | 60 | 45 | 3 | 6 | 22 | 112 | 109 | 11 | -2 | 2 | 23 | 232 | 221 | 15 | -1 | 15 | 23 | 97 | 115 | 21 | 1 | 10 | 24 | 192 | 167 | 12 |
| -2 | 13 | 21 | 123 | 116 | 20 | -3 | 7 | 22 | 0 | 20 | 1 | -1 | 2 | 23 | 225 | 229 | 19 | 0 | 15 | 23 | 121 | 130 | 16 | 2 | 10 | 24 | 100 | 122 | 45 |
| -1 | 13 | 21 | 178 | 164 | 10 | -2 | 7 | 22 | 119 | 135 | 14 | 0 | 2 | 23 | 110 | 94 | 10 | 1 | 15 | 23 | 114 | 115 | 22 | 3 | 10 | 24 | 102 | 103 | 22 |
| 0 | 13 | 21 | 268 | 241 | 13 | -1 | 7 | 22 | 275 | 265 | 9 | 1 | 2 | 23 | 240 | 229 | 9 | 2 | 15 | 23 | 77 | 76 | 41 | -3 | 11 | 24 | 13 | 7 | 12 |
| 1 | 13 | 21 | 178 | 164 | 17 | 0 | 7 | 22 | 225 | 236 | 8 | 2 | 2 | 23 | 213 | 221 | 9 | -2 | 16 | 23 | 99 | 94 | 21 | -2 | 11 | 24 | 112 | 111 | 13 |
| 2 | 13 | 21 | 108 | 116 | 15 | 1 | 7 | 22 | 263 | 266 | 10 | 3 | 2 | 23 | 7 | 52 | 7 | -1 | 16 | 23 | 40 | 30 | 40 | -1 | 11 | 24 | 112 | 117 | 14 |
| 3 | 13 | 21 | 73 | 61 | 21 | 2 | 7 | 22 | 131 | 135 | 13 | -3 | 3 | 23 | 96 | 95 | 16 | 0 | 16 | 23 | 122 | 142 | 16 | 0 | 11 | 24 | 212 | 204 | 15 |
| -2 | 14 | 21 | 90 | 78 | 15 | 3 | 7 | 22 | 21 | 20 | 20 | -2 | 3 | 23 | 59 | 76 | 34 | 1 | 16 | 23 | 76 | 30 | 37 | 1 | 11 | 24 | 125 | 118 | 25 |
| -1 | 14 | 21 | 78 | 75 | 20 | -3 | 8 | 22 | 99 | 103 | 15 | -1 | 3 | 23 | 61 | 66 | 29 | 2 | 16 | 23 | 119 | 94 | 17 | 2 | 11 | 24 | 80 | 111 | 33 |
| 0 | 14 | 21 | 41 | 87 | 40 | -2 | 8 | 22 | 47 | 40 | 46 | 0 | 3 | 23 | 294 | 260 | 13 | -1 | 17 | 23 | 79 | 107 | 17 | 3 | 11 | 24 | 67 | 7 | 23 |
| 1 | 14 | 21 | 65 | 75 | 29 | -1 | 8 | 22 | 171 | 139 | 9 | 1 | 3 | 23 | 92 | 65 | 17 | 0 | 17 | 23 | 57 | 42 | 56 | -2 | 12 | 24 | 63 | 58 | 26 |
| 2 | 14 | 21 | 98 | 78 | 21 | 0 | 8 | 22 | 163 | 159 | 20 | 2 | 3 | 23 | 91 | 77 | 13 | 1 | 17 | 23 | 93 | 107 | 24 | -1 | 12 | 24 | 126 | 81 | 23 |
| -2 | 15 | 21 | 93 | 67 | 15 | 1 | 8 | 22 | 150 | 138 | 13 | 3 | 3 | 23 | 108 | 94 | 12 | -1 | 18 | 23 | 0 | 9 | 1 | 0 | 12 | 24 | 54 | 74 | 53 |
| -1 | 15 | 21 | 71 | 71 | 22 | 2 | 8 | 22 | 68 | 40 | 28 | -3 | 4 | 23 | 52 | 16 | 45 | 0 | 18 | 23 | 30 | 54 | 29 | 1 | 12 | 24 | 109 | 80 | 15 |
| 0 | 15 | 21 | 106 | 118 | 20 | 3 | 8 | 22 | 77 | 104 | 16 | -2 | 4 | 23 | 173 | 156 | 10 | 1 | 18 | 23 | 0 | 9 | 1 | 2 | 12 | 24 | 60 | 58 | 59 |
| 1 | 15 | 21 | 73 | 71 | 40 | -3 | 9 | 22 | 110 | 104 | 23 | -1 | 9 | 23 | 99 | 83 | 34 | 0 | 19 | 23 | 0 | 75 | 1 | -2 | 13 | 24 | 124 | 130 | 12 |
| 2 | 15 | 21 | 89 | 67 | 88 | -2 | 9 | 22 | 200 | 176 | 10 | 0 | 4 | 23 | 122 | 124 | 9 | 0 | 0 | 24 | 115 | 82 | 27 | -1 | 13 | 24 | 275 | 287 | 9 |
| -2 | 16 | 21 | 76 | 71 | 37 | -1 | 9 | 22 | 99 | 103 | 17 | 1 | 4 | 23 | 104 | 83 | 13 | 1 | 0 | 24 | 315 | 301 | 13 | 0 | 13 | 24 | 70 | 57 | 46 |
| -1 | 16 | 21 | 65 | 84 | 24 | 0 | 9 | 22 | 112 | 95 | 17 | 2 | 4 | 23 | 149 | 156 | 12 | 2 | 0 | 24 | 128 | 110 | 10 | 1 | 13 | 24 | 266 | 287 | 34 |
| 0 | 16 | 21 | 22 | 46 | 21 | 1 | 9 | 22 | 130 | 102 | 13 | 3 | 4 | 23 | 0 | 16 | 1 | 3 | 0 | 24 | 32 | 69 | 31 | 2 | 13 | 24 | 141 | 130 | 15 |
| 1 | 16 | 21 | 94 | 84 | 19 | 2 | 9 | 22 | 163 | 176 | 13 | -3 | 5 | 23 | 61 | 66 | 29 | -3 | 1 | 24 | 68 | 70 | 23 | -2 | 14 | 24 | 48 | 16 | 47 |
| 2 | 16 | 21 | 83 | 70 | 27 | 3 | 9 | 22 | 93 | 103 | 15 | -2 | 5 | 23 | 164 | 165 | 9 | -2 | 1 | 24 | 237 | 215 | 11 | -1 | 14 | 24 | 152 | 138 | 17 |
| -2 | 17 | 21 | 90 | 78 | 16 | -3 | 10 | 22 | 218 | 222 | 13 | -1 | 5 | 23 | 219 | 183 | 16 | -1 | 1 | 24 | 171 | 84 | 58 | 0 | 14 | 24 | 42 | 46 | 42 |
| -1 | 17 | 21 | 95 | 112 | 14 | -2 | 10 | 22 | 205 | 206 | 11 | 0 | 5 | 23 | 64 | 76 | 20 | 0 | 1 | 24 | 31 | 37 | 30 | 1 | 14 | 24 | 137 | 138 | 20 |
| 0 | 17 | 21 | 76 | 38 | 27 | -1 | 10 | 22 | 58 | 34 | 36 | 1 | 5 | 23 | 205 | 182 | 9 | 1 | 1 | 24 | 114 | 84 | 13 | 2 | 14 | 24 | 78 | 16 | 30 |
| 1 | 17 | 21 | 129 | 112 | 18 | 0 | 10 | 22 | 63 | 25 | 62 | 2 | 5 | 23 | 156 | 164 | 12 | 2 | 1 | 24 | 230 | 216 | 11 | -2 | 15 | 24 | 105 | 91 | 18 |
| 2 | 17 | 21 | 113 | 78 | 22 | 1 | 10 | 22 | 57 | 35 | 56 | 3 | 5 | 23 | 60 | 66 | 24 | 3 | 1 | 24 | 38 | 70 | 37 | -1 | 15 | 24 | 167 | 203 | 11 |
| -1 | 18 | 21 | 73 | 61 | 19 | 2 | 10 | 22 | 166 | 206 | 12 | -3 | 6 | 23 | 82 | 69 | 19 | -3 | 2 | 24 | 82 | 70 | 19 | 0 | 15 | 24 | 218 | 194 | 14 |
| 0 | 18 | 21 | 53 | 20 | 52 | 3 | 10 | 22 | 168 | 222 | 15 | -2 | 6 | 23 | 122 | 111 | 12 | -2 | 2 | 24 | 271 | 286 | 10 | 1 | 15 | 24 | 222 | 203 | 14 |
| 1 | 18 | 21 | 46 | 61 | 45 | -3 | 11 | 22 | 186 | 172 | 14 | -1 | 6 | 23 | 134 | 123 | 11 | -1 | 2 | 24 | 119 | 115 | 28 | 2 | 15 | 24 | 131 | 91 | 16 |
| -1 | 19 | 21 | 25 | 17 | 24 | -2 | 11 | 22 | 181 | 191 | 12 | 0 | 6 | 23 | 318 | 291 | 9 | 0 | 2 | 24 | 49 | 4 | 32 | -2 | 16 | 24 | 81 | 27 | 80 |
| 0 | 19 | 21 | 135 | 143 | 18 | -1 | 11 | 22 | 235 | 228 | 10 | 1 | 6 | 23 | 133 | 123 | 11 | 1 | 2 | 24 | 125 | 116 | 14 | -1 | 16 | 24 | 61 | 75 | 44 |
| 1 | 19 | 21 | 50 | 17 | 49 | 0 | 11 | 22 | 367 | 323 | 19 | 2 | 6 | 23 | 105 | 111 | 17 | 2 | 2 | 24 | 271 | 287 | 14 | 0 | 16 | 24 | 0 | 18 | 1 |
| 0 | 20 | 21 | 109 | 48 | 28 | 1 | 11 | 22 | 253 | 228 | 11 | 3 | 6 | 23 | 48 | 70 | 47 | 3 | 2 | 24 | 73 | 70 | 18 | 1 | 16 | 24 | 74 | 75 | 34 |
| 0 | 0 | 22 | 390 | 413 | 19 | 2 | 11 | 22 | 170 | 190 | 15 | -3 | 7 | 23 | 45 | 16 | 45 | -3 | 3 | 24 | 0 | 47 | 1 | -1 | 17 | 24 | 104 | 98 | 13 |
| 1 | 0 | 22 | 41 | 23 | 40 | 3 | 11 | 22 | 146 | 172 | 12 | -2 | 7 | 23 | 118 | 122 | 12 | -2 | 3 | 24 | 80 | 37 | 21 | 0 | 17 | 24 | 36 | 57 | 35 |
| 2 | 0 | 22 | 230 | 213 | 10 | -3 | 12 | 22 | 38 | 64 | 37 | -1 | 7 | 23 | 162 | 146 | 9 | -1 | 3 | 24 | 344 | 300 | 20 | 1 | 17 | 24 | 87 | 99 | 24 |
| 3 | 0 | 22 | 210 | 222 | 23 | -2 | 12 | 22 | 80 | 66 | 18 | 0 | 7 | 23 | 63 | 48 | 26 | 0 | 3 | 24 | 89 | 73 | 13 | -1 | 18 | 24 | 54 | 56 | 53 |
| 4 | 0 | 22 | 72 | 84 | 71 | -1 | 12 | 22 | 81 | 94 | 20 | 1 | 7 | 23 | 161 | 145 | 14 | 1 | 3 | 24 | 324 | 300 | 12 | 0 | 18 | 24 | 0 | 52 | 1 |
| -4 | 1 | 22 | 94 | 67 | 20 | 0 | 12 | 22 | 14 | 50 | 13 | 2 | 7 | 23 | 120 | 122 | 14 | 2 | 3 | 24 | 27 | 37 | 26 | 1 | 0 | 25 | 122 | 111 | 15 |
| -3 | 1 | 22 | 140 | 162 | 63 | 1 | 12 | 22 | 127 | 95 | 14 | 3 | 7 | 23 | 0 | 16 | 1 | 3 | 3 | 24 | 41 | 47 | 41 | 2 | 0 | 25 | 82 | 34 | 18 |
| -2 | 1 | 22 | 129 | 128 | 10 | 2 | 12 | 22 | 57 | 66 | 36 | -3 | 8 | 23 | 78 | 53 | 28 | -3 | 4 | 24 | 71 | 70 | 24 | 3 | 0 | 25 | 200 | 195 | 17 |
| -1 | 1 | 22 | 132 | 125 | 10 | 3 | 12 | 22 | 56 | 64 | 31 | -2 | 8 | 23 | 63 | 22 | 24 | -2 | 4 | 24 | 134 | 124 | 11 | -3 | 1 | 25 | 105 | 95 | 14 |
| 0 | 1 | 22 | 273 | 267 | 13 | -3 | 13 | 22 | 62 | 73 | 21 | -1 | 8 | 23 | 51 | 37 | 51 | -1 | 4 | 24 | 0 | 29 | 1 | -2 | 1 | 25 | 202 | 164 | 9 |
| 1 | 1 | 22 | 111 | 126 | 20 | -2 | 13 | 22 | 126 | 163 | 12 | 0 | 8 | 23 | 153 | 138 | 12 | 0 | 4 | 24 | 283 | 280 | 8 | -1 | 1 | 25 | 96 | 78 | 21 |
| 2 | 1 | 22 | 135 | 128 | 9 | -1 | 13 | 22 | 227 | 225 | 9 | 1 | 8 | 23 | 90 | 37 | 19 | 1 | 4 | 24 | 66 | 28 | 25 | 0 | 1 | 25 | 98 | 111 | 13 |
| 3 | 1 | 22 | 141 | 162 | 11 | 0 | 13 | 22 | 88 | 80 | 25 | 2 | 8 | 23 | 45 | 22 | 44 | 2 | 4 | 24 | 136 | 124 | 10 | 1 | 1 | 25 | 103 | 78 | 27 |
| 2 | 1 | 25 | 179 | 164 | 18 | 0 | 15 | 25 | 86 | 64 | 32 | 2 | 11 | 26 | 103 | 99 | 20 | 2 | 9 | 27 | 135 | 153 | 19 | 2 | 8 | 28 | 97 | 78 | 17 |
| 3 | 1 | 25 | 100 | 95 | 16 | 1 | 15 | 25 | 150 | 162 | 18 | -2 | 12 | 26 | 79 | 78 | 20 | -2 | 10 | 27 | 51 | 59 | 51 | -2 | 9 | 28 | 55 | 73 | 39 |
| -3 | 2 | 25 | 0 | 9 | 1 | 2 | 15 | 25 | 30 | 21 | 29 | -1 | 12 | 26 | 158 | 164 | 10 | -1 | 10 | 27 | 167 | 139 | 13 | -1 | 9 | 28 | 46 | 54 | 45 |
| -2 | 2 | 25 | 60 | 14 | 50 | -1 | 16 | 25 | 31 | 46 | 31 | 0 | 12 | 26 | 124 | 120 | 19 | 0 | 10 | 27 | 54 | 60 | 53 | 0 | 9 | 28 | 0 | 33 | 1 |
| -1 | 2 | 25 | 227 | 206 | 13 | 0 | 16 | 25 | 148 | 190 | 22 | 1 | 12 | 26 | 166 | 164 | 14 | 1 | 10 | 27 | 128 | 140 | 18 | 1 | 9 | 28 | 56 | 55 | 56 |
| 0 | 2 | 25 | 61 | 36 | 21 | 1 | 16 | 25 | 59 | 45 | 58 | 2 | 12 | 26 | 107 | 78 | 23 | 2 | 10 | 27 | 86 | 59 | 23 | 2 | 9 | 28 | 99 | 73 | 16 |
| 1 | 2 | 25 | 224 | 206 | 14 | -1 | 17 | 25 | 61 | 49 | 24 | -2 | 13 | 26 | 110 | 97 | 14 | -2 | 11 | 27 | 49 | 69 | 49 | -2 | 10 | 28 | 74 | 81 | 21 |
| 2 | 2 | 25 | 45 | 14 | 45 | 0 | 17 | 25 | 0 | 30 | 1 | -1 | 13 | 26 | 0 | 35 | 1 | -1 | 11 | 27 | 106 | 106 | 14 | -1 | 10 | 28 | 76 | 34 | 22 |
| 3 | 2 | 25 | 47 | 10 | 47 | 1 | 17 | 25 | 30 | 49 | 29 | 0 | 13 | 26 | 101 | 111 | 22 | 0 | 11 | 27 | 52 | 92 | 52 | 0 | 10 | 28 | 95 | 97 | 27 |
| -3 | 3 | 25 | 45 | 39 | 44 | 0 | 18 | 25 | 0 | 22 | 1 | 1 | 13 | 26 | 37 | 35 | 37 | 1 | 11 | 27 | 110 | 105 | 26 | 1 | 10 | 28 | 50 | 35 | 50 |
| -2 | 3 | 25 | 101 | 65 | 15 | 0 | 0 | 26 | 167 | 153 | 14 | 2 | 13 | 26 | 115 | 97 | 18 | 2 | 11 | 27 | 84 | 68 | 26 | 2 | 10 | 28 | 72 | 81 | 33 |
| -1 | 3 | 25 | 91 | 91 | 31 | 1 | 0 | 26 | 101 | 36 | 24 | -2 | 14 | 26 | 28 | 34 | 27 | -2 | 12 | 27 | 86 | 125 | 36 | -2 | 11 | 28 | 61 | 48 | 30 |
| 0 | 3 | 25 | 70 | 5 | 18 | 2 | 0 | 26 | 146 | 116 | 15 | -1 | 14 | 26 | 43 | 39 | 43 | -1 | 12 | 27 | 94 | 111 | 16 | -1 | 11 | 28 | 95 | 101 | 28 |
| 1 | 3 | 25 | 110 | 92 | 21 | 3 | 0 | 26 | 78 | 35 | 67 | 0 | 14 | 26 | 92 | 47 | 25 | 0 | 12 | 27 | 59 | 6 | 59 | 0 | 11 | 28 | 194 | 168 | 50 |
| 2 | 3 | 25 | 51 | 65 | 36 | -3 | 1 | 26 | 56 | 48 | 29 | 1 | 14 | 26 | 30 | 39 | 29 | 1 | 12 | 27 | 108 | 110 | 19 | 1 | 11 | 28 | 64 | 100 | 41 |
| 3 | 3 | 25 | 0 | 38 | 1 | -2 | 1 | 26 | 76 | 80 | 20 | 2 | 14 | 26 | 0 | 35 | 1 | 2 | 12 | 27 | 119 | 125 | 17 | 2 | 11 | 28 | 46 | 48 | 46 |
| -3 | 4 | 25 | 84 | 90 | 33 | -1 | 1 | 26 | 79 | 43 | 35 | -1 | 15 | 26 | 74 | 57 | 19 | -2 | 13 | 27 | 0 | 53 | 1 | -2 | 12 | 28 | 45 | 20 | 44 |
| -2 | 4 | 25 | 57 | 44 | 31 | 0 | 1 | 26 | 324 | 319 | 10 | 0 | 15 | 26 | 132 | 124 | 17 | -1 | 13 | 27 | 0 | 35 | 1 | -1 | 12 | 28 | 60 | 35 | 30 |
| -1 | 4 | 25 | 185 | 172 | 15 | 1 | 1 | 26 | 82 | 43 | 40 | 1 | 15 | 26 | 69 | 57 | 38 | 0 | 13 | 27 | 197 | 213 | 15 | 0 | 12 | 28 | 167 | 159 | 16 |
| 0 | 4 | 25 | 272 | 278 | 9 | 2 | 1 | 26 | 91 | 81 | 15 | -1 | 16 | 26 | 0 | 51 | 1 | 1 | 13 | 27 | 54 | 36 | 53 | 1 | 12 | 28 | 67 | 36 | 48 |
| 1 | 4 | 25 | 175 | 172 | 12 | 3 | 1 | 26 | 46 | 49 | 46 | 0 | 16 | 26 | 58 | 66 | 58 | 2 | 13 | 27 | 78 | 53 | 77 | 2 | 12 | 28 | 0 | 20 | 1 |
| 2 | 4 | 25 | 54 | 45 | 27 | -3 | 2 | 26 | 119 | 132 | 13 | 1 | 16 | 26 | 53 | 51 | 52 | -1 | 14 | 27 | 56 | 65 | 31 | -2 | 13 | 28 | 52 | 66 | 52 |
| 3 | 4 | 25 | 85 | 90 | 15 | -2 | 2 | 26 | 141 | 132 | 12 | 0 | 17 | 26 | 0 | 17 | 1 | 0 | 14 | 27 | 60 | 31 | 60 | -1 | 13 | 28 | 114 | 133 | 13 |
| -3 | 5 | 25 | 0 | 11 | 1 | -1 | 2 | 26 | 159 | 105 | 15 | 1 | 0 | 27 | 104 | 67 | 24 | 1 | 14 | 27 | 0 | 65 | 1 | 0 | 13 | 28 | 57 | 7 | 57 |
| -2 | 5 | 25 | 118 | 114 | 12 | 0 | 2 | 26 | 146 | 129 | 10 | 2 | 0 | 27 | 198 | 166 | 13 | -1 | 15 | 27 | 91 | 76 | 15 | 1 | 13 | 28 | 99 | 133 | 24 |
| -1 | 5 | 25 | 83 | 101 | 31 | 1 | 2 | 26 | 126 | 104 | 20 | 3 | 0 | 27 | 74 | 56 | 19 | 0 | 15 | 27 | 52 | 17 | 52 | -1 | 14 | 28 | 84 | 55 | 18 |
| 0 | 5 | 25 | 171 | 175 | 9 | 2 | 2 | 26 | 141 | 132 | 13 | -3 | 1 | 27 | 124 | 125 | 12 | 1 | 15 | 27 | 78 | 76 | 28 | 0 | 14 | 28 | 68 | 67 | 43 |
| 1 | 5 | 25 | 89 | 101 | 24 | 3 | 2 | 26 | 117 | 133 | 11 | -2 | 1 | 27 | 135 | 127 | 11 | -1 | 16 | 27 | 28 | 22 | 27 | 1 | 14 | 28 | 63 | 55 | 45 |
| 2 | 5 | 25 | 135 | 114 | 13 | -3 | 3 | 26 | 81 | 51 | 19 | -1 | 1 | 27 | 124 | 129 | 20 | 0 | 16 | 27 | 110 | 119 | 27 | -1 | 15 | 28 | 130 | 131 | 12 |
| 3 | 5 | 25 | 32 | 11 | 32 | -2 | 3 | 26 | 68 | 79 | 22 | 0 | 1 | 27 | 136 | 123 | 11 | 1 | 16 | 27 | 38 | 23 | 38 | 0 | 15 | 28 | 0 | 25 | 1 |
| -3 | 6 | 25 | 101 | 68 | 24 | -1 | 3 | 26 | 120 | 91 | 22 | 1 | 1 | 27 | 142 | 129 | 18 | 0 | 0 | 28 | 376 | 331 | 19 | 1 | 15 | 28 | 117 | 132 | 17 |
| -2 | 6 | 25 | 146 | 155 | 10 | 0 | 3 | 26 | 82 | 51 | 15 | 2 | 1 | 27 | 155 | 127 | 18 | 1 | 0 | 28 | 132 | 99 | 21 | 0 | 16 | 28 | 0 | 31 | 1 |
| -1 | 6 | 25 | 104 | 89 | 20 | 1 | 3 | 26 | 125 | 91 | 21 | 3 | 1 | 27 | 141 | 125 | 12 | 2 | 0 | 28 | 49 | 36 | 49 | 1 | 0 | 29 | 227 | 220 | 29 |
| 0 | 6 | 25 | 107 | 83 | 16 | 2 | 3 | 26 | 80 | 79 | 16 | -3 | 2 | 27 | 103 | 106 | 13 | 3 | 0 | 28 | 71 | 71 | 19 | 2 | 0 | 29 | 87 | 76 | 15 |
| 1 | 6 | 25 | 122 | 88 | 14 | 3 | 3 | 26 | 0 | 51 | 1 | -2 | 2 | 27 | 106 | 87 | 15 | -3 | 1 | 28 | 102 | 106 | 13 | 3 | 0 | 29 | 59 | 59 | 26 |
| 2 | 6 | 25 | 128 | 154 | 14 | -3 | 4 | 26 | 146 | 112 | 20 | -1 | 2 | 27 | 79 | 66 | 36 | -2 | 1 | 28 | 97 | 67 | 24 | -3 | 1 | 29 | 52 | 54 | 51 |
| 3 | 6 | 25 | 77 | 69 | 17 | -2 | 4 | 26 | 102 | 64 | 13 | 0 | 2 | 27 | 126 | 122 | 13 | -1 | 1 | 28 | 175 | 128 | 15 | -2 | 1 | 29 | 135 | 139 | 24 |
| -3 | 7 | 25 | 151 | 150 | 11 | -1 | 4 | 26 | 181 | 168 | 15 | 1 | 2 | 27 | 92 | 66 | 27 | 0 | 1 | 28 | 50 | 17 | 50 | -1 | 1 | 29 | 110 | 51 | 20 |
| -2 | 7 | 25 | 136 | 111 | 12 | 0 | 4 | 26 | 78 | 62 | 26 | 2 | 2 | 27 | 107 | 87 | 14 | 1 | 1 | 28 | 130 | 128 | 20 | 0 | 1 | 29 | 37 | 28 | 36 |
| -1 | 7 | 25 | 89 | 41 | 24 | 1 | 4 | 26 | 161 | 168 | 16 | 3 | 2 | 27 | 112 | 107 | 14 | 2 | 1 | 28 | 69 | 67 | 21 | 1 | 1 | 29 | 74 | 51 | 36 |
| 0 | 7 | 25 | 46 | 66 | 46 | 2 | 4 | 26 | 61 | 64 | 22 | -3 | 3 | 27 | 168 | 152 | 15 | 3 | 1 | 28 | 132 | 106 | 12 | 2 | 1 | 29 | 124 | 140 | 10 |
| 1 | 7 | 25 | 96 | 42 | 35 | 3 | 4 | 26 | 107 | 113 | 13 | -2 | 3 | 27 | 135 | 139 | 11 | -3 | 2 | 28 | 108 | 116 | 13 | 3 | 1 | 29 | 87 | 54 | 20 |
| 2 | 7 | 25 | 127 | 111 | 14 | -3 | 5 | 26 | 84 | 69 | 18 | -1 | 3 | 27 | 229 | 239 | 14 | -2 | 2 | 28 | 118 | 118 | 12 | -3 | 2 | 29 | 109 | 104 | 12 |
| 3 | 7 | 25 | 130 | 150 | 10 | -2 | 5 | 26 | 166 | 151 | 10 | 0 | 3 | 27 | 98 | 100 | 27 | -1 | 2 | 28 | 129 | 127 | 31 | -2 | 2 | 29 | 126 | 88 | 14 |
| -3 | 8 | 25 | 49 | 60 | 49 | -1 | 5 | 26 | 109 | 42 | 23 | 1 | 3 | 27 | 226 | 240 | 30 | 0 | 2 | 28 | 70 | 11 | 27 | -1 | 2 | 29 | 301 | 291 | 33 |
| -2 | 8 | 25 | 104 | 91 | 15 | 0 | 5 | 26 | 13 | 5 | 13 | 2 | 3 | 27 | 116 | 140 | 13 | 1 | 2 | 28 | 151 | 126 | 16 | 0 | 2 | 29 | 120 | 99 | 17 |
| -1 | 8 | 25 | 191 | 203 | 11 | 1 | 5 | 26 | 86 | 42 | 32 | 3 | 3 | 27 | 143 | 151 | 11 | 2 | 2 | 28 | 107 | 117 | 12 | 1 | 2 | 29 | 252 | 291 | 26 |
| 0 | 8 | 25 | 26 | 43 | 26 | 2 | 5 | 26 | 151 | 150 | 17 | -3 | 4 | 27 | 66 | 56 | 25 | 3 | 2 | 28 | 139 | 117 | 12 | 2 | 2 | 29 | 90 | 88 | 13 |
| 1 | 8 | 25 | 200 | 202 | 15 | 3 | 5 | 26 | 70 | 69 | 20 | -2 | 4 | 27 | 110 | 112 | 15 | -3 | 3 | 28 | 73 | 42 | 18 | 3 | 2 | 29 | 126 | 104 | 14 |
| 2 | 8 | 25 | 101 | 91 | 16 | -3 | 6 | 26 | 70 | 26 | 22 | -1 | 4 | 27 | 49 | 43 | 49 | -2 | 3 | 28 | 184 | 168 | 13 | -3 | 3 | 29 | 114 | 124 | 12 |
| 3 | 8 | 25 | 80 | 59 | 18 | -2 | 6 | 26 | 0 | 31 | 1 | 0 | 4 | 27 | 310 | 274 | 12 | -1 | 3 | 28 | 112 | 84 | 40 | -2 | 3 | 29 | 70 | 42 | 27 |
| -3 | 9 | 25 | 0 | 35 | 1 | -1 | 6 | 26 | 195 | 185 | 15 | 1 | 4 | 27 | 0 | 43 | 1 | 0 | 3 | 28 | 118 | 105 | 15 | -1 | 3 | 29 | 62 | 38 | 61 |
| -2 | 9 | 25 | 20 | 45 | 20 | 0 | 6 | 26 | 127 | 131 | 14 | 2 | 4 | 27 | 133 | 113 | 19 | 1 | 3 | 28 | 103 | 83 | 22 | 0 | 3 | 29 | 297 | 282 | 11 |
| -1 | 9 | 25 | 200 | 188 | 10 | 1 | 6 | 26 | 183 | 184 | 15 | 3 | 4 | 27 | 63 | 56 | 24 | 2 | 3 | 28 | 175 | 168 | 14 | 1 | 3 | 29 | 55 | 39 | 55 |
| 0 | 9 | 25 | 153 | 164 | 26 | 2 | 6 | 26 | 6 | 31 | 5 | -3 | 5 | 27 | 0 | 41 | 1 | 3 | 3 | 28 | 44 | 42 | 43 | 2 | 3 | 29 | 0 | 41 | 1 |
| 1 | 9 | 25 | 208 | 188 | 21 | 3 | 6 | 26 | 0 | 26 | 1 | -2 | 5 | 27 | 102 | 67 | 18 | -3 | 4 | 28 | 179 | 161 | 10 | 3 | 3 | 29 | 113 | 124 | 15 |
| 2 | 9 | 25 | 65 | 44 | 29 | -3 | 7 | 26 | 39 | 25 | 38 | -1 | 5 | 27 | 191 | 175 | 16 | -2 | 4 | 28 | 106 | 119 | 21 | -3 | 4 | 29 | 50 | 73 | 50 |
| 3 | 9 | 25 | 72 | 34 | 71 | -2 | 7 | 26 | 86 | 59 | 19 | 0 | 5 | 27 | 69 | 18 | 29 | -1 | 4 | 28 | 150 | 182 | 17 | -2 | 4 | 29 | 53 | 60 | 53 |
| -3 | 10 | 25 | 68 | 73 | 34 | -1 | 7 | 26 | 189 | 165 | 12 | 1 | 5 | 27 | 192 | 174 | 32 | 0 | 4 | 28 | 459 | 389 | 15 | -1 | 4 | 29 | 94 | 90 | 25 |
| -2 | 10 | 25 | 24 | 37 | 23 | 0 | 7 | 26 | 51 | 25 | 50 | 2 | 5 | 27 | 64 | 67 | 62 | 1 | 4 | 28 | 168 | 182 | 14 | 0 | 4 | 29 | 84 | 62 | 22 |
| -1 | 10 | 25 | 132 | 115 | 12 | 1 | 7 | 26 | 167 | 165 | 16 | 3 | 5 | 27 | 16 | 41 | 16 | 2 | 4 | 28 | 157 | 119 | 23 | 1 | 4 | 29 | 113 | 90 | 18 |
| 0 | 10 | 25 | 101 | 79 | 27 | 2 | 7 | 26 | 86 | 60 | 19 | -3 | 6 | 27 | 51 | 36 | 50 | 3 | 4 | 28 | 158 | 161 | 12 | 2 | 4 | 29 | 70 | 59 | 24 |
| 1 | 10 | 25 | 114 | 116 | 54 | 3 | 7 | 26 | 0 | 25 | 1 | -2 | 6 | 27 | 176 | 182 | 12 | -3 | 5 | 28 | 0 | 21 | 1 | 3 | 4 | 29 | 82 | 73 | 17 |
| 2 | 10 | 25 | 81 | 37 | 20 | -3 | 8 | 26 | 87 | 75 | 17 | -1 | 6 | 27 | 130 | 91 | 24 | -2 | 5 | 28 | 123 | 125 | 14 | -3 | 5 | 29 | 105 | 108 | 15 |
| 3 | 10 | 25 | 75 | 73 | 20 | -2 | 8 | 26 | 77 | 25 | 22 | 0 | 6 | 27 | 387 | 341 | 13 | -1 | 5 | 28 | 79 | 31 | 38 | -2 | 5 | 29 | 173 | 154 | 17 |
| -2 | 11 | 25 | 176 | 147 | 10 | -1 | 8 | 26 | 46 | 39 | 46 | 1 | 6 | 27 | 99 | 90 | 24 | 0 | 5 | 28 | 375 | 314 | 13 | -1 | 5 | 29 | 238 | 233 | 25 |
| -1 | 11 | 25 | 334 | 282 | 10 | 0 | 8 | 26 | 72 | 52 | 32 | 2 | 6 | 27 | 175 | 182 | 15 | 1 | 5 | 28 | 0 | 31 | 1 | 0 | 5 | 29 | 46 | 23 | 46 |
| 0 | 11 | 25 | 92 | 85 | 30 | 1 | 8 | 26 | 0 | 40 | 1 | 3 | 6 | 27 | 53 | 36 | 39 | 2 | 5 | 28 | 142 | 125 | 17 | 1 | 5 | 29 | 215 | 234 | 14 |
| 1 | 11 | 25 | 289 | 282 | 19 | 2 | 8 | 26 | 40 | 25 | 39 | -3 | 7 | 27 | 51 | 32 | 51 | 3 | 5 | 28 | 58 | 21 | 28 | 2 | 5 | 29 | 128 | 154 | 18 |
| 2 | 11 | 25 | 137 | 147 | 16 | 3 | 8 | 26 | 97 | 74 | 15 | -2 | 7 | 27 | 68 | 21 | 25 | -3 | 6 | 28 | 0 | 50 | 1 | 3 | 5 | 29 | 81 | 108 | 22 |
| -2 | 12 | 25 | 129 | 139 | 14 | -3 | 9 | 26 | 131 | 76 | 15 | -1 | 7 | 27 | 183 | 175 | 10 | -2 | 6 | 28 | 68 | 76 | 26 | -2 | 6 | 29 | 0 | 24 | 1 |
| -1 | 12 | 25 | 119 | 110 | 12 | -2 | 9 | 26 | 61 | 80 | 30 | 0 | 7 | 27 | 265 | 251 | 12 | -1 | 6 | 28 | 268 | 259 | 13 | -1 | 6 | 29 | 0 | 44 | 1 |
| 0 | 12 | 25 | 132 | 113 | 20 | -1 | 9 | 26 | 153 | 127 | 11 | 1 | 7 | 27 | 160 | 174 | 16 | 0 | 6 | 28 | 69 | 25 | 28 | 0 | 6 | 29 | 129 | 150 | 15 |
| 1 | 12 | 25 | 109 | 111 | 49 | 0 | 9 | 26 | 112 | 70 | 18 | 2 | 7 | 27 | 21 | 21 | 21 | 1 | 6 | 28 | 225 | 259 | 18 | 1 | 6 | 29 | 0 | 43 | 1 |
| 2 | 12 | 25 | 156 | 139 | 14 | 1 | 9 | 26 | 122 | 127 | 27 | 3 | 7 | 27 | 31 | 32 | 30 | 2 | 6 | 28 | 53 | 76 | 53 | 2 | 6 | 29 | 61 | 24 | 56 |
| -2 | 13 | 25 | 177 | 187 | 11 | 2 | 9 | 26 | 116 | 80 | 19 | -3 | 8 | 27 | 53 | 49 | 53 | 3 | 6 | 28 | 55 | 50 | 55 | -2 | 7 | 29 | 69 | 52 | 29 |
| -1 | 13 | 25 | 78 | 77 | 20 | 3 | 9 | 26 | 110 | 76 | 13 | -2 | 8 | 27 | 200 | 206 | 10 | -3 | 7 | 28 | 89 | 96 | 22 | -1 | 7 | 29 | 66 | 50 | 32 |
| 0 | 13 | 25 | 222 | 210 | 28 | -3 | 10 | 26 | 114 | 34 | 42 | -1 | 8 | 27 | 217 | 209 | 10 | -2 | 7 | 28 | 136 | 124 | 12 | 0 | 7 | 29 | 189 | 159 | 12 |
| 1 | 13 | 25 | 64 | 77 | 64 | -2 | 10 | 26 | 127 | 101 | 23 | 0 | 8 | 27 | 74 | 58 | 29 | -1 | 7 | 28 | 165 | 141 | 15 | 1 | 7 | 29 | 0 | 50 | 1 |
| 2 | 13 | 25 | 198 | 188 | 13 | -1 | 10 | 26 | 99 | 71 | 21 | 1 | 8 | 27 | 202 | 209 | 15 | 0 | 7 | 28 | 89 | 71 | 40 | 2 | 7 | 29 | 62 | 52 | 30 |
| -2 | 14 | 25 | 43 | 45 | 42 | 0 | 10 | 26 | 107 | 117 | 25 | 2 | 8 | 27 | 218 | 207 | 28 | 1 | 7 | 28 | 116 | 141 | 19 | -2 | 8 | 29 | 53 | 59 | 53 |
| -1 | 14 | 25 | 179 | 177 | 9 | 1 | 10 | 26 | 100 | 72 | 23 | 3 | 8 | 27 | 57 | 50 | 48 | 2 | 7 | 28 | 95 | 124 | 20 | -1 | 8 | 29 | 45 | 16 | 45 |
| 0 | 14 | 25 | 244 | 266 | 15 | 2 | 10 | 26 | 107 | 101 | 16 | -3 | 9 | 27 | 0 | 69 | 1 | 3 | 7 | 28 | 83 | 96 | 28 | 0 | 8 | 29 | 87 | 59 | 33 |
| 1 | 14 | 25 | 184 | 176 | 17 | -2 | 11 | 26 | 84 | 99 | 19 | -2 | 9 | 27 | 139 | 153 | 11 | -2 | 8 | 28 | 96 | 78 | 23 | 1 | 8 | 29 | 53 | 16 | 52 |
| 2 | 14 | 25 | 0 | 44 | 1 | -1 | 11 | 26 | 169 | 176 | 13 | -1 | 9 | 27 | 114 | 126 | 14 | -1 | 8 | 28 | 133 | 111 | 12 | 2 | 8 | 29 | 54 | 60 | 53 |
| -2 | 15 | 25 | 39 | 22 | 39 | 0 | 11 | 26 | 63 | 119 | 62 | 0 | 9 | 27 | 194 | 140 | 15 | 0 | 8 | 28 | 67 | 55 | 35 | -2 | 9 | 29 | 68 | 24 | 25 |
| -1 | 15 | 25 | 178 | 162 | 9 | 1 | 11 | 26 | 167 | 176 | 29 | 1 | 9 | 27 | 117 | 126 | 28 | 1 | 8 | 28 | 133 | 111 | 17 | -1 | 9 | 29 | 17 | 2 | 17 |
| 0 | 9 | 29 | 95 | 67 | 19 | -1 | 8 | 30 | 104 | 96 | 26 | -1 | 9 | 31 | 96 | 99 | 15 | 1 | 0 | 33 | 154 | 164 | 15 | 1 | 5 | 34 | 111 | 115 | 19 |
| 1 | 9 | 29 | 0 | 2 | 1 | 0 | 8 | 30 | 126 | 95 | 18 | 0 | 9 | 31 | 71 | 30 | 37 | 2 | 0 | 33 | 84 | 33 | 27 | -1 | 6 | 34 | 68 | 61 | 28 |
| 2 | 9 | 29 | 0 | 24 | 1 | 1 | 8 | 30 | 75 | 96 | 35 | 1 | 9 | 31 | 126 | 98 | 16 | -2 | 1 | 33 | 122 | 104 | 20 | 0 | 6 | 34 | 0 | 17 | 1 |
| -2 | 10 | 29 | 46 | 46 | 45 | 2 | 8 | 30 | 83 | 133 | 51 | 2 | 9 | 31 | 115 | 71 | 33 | -1 | 1 | 33 | 109 | 108 | 18 | 1 | 6 | 34 | 107 | 62 | 20 |
| -1 | 10 | 29 | 161 | 167 | 10 | -2 | 9 | 30 | 0 | 51 | 1 | -1 | 10 | 31 | 63 | 62 | 28 | 0 | 1 | 33 | 163 | 152 | 11 | -1 | 7 | 34 | 60 | 72 | 36 |
| 0 | 10 | 29 | 79 | 35 | 22 | -1 | 9 | 30 | 135 | 129 | 18 | 0 | 10 | 31 | 87 | 85 | 20 | 1 | 1 | 33 | 92 | 108 | 23 | 0 | 7 | 34 | 37 | 52 | 37 |
| 1 | 10 | 29 | 163 | 167 | 61 | 0 | 9 | 30 | 206 | 209 | 16 | 1 | 10 | 31 | 84 | 62 | 25 | 2 | 1 | 33 | 82 | 104 | 40 | 1 | 7 | 34 | 61 | 73 | 61 |
| 2 | 10 | 29 | 0 | 46 | 1 | 1 | 9 | 30 | 144 | 129 | 40 | -1 | 11 | 31 | 54 | 62 | 37 | -2 | 2 | 33 | 97 | 78 | 15 | -1 | 8 | 34 | 0 | 37 | 1 |
| -2 | 11 | 29 | 96 | 55 | 16 | 2 | 9 | 30 | 83 | 51 | 61 | 0 | 11 | 31 | 0 | 43 | 1 | -1 | 2 | 33 | 77 | 115 | 26 | 0 | 8 | 34 | 100 | 86 | 17 |
| -1 | 11 | 29 | 97 | 79 | 15 | -2 | 10 | 30 | 30 | 20 | 29 | 1 | 11 | 31 | 78 | 62 | 32 | 0 | 2 | 33 | 143 | 129 | 14 | 1 | 8 | 34 | 73 | 37 | 32 |
| 0 | 11 | 29 | 143 | 97 | 17 | -1 | 10 | 30 | 142 | 136 | 11 | -1 | 12 | 31 | 15 | 13 | 14 | 1 | 2 | 33 | 86 | 114 | 25 | -1 | 9 | 34 | 81 | 62 | 21 |
| 1 | 11 | 29 | 82 | 79 | 26 | 0 | 10 | 30 | 14 | 24 | 14 | 0 | 12 | 31 | 0 | 33 | 1 | 2 | 2 | 33 | 75 | 79 | 50 | 0 | 9 | 34 | 0 | 42 | 1 |
| 2 | 11 | 29 | 0 | 54 | 1 | 1 | 10 | 30 | 129 | 137 | 17 | 1 | 12 | 31 | 0 | 14 | 1 | -2 | 3 | 33 | 0 | 33 | 1 | 1 | 9 | 34 | 78 | 62 | 33 |
| -2 | 12 | 29 | 46 | 42 | 46 | 2 | 10 | 30 | 76 | 20 | 75 | -1 | 13 | 31 | 92 | 107 | 32 | -1 | 3 | 33 | 69 | 31 | 31 | 0 | 10 | 34 | 83 | 63 | 27 |
| -1 | 12 | 29 | 17 | 49 | 16 | -2 | 11 | 30 | 50 | 33 | 50 | 0 | 13 | 31 | 41 | 17 | 40 | 0 | 3 | 33 | 188 | 199 | 11 | 1 | 0 | 35 | 0 | 6 | 1 |
| 0 | 12 | 29 | 227 | 193 | 18 | -1 | 11 | 30 | 65 | 59 | 26 | 0 | 0 | 32 | 185 | 180 | 18 | 1 | 3 | 33 | 71 | 31 | 44 | -1 | 1 | 35 | 61 | 24 | 54 |
| 1 | 12 | 29 | 0 | 49 | 1 | 0 | 11 | 30 | 39 | 7 | 39 | 1 | 0 | 32 | 139 | 166 | 15 | 2 | 3 | 33 | 55 | 33 | 55 | 0 | 1 | 35 | 89 | 39 | 25 |
| -1 | 13 | 29 | 47 | 66 | 47 | 1 | 11 | 30 | 75 | 60 | 36 | 2 | 0 | 32 | 46 | 38 | 45 | -2 | 4 | 33 | 92 | 73 | 19 | 1 | 1 | 35 | 62 | 24 | 45 |
| 0 | 13 | 29 | 0 | 42 | 1 | -1 | 12 | 30 | 46 | 54 | 45 | -2 | 1 | 32 | 128 | 126 | 12 | -1 | 4 | 33 | 112 | 140 | 14 | -1 | 2 | 35 | 75 | 63 | 74 |
| 1 | 13 | 29 | 81 | 66 | 28 | 0 | 12 | 30 | 34 | 25 | 34 | -1 | 1 | 32 | 92 | 124 | 21 | 0 | 4 | 33 | 78 | 75 | 24 | 0 | 2 | 35 | 0 | 71 | 1 |
| -1 | 14 | 29 | 97 | 92 | 21 | 1 | 12 | 30 | 40 | 55 | 40 | 0 | 1 | 32 | 105 | 106 | 17 | 1 | 4 | 33 | 148 | 139 | 23 | 1 | 2 | 35 | 106 | 63 | 21 |
| 0 | 14 | 29 | 55 | 26 | 54 | -1 | 13 | 30 | 72 | 88 | 34 | 1 | 1 | 32 | 98 | 124 | 21 | 2 | 4 | 33 | 27 | 74 | 26 | -1 | 3 | 35 | 0 | 30 | 1 |
| 1 | 14 | 29 | 92 | 92 | 21 | 0 | 13 | 30 | 0 | 4 | 1 | 2 | 1 | 32 | 125 | 126 | 24 | -2 | 5 | 33 | 82 | 76 | 19 | 0 | 3 | 35 | 96 | 80 | 20 |
| 0 | 15 | 29 | 0 | 30 | 1 | 1 | 13 | 30 | 69 | 87 | 33 | -2 | 2 | 32 | 77 | 79 | 25 | -1 | 5 | 33 | 120 | 120 | 17 | 1 | 3 | 35 | 0 | 30 | 1 |
| 0 | 0 | 30 | 95 | 91 | 28 | -1 | 14 | 30 | 85 | 87 | 33 | -1 | 2 | 32 | 63 | 50 | 39 | 0 | 5 | 33 | 80 | 57 | 23 | -1 | 4 | 35 | 89 | 93 | 19 |
| 1 | 0 | 30 | 12 | 14 | 12 | 0 | 14 | 30 | 33 | 10 | 33 | 0 | 2 | 32 | 93 | 84 | 19 | 1 | 5 | 33 | 133 | 121 | 17 | 0 | 4 | 35 | 0 | 18 | 1 |
| 2 | 0 | 30 | 144 | 143 | 13 | 1 | 0 | 31 | 23 | 11 | 23 | 1 | 2 | 32 | 69 | 50 | 36 | 2 | 5 | 33 | 77 | 76 | 77 | 1 | 4 | 35 | 109 | 93 | 21 |
| 3 | 0 | 30 | 104 | 93 | 16 | 2 | 0 | 31 | 128 | 146 | 14 | 2 | 2 | 32 | 62 | 79 | 61 | -2 | 6 | 33 | 20 | 19 | 19 | -1 | 5 | 35 | 46 | 50 | 45 |
| -3 | 1 | 30 | 70 | 70 | 27 | -2 | 1 | 31 | 123 | 115 | 15 | -2 | 3 | 32 | 181 | 163 | 11 | -1 | 6 | 33 | 0 | 42 | 1 | 0 | 5 | 35 | 0 | 6 | 1 |
| -2 | 1 | 30 | 71 | 120 | 70 | -1 | 1 | 31 | 159 | 177 | 28 | -1 | 3 | 32 | 66 | 70 | 36 | 0 | 6 | 33 | 65 | 61 | 32 | 1 | 5 | 35 | 27 | 49 | 26 |
| -1 | 1 | 30 | 203 | 200 | 32 | 0 | 1 | 31 | 187 | 200 | 15 | 0 | 3 | 32 | 34 | 39 | 34 | 1 | 6 | 33 | 55 | 42 | 54 | -1 | 6 | 35 | 107 | 115 | 16 |
| 0 | 1 | 30 | 54 | 39 | 54 | 1 | 1 | 31 | 173 | 177 | 21 | 1 | 3 | 32 | 77 | 70 | 30 | 2 | 6 | 33 | 0 | 20 | 1 | 0 | 6 | 35 | 70 | 54 | 27 |
| 1 | 1 | 30 | 182 | 200 | 38 | 2 | 1 | 31 | 108 | 116 | 15 | 2 | 3 | 32 | 121 | 163 | 30 | -1 | 7 | 33 | 111 | 112 | 14 | 1 | 6 | 35 | 126 | 115 | 17 |
| 2 | 1 | 30 | 126 | 120 | 12 | -2 | 2 | 31 | 72 | 95 | 24 | -2 | 4 | 32 | 81 | 70 | 21 | 0 | 7 | 33 | 69 | 44 | 29 | -1 | 7 | 35 | 0 | 56 | 1 |
| 3 | 1 | 30 | 53 | 70 | 53 | -1 | 2 | 31 | 0 | 79 | 1 | -1 | 4 | 32 | 225 | 263 | 11 | 1 | 7 | 33 | 137 | 111 | 18 | 0 | 7 | 35 | 77 | 84 | 24 |
| -3 | 2 | 30 | 49 | 33 | 48 | 0 | 2 | 31 | 25 | 15 | 24 | 0 | 4 | 32 | 96 | 98 | 20 | -1 | 8 | 33 | 62 | 73 | 33 | 1 | 7 | 35 | 48 | 56 | 48 |
| -2 | 2 | 30 | 37 | 64 | 36 | 1 | 2 | 31 | 87 | 79 | 24 | 1 | 4 | 32 | 219 | 263 | 14 | 0 | 8 | 33 | 6 | 18 | 5 | -1 | 8 | 35 | 80 | 26 | 38 |
| -1 | 2 | 30 | 143 | 136 | 23 | 2 | 2 | 31 | 56 | 94 | 55 | 2 | 4 | 32 | 89 | 71 | 24 | 1 | 8 | 33 | 82 | 74 | 27 | 0 | 8 | 35 | 46 | 50 | 46 |
| 0 | 2 | 30 | 146 | 144 | 12 | -2 | 3 | 31 | 165 | 165 | 18 | -2 | 5 | 32 | 42 | 55 | 41 | -1 | 9 | 33 | 69 | 70 | 27 | 0 | 9 | 35 | 0 | 7 | 1 |
| 1 | 2 | 30 | 106 | 136 | 20 | -1 | 3 | 31 | 71 | 87 | 32 | -1 | 5 | 32 | 96 | 113 | 19 | 0 | 9 | 33 | 63 | 27 | 63 | 0 | 0 | 36 | 97 | 106 | 35 |
| 2 | 2 | 30 | 63 | 64 | 23 | 0 | 3 | 31 | 103 | 113 | 20 | 0 | 5 | 32 | 138 | 131 | 14 | 1 | 9 | 33 | 0 | 70 | 1 | 1 | 0 | 36 | 55 | 1 | 55 |
| 3 | 2 | 30 | 52 | 33 | 51 | 1 | 3 | 31 | 88 | 86 | 31 | 1 | 5 | 32 | 104 | 113 | 45 | -1 | 10 | 33 | 8 | 50 | 8 | -1 | 1 | 36 | 23 | 76 | 22 |
| -3 | 3 | 30 | 0 | 21 | 1 | 2 | 3 | 31 | 117 | 166 | 19 | 2 | 5 | 32 | 0 | 55 | 1 | 0 | 10 | 33 | 48 | 11 | 48 | 0 | 1 | 36 | 28 | 24 | 28 |
| -2 | 3 | 30 | 54 | 63 | 49 | -2 | 4 | 31 | 126 | 122 | 13 | -2 | 6 | 32 | 61 | 30 | 32 | 1 | 10 | 33 | 46 | 50 | 45 | 1 | 1 | 36 | 0 | 76 | 1 |
| -1 | 3 | 30 | 57 | 68 | 56 | -1 | 4 | 31 | 69 | 68 | 36 | -1 | 6 | 32 | 134 | 146 | 12 | 0 | 11 | 33 | 132 | 125 | 13 | -1 | 2 | 36 | 0 | 26 | 1 |
| 0 | 3 | 30 | 300 | 302 | 11 | 0 | 4 | 31 | 103 | 87 | 18 | 0 | 6 | 32 | 96 | 116 | 17 | 0 | 0 | 34 | 254 | 258 | 25 | 0 | 2 | 36 | 49 | 87 | 48 |
| 1 | 3 | 30 | 43 | 68 | 43 | 1 | 4 | 31 | 100 | 69 | 20 | 1 | 6 | 32 | 145 | 146 | 15 | 1 | 0 | 34 | 57 | 41 | 56 | 1 | 2 | 36 | 41 | 25 | 40 |
| 2 | 3 | 30 | 0 | 63 | 1 | 2 | 4 | 31 | 110 | 122 | 19 | 2 | 6 | 32 | 0 | 30 | 1 | 2 | 0 | 34 | 0 | 45 | 1 | -1 | 3 | 36 | 56 | 32 | 47 |
| -2 | 4 | 30 | 134 | 121 | 16 | -2 | 5 | 31 | 109 | 93 | 15 | -2 | 7 | 32 | 49 | 23 | 49 | -2 | 1 | 34 | 51 | 20 | 50 | 0 | 3 | 36 | 84 | 38 | 25 |
| -1 | 4 | 30 | 99 | 113 | 27 | -1 | 5 | 31 | 159 | 179 | 32 | -1 | 7 | 32 | 99 | 115 | 18 | -1 | 1 | 34 | 120 | 141 | 17 | 1 | 3 | 36 | 0 | 32 | 1 |
| 0 | 4 | 30 | 229 | 206 | 10 | 0 | 5 | 31 | 0 | 25 | 1 | 0 | 7 | 32 | 106 | 82 | 52 | 0 | 1 | 34 | 150 | 131 | 13 | -1 | 4 | 36 | 78 | 49 | 29 |
| 1 | 4 | 30 | 98 | 113 | 19 | 1 | 5 | 31 | 167 | 178 | 14 | 1 | 7 | 32 | 115 | 115 | 17 | 1 | 1 | 34 | 131 | 141 | 17 | 0 | 4 | 36 | 23 | 16 | 23 |
| 2 | 4 | 30 | 85 | 121 | 29 | 2 | 5 | 31 | 0 | 93 | 1 | 2 | 7 | 32 | 0 | 23 | 1 | 2 | 1 | 34 | 33 | 20 | 32 | 1 | 4 | 36 | 0 | 49 | 1 |
| -2 | 5 | 30 | 110 | 114 | 17 | -2 | 6 | 31 | 140 | 145 | 13 | -2 | 8 | 32 | 50 | 83 | 49 | -2 | 2 | 34 | 84 | 76 | 26 | -1 | 5 | 36 | 55 | 40 | 55 |
| -1 | 5 | 30 | 78 | 81 | 22 | -1 | 6 | 31 | 80 | 33 | 21 | -1 | 8 | 32 | 81 | 91 | 21 | -1 | 2 | 34 | 92 | 103 | 21 | 0 | 5 | 36 | 44 | 40 | 43 |
| 0 | 5 | 30 | 125 | 120 | 14 | 0 | 6 | 31 | 167 | 181 | 12 | 0 | 8 | 32 | 70 | 38 | 28 | 0 | 2 | 34 | 123 | 120 | 15 | 1 | 5 | 36 | 51 | 40 | 51 |
| 1 | 5 | 30 | 52 | 81 | 52 | 1 | 6 | 31 | 42 | 33 | 41 | 1 | 8 | 32 | 82 | 90 | 28 | 1 | 2 | 34 | 103 | 103 | 20 | 0 | 6 | 36 | 85 | 58 | 23 |
| 2 | 5 | 30 | 105 | 114 | 20 | 2 | 6 | 31 | 151 | 145 | 34 | 2 | 8 | 32 | 112 | 83 | 33 | 2 | 2 | 34 | 0 | 76 | 1 | 0 | 7 | 36 | 88 | 105 | 22 |
| -2 | 6 | 30 | 135 | 142 | 13 | -2 | 7 | 31 | 78 | 57 | 22 | -1 | 9 | 32 | 36 | 39 | 35 | -2 | 3 | 34 | 51 | 39 | 50 | 1 | 0 | 37 | 87 | 62 | 26 |
| -1 | 6 | 30 | 109 | 121 | 15 | -1 | 7 | 31 | 77 | 100 | 48 | 0 | 9 | 32 | 50 | 68 | 50 | -1 | 3 | 34 | 95 | 103 | 17 | -1 | 1 | 37 | 75 | 69 | 62 |
| 0 | 6 | 30 | 47 | 48 | 47 | 0 | 7 | 31 | 142 | 140 | 12 | 1 | 9 | 32 | 65 | 39 | 42 | 0 | 3 | 34 | 161 | 176 | 24 | 0 | 1 | 37 | 68 | 67 | 37 |
| 1 | 6 | 30 | 111 | 121 | 18 | 1 | 7 | 31 | 86 | 99 | 34 | -1 | 10 | 32 | 60 | 63 | 29 | 1 | 3 | 34 | 101 | 103 | 21 | 1 | 1 | 37 | 82 | 69 | 35 |
| 2 | 6 | 30 | 121 | 142 | 33 | 2 | 7 | 31 | 0 | 57 | 1 | 0 | 10 | 32 | 25 | 31 | 24 | 2 | 3 | 34 | 0 | 39 | 1 | -1 | 2 | 37 | 24 | 56 | 24 |
| -2 | 7 | 30 | 63 | 54 | 33 | -2 | 8 | 31 | 0 | 37 | 1 | 1 | 10 | 32 | 63 | 63 | 63 | -2 | 4 | 34 | 42 | 45 | 42 | 0 | 2 | 37 | 13 | 29 | 12 |
| -1 | 7 | 30 | 70 | 95 | 23 | -1 | 8 | 31 | 88 | 88 | 20 | -1 | 11 | 32 | 80 | 66 | 19 | -1 | 4 | 34 | 107 | 135 | 15 | 0 | 3 | 37 | 121 | 103 | 17 |
| 0 | 7 | 30 | 70 | 45 | 30 | 0 | 8 | 31 | 74 | 23 | 27 | 0 | 11 | 32 | 0 | 42 | 1 | 0 | 4 | 34 | 75 | 89 | 37 | 0 | 4 | 37 | 167 | 153 | 13 |
| 1 | 7 | 30 | 77 | 95 | 28 | 1 | 8 | 31 | 100 | 88 | 70 | 1 | 11 | 32 | 19 | 66 | 19 | 1 | 4 | 34 | 130 | 135 | 34 | 0 | 5 | 37 | 48 | 60 | 47 |
| 2 | 7 | 30 | 64 | 54 | 44 | 2 | 8 | 31 | 0 | 37 | 1 | -1 | 12 | 32 | 0 | 42 | 1 | -1 | 5 | 34 | 115 | 116 | 14 | | | | | | |
| -2 | 8 | 30 | 130 | 134 | 14 | -2 | 9 | 31 | 83 | 71 | 22 | 0 | 12 | 32 | 55 | 87 | 54 | 0 | 5 | 34 | 0 | 4 | 1 | | | | | | |

### EXAMPLE 2

### Synthesis of 051810

The preparation of 051810 having the basic structure I can be accomplished by a common general method, i.e. the condensation of a bicyclic Windaus-Grundmann type ketone II with the allylic phosphine oxide III to the corresponding 2-methylene-19-nor-vitamin D analog IV followed by deprotection at C-1 and C-3 in the latter compound IV to obtain compound I, i.e. 051810.

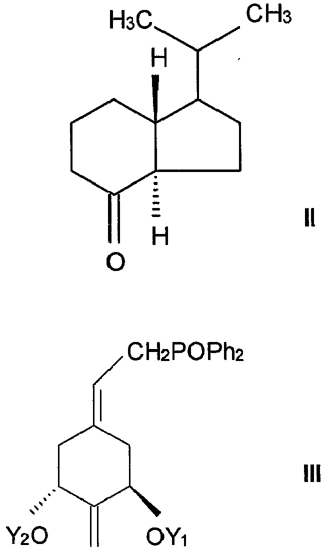

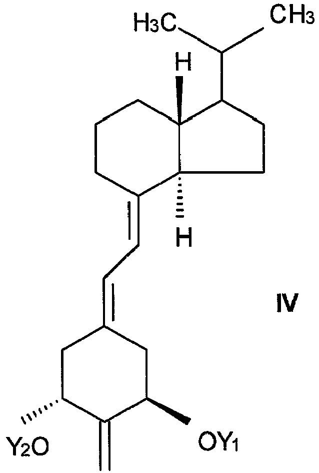

In phosphine oxide III, Y₁ and Y₂ are preferably hydroxy-protecting groups such as silyl protecting groups. The t-butyldimethylsilyl (TMDMS) group is an example of a particularly useful hydroxy-protecting group. The process described above represents an application of the convergent synthesis concept, which has been applied effectively to the preparation of numerous vitamin D compounds (see Lythgoe et al., J. Chem. Soc. Perkin Trans. I, 590 (1978); Lythgoe, Chem. Soc. Rev. 9, 449 (1983); Toh et al., J. Org. Chem. 48, 1414 (1983); Baggiolini et al., J. Org. Chem. 51, 3098 (1986); Sardina et al., J. Org. Chem. 51, 1264 (1986); J. Org. Chem. 51, 1269 (1986); DeLuca et al., U.S. Pat. No. 5,086,191; DeLuca et al., U.S. Pat. No. 5,536,713; and DeLuca et al, U.S. Pat. No. 5,843,928 all of which are hereby incorporated by reference in their entirety and for all purposes as if fully set forth herein.

Phosphine oxide III is a convenient reagent that can be used to prepare a large number of 19-nor-vitamin D compounds and is prepared according to the procedures described by Sicinski et al., J. Med. Chem., 41, 4662 (1998), DeLuca et al., U.S. Pat. No. 5,843,928; Perlman et al., Tetrahedron Lett. 32, 7663 (1991); and DeLuca et al., U.S. Pat. No. 5,086,191 which are hereby incorporated by reference in their entirety as if fully set forth herein.

The overall process of the synthesis of compound I is illustrated and described more completely in U.S. Pat. No. 5,843,928 entitled "2-Alkylidene-19-Nor-Vitamin D Compounds" and in U.S. Pat. No. 7,238,681, entitled "2-Methylene-18,19-Dinor-1α-Hydroxy-Homopregnacalciferol and Its Uses" the specifications of which are specifically incorporated herein by reference.

## Claims

1. 1α-hydroxy-2-methylene-18,19-dinor-homopregnacalciferol in crystalline form.

2. The crystalline form of 1α-hydroxy-2-methylene-18,19-dinor-homopregnacalciferol having molecular packing arrangement defined by space group P2 and unit cell dimensions a=4.8Å, b=22.9Å, c=36.1Å, α=90°, β=90° and γ=90°.

3. A three dimensional structure for 1α-hydroxy-2-methylene-18,19-dinor-homopregnacalciferol as defined by the molecular packing arrangement set forth in claim 2.

4. A method of purifying 1α-hydroxy-2-methylene-18,19-dinor-homopregnacalciferol, comprising the steps of:
(a) dissolving a product containing 1α-hydroxy-2-methylene-18,19-dinor-homopregnacalciferol to be purified in a solvent comprising ethyl acetate;
(b) adding hexane to said solvent and dissolved product to form a mixture;
(c) cooling said mixture containing said dissolved product below ambient temperature for a sufficient amount of time to form a precipitate of 1α-hydroxy-2-methylene-18,19-dinor-homopregnacalciferol crystals; and
(d) separating the 1α-hydroxy-2-methylene-18,19-dinor-homopregnacalciferol crystals from the mixture.

5. The method of claim 4 wherein the step of separating comprises filtering the mixture and precipitate to obtain the crystals.

6. The method of claim 4 including a further step (d) comprising repeating steps (a) through (c) using the recovered crystals from step (c) as the product of step (a).

7. The method of claim 4 wherein a ratio of ethyl acetate and hexane is about 1:99, by volume.

8. A method of preparing 1α-hydroxy-2-methylene-18,19-dinor-homopregnacalciferol crystals by diffusive exchange of solvents, comprising the steps of:
(a) dissolving a product containing 1α-hydroxy-2-methylene-18,19-dinor-homopregnacalciferol in a first solvent comprising benzene;
(b) providing a second solvent comprising hexane;
(c) allowing said first solvent with dissolved product and said second solvent to diffuse together for a sufficient amount of time to form a precipitate of 1α-hydroxy-2-methylene-18,19-dinor-homopregnacalciferol crystals; and
(d) recovering the 1α-hydroxy-2-methyelene-18,19-dinor-homopregnacalciferol crystals.

9. The method of claim 8 wherein a ratio of benzene and hexane is about 13:87, by volume.

10. The method of claim 8 wherein the step of recovering comprises filtering to obtain the crystals.

11. The method of claim 8 wherein the step of allowing said first solvent with dissolved product and said second solvent to diffuse together takes place in a closed system purged with argon.

12. The method of claim 8 wherein the step of allowing said first solvent with dissolved product and said second solvent to diffuse together takes place at room temperature.
